# EUROPEAN PATENT SPECIFICATION

(11) **EP 2 086 326 B1**
(45) Date of publication and mention of the grant of the patent: **08.01.2014**
(21) Application number: 07853118.3
(22) Date of filing: 12.11.2007
(51) Int. Cl.: C07D 401/14, C07D 401/04, C07D 417/04, C07D 417/14, A01N 43/66, A61K 31/53

(54) **CYCLOHEXENYL-ARYL COMPOUNDS FOR INFLAMMATION AND IMMUNE-RELATED USES**
CYCLOHEXENYL-ARYL-VERBINDUNGEN GEGEN ENTZÜNDUNGEN UND IMMUNSPEZIFISCHE ERKRANKUNGEN
COMPOSÉS CYCLOHEXÉNYL-ARYLE UTILISÉS DANS LE CADRE D'UNE INFLAMMATION OU DE TROUBLES IMMUNITAIRES

(30) Priority: 13.11.2006 US 858610 P
(43) Date of publication of application: 12.08.2009
(73) Proprietor: Synta Pharmaceuticals Corp., Lexington, MA 02421 (US)
(72) Inventor: BOHNERT, Gary, Cambridge, MA 02141 (US); CHEN, Shoujun, Bedford, MA 01730 (US)
(74) Representative: Wichmann, Hendrik
(86) International application number: PCT/US2007/023853
(87) International publication number: WO 2008/063504

(56) References cited:
- WO-A2-2006/083477
- WO-A2-2007/087443
- US-A1- 2005 107 399
- US-A1- 2006 100 204
- US-A1- 2006 173 021
- US-B1- 6 720 317

## Description

### FIELD OF THE INVENTION

This invention relates to biologically active chemical compounds, namely cyclohexenyl-phenyl derivatives that may be used for immunosuppression or to treat or prevent inflammatory conditions and immune disorders.

### BACKGROUND OF THE INVENTION

Inflammation is a mechanism that protects mammals from invading pathogens. However, while transient inflammation is necessary to protect a mammal from infection, uncontrolled inflammation causes tissue damage and is the underlying cause of many illnesses. Inflammation is typically initiated by binding of an antigen to T-cell antigen receptor. Antigen binding by a T-cell initiates calcium influx into the cell via calcium ion channels, such as Ca²⁺-release-activated Ca²⁺ channels (CRAC). Calcium ion influx in turn initiates a signaling cascade that leads to activation of these cells and an inflammatory response characterized by cytokine production.

Interleukin 2 (IL-2) is a cytokine that is secreted by T cells in response to calcium ion influx into the cell. IL-2 modulates immunological effects on many cells of the immune system. For example, it is a potent T cell mitogen that is required for the T cell proliferation, promoting their progression from G1 to S phase of the cell cycle; it stimulates the growth of NK cells; and it acts as a growth factor to B cells and stimulates antibody synthesis.

IL-2, although useful in the immune response, can cause a variety of problems. IL-2 damages the blood-brain barrier and the endothelium of brain vessels. These effects may be the underlying causes of neuropsychiatric side effects observed under IL-2 therapy, e.g. fatigue, disorientation and depression. It also alters the electrophysiological behaviour of neurons.

Due to its effects on both T and B cells, IL-2 is a major central regulator of immune responses. It plays a role in inflammatory reactions, tumour surveillance, and hematopoiesis. It also affects the production of other cytokines, inducing IL-1, TNF-α and TNF-β secretion, as well as stimulating the synthesis of IFN-y in peripheral leukocytes.

T cells that are unable to produce IL-2 become inactive (anergic). This renders them potentially inert to any antigenic stimulation they might receive in the future. As a result, agents which inhibit IL-2 production can be used for immunosupression or to treat or prevent inflammation and immune disorders. This approach has been clinically validated with immunosuppressive drugs such as cyclosporin, FK506, and RS61443. Despite this proof of concept, agents that inhibit IL-2 production remain far from ideal. Among other problems, efficacy limitations and unwanted side effects (including dose-dependant nephrotoxicity and hypertension) hinder their use.

Over production of proinflammatory cytokines other than IL-2 has also been implicated in many autoimmune diseases. For example, Interleukin 5 (IL-5), a cytokine that increases the production of eosinophils, is increased in asthma. Overproduction of IL-5 is associated with accumulation of eosinophils in the asthmatic bronchial mucosa, a hall mark of allergic inflammation. Thus, patients with asthma and other inflammatory disorders involving the accumulation of eosinophils would benefit from the development of new drugs that inhibit the production of IL-5.

Interleukin 4 (IL-4) and interleukin 13 (IL-13) have been identified as mediators of the hypercontractility of smooth muscle found in inflammatory bowel disease and asthma. Thus, patients with athsma and inflammatory bowel disease would benefit from the development of new drugs that inhibit IL-4 and IL-13 production.

Granulocyte macrophage-colony stimulating factor (GM-CSF) is a regulator of maturation of granulocyte and macrophage lineage population and has been implicated as a key factor in inflammatory and autoimmune diseases. Anti-GM-CSF antibody blockade has been shown to ameliorate autoimmune disease. Thus, development of new drugs that inhibit the production of GM-CSF would be beneficial to patients with an inflammatory or autoimmune disease.

There is therefore a continuing need for new drugs which overcome one or more of the shortcomings of drugs currently used for immunosuppression or in the treatment or prevention of inflammatory disorders, allergic disorders and autoimmune disorders. Desirable properties of new drugs include efficacy against diseases or disorders that are currently untreatable or poorly treatable, new mechanism of action, oral bioavailability and/or reduced side effects.

US 2005/107399 A1 discloses on claims 6, 47, 49-51 and 55-86 compounds acting as cytokine inhibitors (and thus against inflammation, immune diseases and allergy) having the following core: substituted cycloheterocycloalkyl-carbocyclyl or heterocyclyl- NH-CO-heteroaryl. These compounds are shown on example 14 page 101 to be active as inhibitors of THF with IC50 below 10 µM.

US 2006/173021 A1 discloses on claims 1-163 compounds with the following core: an optionally substituted aryl or heteroaryl linked to a pyrimidine. These compounds are disclosed as acting against autoimmune, allergic and inflammatory illnesses or conditions. Particularly interesting are examples 5, 57-59, 61-84 as seen on pages 21, 30, 31-37. These compounds are shown to be active as IL-2 inhibitors on page 56 example 2 in the nanomolar order of magnitude.

### SUMMARY OF THE INVENTION

This invention meets the above-mentioned needs by providing certain cyclohexenyl-phenyl derivatives that inhibit the activity of CRAC ion channels and inhibit the production of IL-2, IL-4, IL-5, IL-13, GM-CSF, TNF-α, and IFNγ. These compounds are particularly useful for immunosuppression and/or to treat or prevent inflammatory conditions and immune disorders. Additionally, compounds of the invention have good oral bioavailability and solubility.

The invention relates to compounds of formula (I): 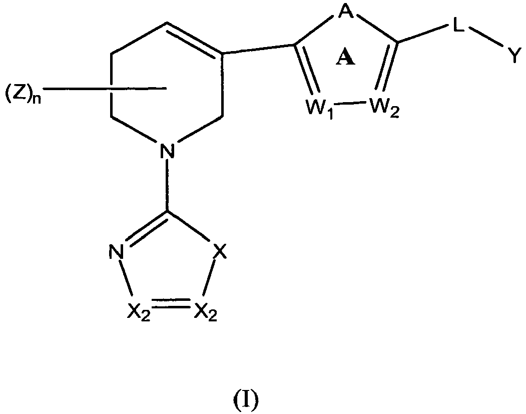 wherein:
A is -CR₁=CR₂-, -N=CR₁-, or -CR₁=N-;
W₁ and W₂ are each, independently, CR₁ or N;
X is -S-, -O-, or -NR_{c}-;
each of X₂ are independently selected from -N- or -CR_{d}-;
Y is an optionally substituted aryl, an optionally substituted heteroaryl, an optionally substituted alkyl, an optionally substituted cycloalkyl, or an optionally substituted alkenyl;
L is -NRCH₂-, -CH₂NR-, -NR-C(O)-, or -C(O)-NR-;
Z is alkyl, halo, or -C(O)R₅-;
R, for each occurrence, is independently -H, alkyl, -C(O)-R₁₃, or -C(O)OR₁₃;
R_{c} is -H or C₁-C₄ alkyl;
R_{d} for each occurrence is independently -H or lower alkyl;
R₁ and R₂, for each occurrence, are independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, -OR₅, -SR₅, -NR₆R₇, -C(O)NR₆R₇, -NR₅C(O)R₅, -C(O)R₅, -C(O)OR₅, -OC(O)R₅, -C(O)SR₅, -SC(O)R₅, -C(S)NR₆R₇, -NR₅C(S)R₅, -C(S)R₅, -C(S)OR₅, -OC(S)R₅, -C(S)SR₅, -SC(S)R₅, -C(NR₈)NR₆R₇, -NR₅C(NR₈)R₅, -C(NR₈)R₅, -C(NR₈)OR₅, -OC(NR₈)R₅, -C(NR₈)SR₅, -SC(NR₈)R₅, -OC(O)OR₅, -OC(O)NR₆R₇, -NR₅C(O)OR₅, -NR₅C(O)NR₆R₇, -SC(O)OR₅, -SC(O)NR₆R₇, -SC(O)SR₅, -NR₅C(O)SR₅, -OC(O)SR₅, -OC(S)OR₅, -OC(S)NR₆R₇, -NR₅C(S)OR₅, -NR₅C(S)NR₆R₇, -SC(S)OR₅, -SC(S)NR₆R₇, -SC(S)SR₅, -NR₅C(S)SR₅, -OC(S)SR₅, -OC(NR₈)OR₅, -OC(NR₈)NR₆R₇, -NR₅C(NR₈)OR₅, -NR₅C(NR₈)NR₆R₇, -SC(NF₈)OR₅, -SC(NR₈)NR₆R₇, -SC(NR₈)SR₅, -NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, -S(O)ₚOR₅, -OS(O)ₚR₅, or -OS(O)OR₅;
R₅, for each occurrence, is independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
R₆ and R₇, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₆ and R₇ taken together with the nitrogen to which they are attached are an optionally substituted heterocyclyl or optionally substituted heteroaryl;
R₈, for each occurrence, is independently -H, a halo, an alkyl, -OR₅, -NR₆R₇, -C(O)R₅, -C(O)OR₅, or -C(O)NR₆R₇;
R₁₁ is H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, -OR₅, -SR₅, -NR₆R₇, -C(O)NR₆R₇, -C(O)R₅, -C(O)OR₅, -C(O)SR₅, -C(S)NR₆R₇, -C(S)R₅, -C(S)OR₅, -C(S)SR₅, -C(NR₈)NR₆R₇, -C(NR₈)R₅, -C(NR₈)OR₅, or -C(N₈)SR₅;
n is 0, 1, 2, 3, 4, 5, 6, 7; and
p is 1 or 2;
or a pharmaceutically acceptable salt, solvate, or clathrate thereof.

In some embodiments, the compound of formula (I) is not:
2,6-Difluoro-N-[4-(1-thiazol-2-yl-4-methyl-1,2,5,6-tetrahydro-pyridin-3-yl)-phenyl]-benzamide;
N-[4-(1-thiazol-2-yl-4-methyl-1,2,5,6-tetrahydro-pyridin-3-yl)-phenyl]-3-fluoro-isonicotinamide;
N-[4-(1-thiazol-2-yl-4-methyl-1,2,5,6-tetrahydro-pyridin-3-yl)-phenyl]-3-methyl-isonicotinamide;
N-[4-(1-thiazol-2-yl-4-methyl-1,2,5,6-tetrahydro-pyridin-3-yl)-phenyl]-3-fluoro-isonicotinamide;
2,6-Difluoro-N-[4-(1-oxazol-2-yl-4-methyl-1,2,5,6-tetrahydro-pyridin-3-yl)-phenyl]-benzamide;
N-[4-(1-oxazol-2-yl-4-methyl-1,2,5,6-tetrahydro-pyridin-3-yl)-phenyl]-3-fluoro-isonicotinamide;
2,6-Difluoro-N-[5-(1-thiazol-2-yl-4-methyl-1,2,5,6-tetrahydro-pyridin-3-yl)-pyridin-2-yl]-benzamide, hydrochloride;
N-[4-(1-thiazol-2-yl-4-methyl-1,2,5,6-tetrahydro-pyridin-3-yl)-phenyl]-3-fluoro-isonicotinamide, dihydrochloride;
2,6-Difluoro-N-[4-(1-thiazol-2-yl-4-methyl-1,2,5,6-tetrahydro-pyridin-3-yl)-phenyl]-benzamide, hydrochloride;
2,6-Difluoro-N-[4-(1-thiazol-2-yl-4-methyl-1,2,5,6-tetrahydro-pyridin-3-yl)-phenyl]-benzamide;
5-Methyl-pyrimidine-4-carboxylic acid [4-(1-thiazol-2-yl-4-methyl-1,2,5,6-tetrahydro-pyridin-3-yl)-phenyl]-amide;
2,6-Difluoro-N-[4-(1-thiazol-2-yl-4-formyl-1,2,5,6-tetrahydro-pyridin-3-yl)-phenyl]-benzamide;
2,6-Difluoro-N-[4-(1-oxazol-2-yl-4-methyl-1,2,5,6-tetrahydro-pyridin-3-yl)-phenyl]-benzamide, hydrochloride;
2,6-Difluoro-N- {4-[1-(4-methyl-oxazol-2-yl)-4-methyl-1,2,5,6-tetrahydro-pyridin-3-yl]-phenyl}-benzamide;
5-Methyl-pyrimidine-4-carboxylic acid {4-[1-(4,5-dimethyl-oxazol-2-yl)-4-methyl-1,2,5,6-tetrahydro-pyridin-3-yl]-phenyl}-amide;
2,6-Difluoro-N-{4-[1-(4,5-dimethyl-oxazol-2-yl)-4-methyl-1,2,5,6-tetrahydro-pyridin-3-yl]-p henyl}-benzamide;
N-{4-[1-(1H-tetrazol-5-yl)-4-methyl-1,2,5,6-tetrahydro-pyridin-3-yl]-phenyl}-3-fluoro-isonicotinamide;
N-{4-[1-(1-methyl-1H-tetrazol-5-yl)-4-methyl-1,2,5,6-tetrahydro-pyridin-3-yl]-phenyl}-3-fluoro-isonicotinamide;
N-{4-[1-(2-methyl-2H-tetrazol-5-yl)-4-methyl-1,2,5,6-tetrahydro-pyridin-3-yl]-phenyl}-3-fluoro-isonicotinamide;
2,6-Difluoro-N-{4-[1-(2-methyl-2H-tetrazol-5-yl)-4-methyl-1,2,5,6-tetrahydro-pyridin-3-yl]-phenyl}-benzamide;
2,6-Difluoro-N-{4-[1-(1H-tetrazol-5-yl)-4-methyl-1,2,5,6-tetrahydro-pyridin-3-yl]-phenyl}-benzamide;
2,6-Difluoro-N-{4-[1-(1-methyl-1H-tetrazol-5-yl)-4-methyl-1,2,5,6-tetrahydro-pyridin-3-yl]-phenyl}-benzamide;
2,6-Difluoro-N-[5-(1-thiazol-2-yl-4-methyl-1,2,5,6-tetrahydro-pyridin-3-yl)-pyrimidin-2-yl]-benzamide; or
2,6-Difluoro-N-[5-(1-thiazol-2-yl-4-methyl-1,2,5,6-tetrahydro-pyridin-3-yl)-pyrimidin-2-yl]-benzamide, hydrochloride.

The invention also relates to compounds of formula (II): 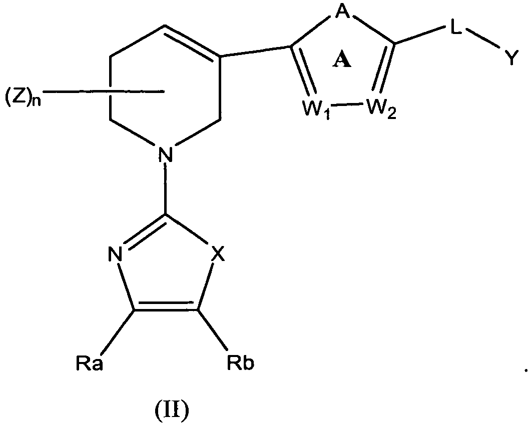 wherein:
Rₐ and R_{b} are each, independently, -H or C₁-C₄ alkyl; and A, L, X, Y, W₁, W₂, Z, and n are defined as for formula (I);
or a pharmaceutically acceptable salt, solvate, or clathrate thereof.

The invention also relates to compounds of formula (III): 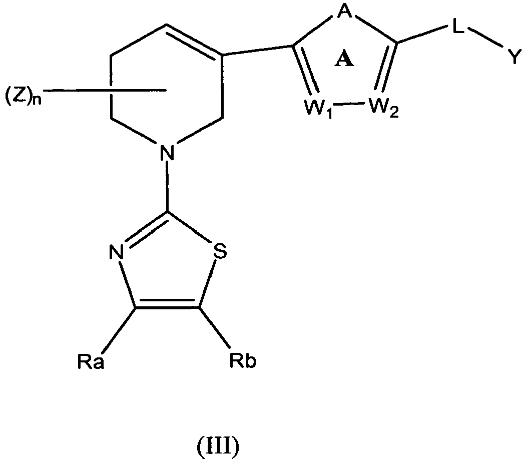 wherein A, L, Y, W₁, W₂, Z, and n are defined as for formula (I); Rₐ and R_{b} are defined as for formula (II); or a pharmaceutically acceptable salt, solvate, or clathrate thereof.

The invention also relates to compounds of formula (IV): 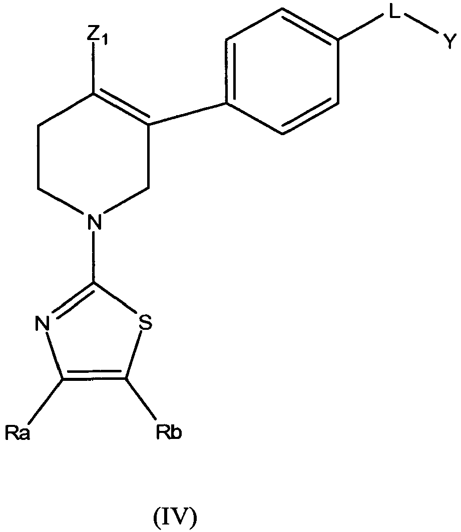 wherein Z₁ is alkyl, halo, or -C(O)R₅; L, Y, are defined as for formula (I); and Rₐ and R_{b} are defined as for formula (II); or a pharmaceutically acceptable salt, solvate, or clathrate thereof.

The invention also relates to compounds of formula (V): 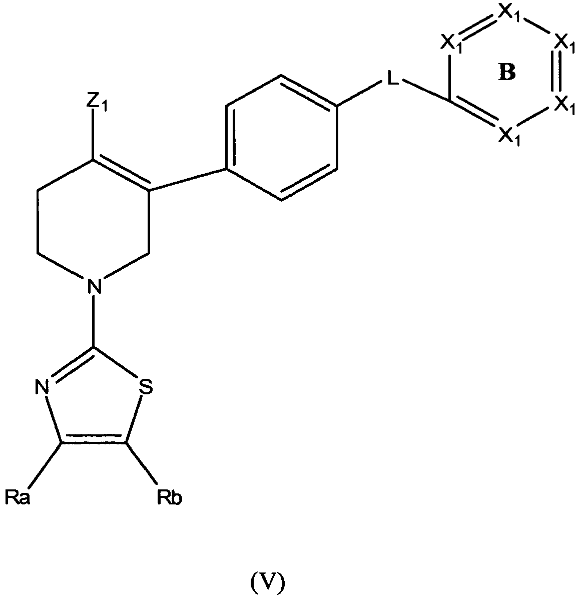 wherein each X₁ is independently N or CR₁ and at least two X₁ groups are CR₁; L is -NR-C(O)- or -C(O)-NR-; Rₐ and R_{b} are defined as for formula (II); and Z₁ is defined as for formula (IV); or a pharmaceutically acceptable salt, solvate, or clathrate thereof.

A compound of the invention or a pharmaceutically acceptable salt, solvate, or clathrate thereof is particularly useful for inhibiting immune cell (*e.g*.*,* T-cells and/or B-cells) activation (*e.g*., activation in response to an antigen). In particular, a compound of the invention or a pharmaceutically acceptable salt, solvate, or clathrate thereof can inhibit the production of certain cytokines that regulate immune cell activation. For example, a compound of the invention or a pharmaceutically acceptable salt, solvate, or clathrate thereof can inhibit the production of IL-2, IL-4, IL-5, IL-13, GM-CSF, TNF-α, INF-γ or combinations thereof. Moreover, a compound of the invention or a pharmaceutically acceptable salt, solvate, or clathrate thereof can modulate the activity of one or more ion channel involved in activation of immune cells, such as CRAC ion channels.

A compound of the invention or a pharmaceutically acceptable salt, solvate, or clathrate thereof is particularly useful for immunosuppression or for treating or preventing inflammatory conditions, allergic disorders, and immune disorders.

The invention also encompasses pharmaceutical compositions comprising a compound of the invention or a pharmaceutically acceptable salt, solvate, or clathrate thereof; and a pharmaceutically acceptable carrier or vehicle. These compositions may further comprise additional agents. These compositions are useful for immunosuppression and treating or preventing inflammatory conditions, allergic disorders and immune disorders.

The invention further encompasses a compound of the invention or a pharmaceutically acceptable salt, solvate, or clathrate thereof, or a pharmaceutical composition comprising a compound of the invention or a pharmaceutically acceptable salt, solvate, or clathrate thereof for use in treating or preventing inflammatory conditions, allergic disorders, and immune disorders. This may also comprise administering to the subject an additional agent separately or in a combination composition with the compound of the invention or a pharmaceutically acceptable salt, solvate, or clathrate thereof.

The invention further encompasses a compound of the invention or a pharmaceutically acceptable salt, solvate, or clathrate thereof, or a pharmaceutical composition comprising a compound of the invention or a pharmaceutically acceptable salt, solvate, or clathrate thereof for use in suppressing the immune system of a subject. This may also comprise administering to the subject an additional agent separately or in a combination composition with the compound of the invention or a pharmaceutically acceptable salt, solvate, or clathrate thereof.

The invention further encompasses a compound of the invention or a pharmaceutically acceptable salt, solvate, or clathrate thereof or a pharmaceutical composition comprising a compound of the invention or a pharmaceutically acceptable salt, solvate, or clathrate thereof for use in inhibiting immune cell activation, including inhibiting proliferation of T cells and/or B cells, *in vivo* or *in vitro.*

The invention further encompasses a compound of the invention or a pharmaceutically acceptable salt, solvate, or clathrate thereof or a pharmaceutical composition comprising a compound of the invention or a pharmaceutically acceptable salt, solvate, or clathrate thereof for use in inhibiting cytokine production in a cell, (*e.g*., IL-2, IL-4, IL-5, IL-13, GM-CSF, TNF-α, and/or INF-γ production) *in vivo* or *in vitro.*

The invention further encompasses a compound of the invention or a pharmaceutically acceptable salt, solvate, or clathrate thereof or a pharmaceutical composition comprising a compound of the invention or a pharmaceutically acceptable salt, solvate, or clathrate thereof for use in modulating ion channel activity (*e.g*., CRAC) *in vivo* or *in vitro.*

The present invention may be practiced with a compound of the invention alone, or in combination with other agents, such as other immunosuppressive agents, anti-inflammatory agents, agents for the treatment of allergic disorders or agents for the treatment of immune disorders.

### DETAILED DESCRIPTION OF THE INVENTION

### DEFINITIONS

Unless otherwise specified, the below terms used herein are defined as follows:
As used herein, the term an "aromatic ring" or "aryl" means a monocyclic or polycyclic-aromatic ring or ring radical comprising carbon and hydrogen atoms. Examples of suitable aryl groups include, but are not limited to, phenyl, tolyl, anthacenyl, fluorenyl, indenyl, azulenyl, and naphthyl, as well as benzo-fused carbocyclic moieties such as 5,6,7,8-tetrahydronaphthyl. An aryl group can be unsubstituted or substituted with one or more substituents (including without limitation alkyl (preferably, lower alkyl or alkyl substituted with one or more halo), hydroxy, alkoxy (preferably, lower alkoxy), alkylthio, cyano, halo, amino, and nitro. In certain embodiments, the aryl group is a monocyclic ring, wherein the ring comprises 6 carbon atoms.

As used herein, the term "alkyl" means a saturated straight chain or branched non-cyclic hydrocarbon typically having from 1 to 10 carbon atoms. Representative saturated straight chain alkyls include methyl, ethyl, n-propyl, n-butyl, n-pentyl, n-hexyl, n-heptyl, n-octyl, n-nonyl and n-decyl; while saturated branched alkyls include isopropyl, sec-butyl, isobutyl, tert-butyl, isopentyl, 2-methylbutyl, 3-methylbutyl, 2-methylpentyl, 3-methylpentyl, 4-methylpentyl, 2-methylhexyl, 3-methylhexyl, 4-methylhexyl, 5-methylhexyl, 2,3-dimethylbutyl, 2,3-dimethylpentyl, 2,4-dimethylpentyl, 2,3-dimethylhexyl, 2,4-dimethylhexyl, 2,5-dimethylhexyl, 2,2-dimethylpentyl, 2,2-dimethylhexyl, 3,3-dimtheylpentyl, 3,3-dimethylhexyl, 4,4-dimethylhexyl, 2-ethylpentyl, 3-ethylpentyl, 2-ethylhexyl, 3-ethylhexyl, 4-ethylhexyl, 2-methyl-2-ethylpentyl, 2-methyl-3-ethylpentyl, 2-methyl-4-ethylpentyl, 2-methyl-2-ethylhexyl, 2-methyl-3-ethylhexyl, 2-methyl-4-ethylhexyl, 2,2-diethylpentyl, 3,3-diethylhexyl, 2,2-diethylhexyl, 3,3-diethylhexyl and the like. Alkyl groups included in compounds of this invention may be optionally substituted with one or more substituents, such as amino, alkylamino, alkoxy, alkylthio, oxo, halo, acyl, nitro, hydroxyl, cyano, aryl, alkylaryl, aryloxy, arylthio, arylamino, carbocyclyl, carbocyclyloxy, carbocyclylthio, carbocyclylamino, heterocyclyl, heterocyclyloxy, heterocyclylamino, heterocyclylthio, and the like. In addition, any carbon in the alkyl segment may be substituted with oxygen (=O), sulfur (=S), or nitrogen (=NR²³, wherein R²³ is -H, an alkyl, acetyl, or aralkyl). Lower alkyls are typically preferred for the compounds of this invention.

The term alkylene refers to an alkyl group that has two points of attachment to two moieties (e.g., {-CH₂-}, -{CH₂CH₂-}, 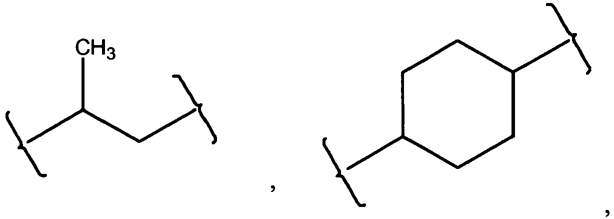 etc., wherein the brackets indicate the points of attachment). Alkylene groups may be substituted or unsubstituted.

An aralkyl group refers to an aryl group that is attached to another moiety via an alkylene linker. Aralkyl groups can be substituted or unsubstituted.

The term "alkoxy," as used herein, refers to an alkyl group which is linked to another moiety though an oxygen atom. Alkoxy groups can be substituted or unsubstituted.

The term "alkoxyalkoxy," as used herein, refers to an alkoxy group in which the alkyl portion is substituted with another alkoxy group.

The term "alkyl sulfanyl," as used herein, refers to an alkyl group which is linked to another moiety though a divalent sulfur atom. Alkyl sulfanyl groups can be substituted or unsubstituted.

The term "alkylamino," as used herein, refers to an amino group in which one hydrogen atom attached to the nitrogen has been replaced by an alkyl group. The term "dialkylamino," as used herein, refers to an amino group in which two hydrogen atoms attached to the nitrogen have been replaced by alkyl groups, in which the alkyl groups can be the same or different. Alkylamino groups and dialkylamino groups can be substituted or unsubstituted.

As used herein, the term "alkenyl" means a straight chain or branched, hydrocarbon radical typically having from 2 to 10 carbon atoms and having at least one carbon-carbon double bond. Representative straight chain and branched alkenyls include vinyl, allyl, 1-butenyl, 2-butenyl, isobutylenyl, 1-pentenyl, 2-pentenyl, 3-methyl-1-butenyl, 1-methyl-2-butenyl, 2,3-dimethyl-2-butenyl, 1-hexenyl, 2-hexenyl, 3-hexenyl, 1-heptenyl, 2-heptenyl, 3-heptenyl, 1-octenyl, 2-octenyl, 3-octenyl, 1-nonenyl, 2-nonenyl, 3-nonenyl, 1-decenyl, 2-decenyl, 3-decenyl and the like. Alkenyl groups can be substituted or unsubstituted.

As used herein, the term "alkynyl" means a straight chain or branched, hydrocarbonon radical typically having from 2 to 10 carbon atoms and having at lease one carbon-carbon triple bond. Representative straight chain and branched alkynyls include acetylenyl, propynyl, 1-butynyl, 2-butynyl, 1-pentynyl, 2-pentynyl, 3-methyl-1-butynyl, 4-pentynyl,-1-hexynyl, 2-hexynyl, 5-hexynyl, 1-heptynyl, 2-heptynyl, 6-heptynyl, 1-octynyl, 2-octynyl, 7-octynyl, 1-nonynyl, 2-nonynyl, 8-nonynyl, 1-decynyl, 2-decynyl, 9-decynyl and the like. Alkynyl groups can be substituted or unsubstituted.

As used herein, the term "cycloalkyl" means a saturated, mono- or polycyclic alkyl radical typically having from 3 to 10 carbon atoms. Representative cycloalkyls include cyclopropyl, cyclobutyl, cyclopentyl, cyclohexyl, cycloheptyl, cyclooctyl, cyclononyl, cyclodecyl, adamantly, decahydronaphthyl, octahydropentalene, bicycle[1.1.1]pentanyl, and the like. Cycloalkyl groups can be substituted or unsubstituted.

As used herein, the term "cycloalkenyl" means a cyclic non-aromatic alkenyl radical having at least one carbon-carbon double bond in the cyclic system and typically having from 5 to 10 carbon atoms. Representative cycloalkenyls include cyclopentenyl, cyclopentadienyl, cyclohexenyl, cyclohexadienyl, cycloheptenyl, cycloheptadienyl, cycloheptatrienyl, cyclooctenyl, cyclooctadienyl, cyclooctatrienyl, cyclooctatetraenyl, cyclononenyl, cyclononadienyl, cyclodecenyl, cyclodecadienyl and the like. Cycloalkenyl groups can be substituted or unsubstituted.

As used herein, the term "heterocycle" or "heterocyclyl" means a monocyclic or polycyclic heterocyclic ring (typically having 3- to 14-members) which is either a saturated ring or a unsaturated non-aromatic ring. A 3-membered heterocycle can contain up to 3 heteroatoms, and a 4- to 14-membered heterocycle can contain from 1 to about 8 heteroatoms. Each heteroatom is independently selected from nitrogen, which can be quatemized; oxygen; and sulfur, including sulfoxide and sulfone. The heterocycle may be attached via any heteroatom or carbon atom. Representative heterocycles include morpholinyl, thiomorpholinyl, pyrrolidinonyl, pyrrolidinyl, piperidinyl, piperazinyl, hydantoinyl, valerolactamyl, oxiranyl, oxetanyl, tetrahydrofuranyl, tetrahydropyranyl, tetrahydropyrindinyl, tetrahydropyrimidinyl, tetrahydrothiophenyl, tetrahydrothiopyranyl, and the like. A heteroatom may be substituted with a protecting group known to those of ordinary skill in the art, for example, the hydrogen on a nitrogen may be substituted with a tert-butoxycarbonyl group. Furthermore, the heterocyclyl may be optionally substituted with one or more substituents (including without limitation a halogen atom, an alkyl radical, or aryl radical). Only stable isomers of such substituted heterocyclic groups are contemplated in this definition. Heterocyclyl groups can be substituted or unsubstituted.

As used herein, the term "heteroaromatic" or "heteroaryl" means a monocyclic or polycyclic heteroaromatic ring (or radical thereof) comprising carbon atom ring members and one or more heteroatom ring members (such as, for example, oxygen, sulfur or nitrogen). Typically, the heteroaromatic ring has from 5 to about 14 ring members in which at least 1 ring member is a heteroatom selected from oxygen, sulfur and nitrogen. In another embodiment, the heteroaromatic ring is a 5 or 6 membered ring and may contain from 1 to about 4 heteroatoms. In another embodiment, the heteroaromatic ring system has a 7 to 14 ring members and may contain from 1 to about 7 heteroatoms. Representative heteroaryls include pyridyl, furyl, thienyl, pyrrolyl, oxazolyl, imidazolyl, indolizinyl, thiazolyl, isoxazolyl, pyrazolyl, isothiazolyl, pyridazinyl, pyrimidinyl, pyrazinyl, triazinyl, triazolyl, pyridinyl, thiadiazolyl, pyrazinyl, quinolyl, isoquniolyl, indazolyl, benzoxazolyl, benzofuryl, benzothiazolyl, indolizinyl, imidazopyridinyl, isothiazolyl, tetrazolyl, benzimidazolyl, benzoxazolyl, benzothiazolyl, benzothiadiazolyl, benzoxadiazolyl, indolyl, tetrahydroindolyl, azaindolyl, imidazopyridyl, qunizaolinyl, purinyl, pyrrolo[2,3]pyrimidyl, pyrazolo[3,4]pyrimidyl or benzo(b)thienyl and the like. These heteroaryl groups may be optionally substituted with one or more substituents A heteroaralkyl group refers to a heteroaryl group that is attached to another moiety via an alkylene linker. Heteroaralkyl groups can be substituted or unsubstituted.

As used herein, the term "halogen" or "halo" means -F, -Cl, -Br or -I.

As used herein, the term "haloalkyl" means an alkyl group in which one or more -H is replaced with a halo group. Examples of haloalkyl groups include -CF₃, -CHF₂, -CCl₃, -CH₂CH₂Br, -CH₂CH(CH₂CH₂Br)CH₃, -CHICH₃, and the like.

As used herein, the term "haloalkoxy" means an alkoxy group in which one or more -H is replaced with a halo group. Examples of haloalkoxy groups include -OCF₃ and -OCHF₂.

As used herein, the term "contiguous linear connectivity" means connected together so as to form an uninterrupted linear array or series of atoms. For example, a linker of the compounds described herein having a specified number of atoms in contiguous linear connectivity has at least that number of atoms connected together so as to form an uninterrupted chain, but may also include additional atoms that are not so connected (*e.g*., branches or atoms contained within a ring system).

As used herein, the term "linker" means a diradical having from 1-3 atoms in contiguous linear connectivity (*i.e*., as defined above and excluding atoms present in any side chains and branches), that covalently connects the A ring portion of a compound of this invention to the Y group of the compound, as illustrated in formula (I). The atoms of the linker in contiguous linear connectivity may be connected by saturated or unsaturated covalent bonds. Linkers include, but are not limited to, alkylidene, alkenylidene, alkynylidene and cycloalkylidene (such as lower alkylidene, cycloalkylidene, alkylycloalkylidene and alkyl-substituted alkylidene) linkers wherein one or more (*e.g*., between 1 and 3, (*e.g*., 1 or 2)) carbon atoms may be optionally replaced with O, S, or N and wherein two or more (*e.g*., 2-3 (*e.g*., 2 or 3)) adjacent atoms may be optionally linked together to form a carbocyclic or heterocyclic moiety within the linker (which may be monocyclic, polycyclic and/or fused, and which may be saturated, unsaturated, or aromatic). Examples of specific linkers useful in the compounds of the invention include (without limitation) diradicals of alkyl, alkenyl, alynyl, alkoxy, alkoxyalkyl, alkylaminoalkyl, cycloalkyl, alkylcycloalkyl, and alkyl-substituted alkylcycloalkyl (wherein one or more carbon atoms in any of these linkers may be optionally replaced with O, S, or N).

The terms "bioisostere" and "bioisosteric replacement" have the same meanings as those generally recognized in the art. Bioisosteres are atoms, ions, or molecules in which the peripheral layers of electrons can be considered substantially identical. The term bioisostere is usually used to mean a portion of an overall molecule, as opposed to the entire molecule itself. Bioisosteric replacement involves using one bioisostere to replace another with the expectation of maintaining or slightly modifying the biological activity of the first bioisostere. The bioisosteres in this case are thus atoms or groups of atoms having similar size, shape and electron density. Preferred bioisosteres of esters, amides or carboxylic acids are compounds containing two sites for hydrogen bond acceptance. In one embodiment, the ester, amide or carboxylic acid bioisostere is a 5-membered monocyclic heteroaryl ring, such as an optionally substituted IH-imidazolyl, an optionally substituted oxazolyl, 1H-tetrazolyl, [1,2,4]triazolyl, or an optionally substituted [1,2,4]oxadiazolyl.

As used herein, the terms "subject", "patient" and "animal", are used interchangeably and include, but are not limited to, a cow, monkey, horse, sheep, pig, chicken, turkey, quail, cat, dog, mouse, rat, rabbit, guinea pig and human. The preferred subject, patient or animal is a human.

As used herein, the term "lower" refers to a group having up to four carbon atoms. For example, a "lower alkyl" refers to an alkyl radical having from 1 to 4 carbon atoms, and a "lower alkenyl" or "lower alkynyl" refers to an alkenyl or alkynyl radical having from 2 to 4 carbon atoms, respectively. A lower alkoxy or a lower alkyl sulfanyl refers to an alkoxy or a alkyl sulfanyl having from 1 to 4 carbon atoms. Lower substituents are typically preferred.

Where a particular substituent, such as an alkyl substituent, occurs multiple times in a given structure or moeity, the identity of the substitutent is independent in each case and may be the same as or different from other occurrences of that substituent in the structure or moiety. Furthermore, individual substituents in the specific embodiments and exemplary compounds of this invention are preferred in combination with other such substituents in the compounds of this invention, even if such individual substituents are not expressly noted as being preferred or not expressly shown in combination with other substituents.

The compounds of the invention are defined herein by their chemical structures and/or chemical names. Where a compound is referred to by both a chemical structure and a chemical name, and the chemical structure and chemical name conflict, the chemical structure is determinative of the compound's identity.

Suitable substituents for an alkyl, alkoxy, alkyl sulfanyl, alkylamino, dialkylamino, alkylene, alkenyl, alkynyl, cycloalkyl, cycloalkenyl, heterocyclyl, aryl, aralkyl, heteroaryl, and heteroarylalkyl groups include any substituent which will form a stable compound of the invention. Examples of substituents for an alkyl, alkoxy, alkylsulfanyl, alkylamino, dialkylamino, alkylene, alkenyl, alkynyl, cycloalkyl, cycloalkenyl, heterocyclyl, aryl, aralkyl, heteroaryl, and heteroarylalkyl include an alkyl, alkoxy, alkyl sulfanyl, alkylamino, dialkylamino, an alkenyl, an alkenyl, an cycloalkyl, an cycloalkenyl, an heterocyclyl, an aryl, an heteroaryl, an aralkyl, an heteraralkyl, a haloalkyl, -C(O)NR₁₃R₁₄, -NR₁₅C(O)R₁₆, halo, -OR₁₅, cyano, nitro, haloalkoxy, -C(O)R₁₅, -NR₁₃R₁₄, -SR₁₅, -C(O)OR₁₅, -OC(O)R₁₅, -NR₁₅C(O)NR₁₃R₁₄, -OC(O)NR₁₃R₁₄, -NR₁₅C(O)OR₁₆, -S(O)ₚR₁₅, or -S(O)ₚNR₁₃R₁₄, wherein R₁₃ and R₁₄, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₁₃ and R₁₄ taken together with the nitrogen to which they are attached is optionally substituted heterocyclyl or optionally substituted heteroaryl; and R₁₅ and R₁₆ for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;

In addition, alkyl, cycloalkyl, alkylene, a heterocyclyl, and any saturated portion of a alkenyl, cycloalkenyl, alkynyl, aralkyl, and heteroaralkyl groups, may also be substituted with =O, =S, =N-R₁₅.

When a heterocyclyl, heteroaryl, or heteroaralkyl group contains a nitrogen atom, it may be substituted or unsubstituted. When a nitrogen atom in the aromatic ring of a heteroaryl group has a substituent the nitrogen may be a quaternary nitrogen.

Choices and combinations of substituents and variables envisioned by this invention are only those that result in the formation of stable compounds. The term "stable", as used herein, refers to compounds which possess stability sufficient to allow manufacture and which maintains the integrity of the compound for a sufficient period of time to be useful for the purposes detailed herein (e.g., therapeutic or prophylactic administration to a subject). Typically, such compounds are stable at a temperature of 40°C or less, in the absence of excessive moisture, for at least one week. Such choices and combinations will be apparent to those of ordinary skill in the art and may be determined without undue experimentation.

Unless indicated otherwise, the compounds of the invention containing reactive functional groups (such as, without limitation, carboxy, hydroxy, and amino moieties) also include protected derivatives thereof. "Protected derivatives" are those compounds in which a reactive site or sites are blocked with one ore more protecting groups. Suitable protecting groups for carboxy moieties include benzyl, tert-butyl, and the like. Suitable protecting groups for amino and amido groups include acetyl, tert-butoxycarbonyl, benzyloxycarbonyl, and the like. Suitable protecting groups for hydroxy include benzyl and the like. Other suitable protecting groups are well known to those of ordinary skill in the art and include those found in T. W. Greene, Protecting Groups in Organic Synthesis, John Wiley & Sons, Inc. 1981.

As used herein, the term "compound(s) of this invention" and similar terms refers to a compound of any one of formulas (I) through (IX), or Table 1, or a pharmaceutically acceptable salt, solvate, or clathrate thereof and also include protected derivatives thereof.

As used herein, the term "pharmaceutically acceptable salt," is a salt formed from an acid and a basic group of one of the compounds of any one of formulas (I) through (IX) or of Table 1. Illustrative salts include, but are not limited, to sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, and pamoate (*i.e*., 1,1'-methylene-bis-(2-hydroxy-3-naphthoate)) salts. The term "pharmaceutically acceptable salt" also refers to a salt prepared from a compound of any one of formulas (I) through (IX) or Table 1 having an acidic functional group, such as a carboxylic acid functional group, and a pharmaceutically acceptable inorganic or organic base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or trialkylamines; dicyclohexylamine; tributyl amine; pyridine; N-methyl,N-ethylamine; diethylamine; triethylamine; mono-, bis-, or tris-(2-hydroxy-lower alkyl amines), such as mono-, bis-, or tris-(2-hydroxyethyl)- amine, 2-hydroxy-tert-butylamine, or tris-(hydroxymethyl)methylamine, N, N,-di-lower alkyl-N-(hydroxy lower alkyl)-amines, such as N,N-dimethyl-N-(2-hydroxyethyl)- amine, or tri-(2-hydroxyethyl)amine; N-methyl-D-glucamine; and amino acids such as arginine, lysine, and the like. The term "pharmaceutically acceptable salt" also refers to a salt prepared from a compound of any one of formulas (I) through (IX) or Table 1 having a basic functional group, such as an amino functional group, and a pharmaceutically acceptable inorganic or organic acid. Suitable acids include, but are not limited to, hydrogen sulfate, citric acid, acetic acid, oxalic acid, hydrochloric acid, hydrogen bromide, hydrogen iodide, nitric acid, phosphoric acid, isonicotinic acid, lactic acid, salicylic acid, tartaric acid, ascorbic acid, succinic acid, maleic acid, besylic acid, fumaric acid, gluconic acid, glucaronic acid, saccharic acid, formic acid, benzoic acid, glutamic acid, methanesulfonic acid, ethanesulfonic acid, benzenesulfonic acid,and *p*-toluenesulfonic acid.

As used herein, the term "pharmaceutically acceptable solvate," is a solvate formed from the association of one or more solvent molecules to one or more molecules of a compound of any one of formulas (I) through (IX) or Table 1. The term solvate includes hydrates (*e.g*., hemi-hydrate, mono-hydrate, dihydrate, trihydrate, tetrahydrate, and the like).

As used herein, the term "clathrate" means a compound of the present invention or a salt thereof in the form of a crystal lattice that contains spaces (*e.g*., channels) that have a guest molecule (*e.g*., a solvent or water) trapped within.

As used herein, the term "asthma" means a pulmonary disease, disorder or condition characterized by reversible airway obstruction, airway inflammation, and increased airway responsiveness to a variety of stimuli.

"Immunosuppression" refers to impairment of any component of the immune system resulting in decreased immune function. This impairment may be measured by any conventional means including whole blood assays of lymphocyte function, detection of lymphocyte proliferation and assessment of the expression of T cell surface antigens. The antisheep red blood cell (SRBC) primary (IgM) antibody response assay (usually referred to as the plaque assay) is one specific method. This and other methods are described in Luster, M.I., Portier, C., Pait, D.G., White, K.L., Jr., Gennings, C., Munson, A.E., and Rosenthal, G.J. (1992). "Risk Assessment in Immunotoxicology I: Sensitivity and Predictability of Immune Tests." Fundam. Appl. Toxicol., 18, 200-210. Measuring the immune response to a T-cell dependent immunogen is another particularly useful assay (Dean, J.H., House, R.V., and Luster, M.I. (2001). "Immunotoxicology: Effects of, and Responses to, Drugs and Chemicals." In Principles and Methods of Toxicology: Fourth Edition (A.W. Hayes, Ed.), pp. 1415-1450, Taylor & Francis, Philadelphia, Pennsylvania).

The compounds of this invention can be used to treat subjects with immune disorders. As used herein, the term "immune disorder" and like terms means a disease, disorder or condition caused by the immune system of an animal, including autoimmune disorders. Immune disorders include those diseases, disorders or conditions that have an immune component and those that are substantially or entirely immune system-mediated. Autoimmune disorders are those wherein the animal's own immune system mistakenly attacks itself, thereby targeting the cells, tissues, and/or organs of the animal's own body. For example, the autoimmune reaction is directed against the nervous system in multiple sclerosis and the gut in Crohn's disease. In other autoimmune disorders such as systemic lupus erythematosus (lupus), affected tissues and organs may vary among individuals with the same disease. One person with lupus may have affected skin and joints whereas another may have affected skin, kidney, and lungs. Ultimately, damage to certain tissues by the immune system may be permanent, as with destruction of insulin-producing cells of the pancreas in Type 1 diabetes mellitus. Specific autoimmune disorders that may be ameliorated using the compounds and methods of this invention include without limitation, autoimmune disorders of the nervous system (*e.g*., multiple sclerosis, myasthenia gravis, autoimmune neuropathies such as Guillain-Barré, and autoimmune uveitis), autoimmune disorders of the blood (*e.g*., autoimmune hemolytic anemia, pernicious anemia, and autoimmune thrombocytopenia), autoimmune disorders of the blood vessels (*e.g*., temporal arteritis, anti-phospholipid syndrome, vasculitides such as Wegener's granulomatosis, and Behcet's disease), autoimmune disorders of the skin (*e.g*., psoriasis, dermatitis herpetiformis, pemphigus vulgaris, and vitiligo), autoimmune disorders of the gastrointestinal system (*e.g*., Crohn's disease, ulcerative colitis, primary biliary cirrhosis, and autoimmune hepatitis), autoimmune disorders of the endocrine glands (*e.g*., Type 1 or immune-mediated diabetes mellitus, Grave's disease. Hashimoto's thyroiditis, autoimmune oophoritis and orchitis, and autoimmune disorder of the adrenal gland); and autoimmune disorders of multiple organs (including connective tissue and musculoskeletal system diseases) (*e.g*., rheumatoid arthritis, systemic lupus erythematosus, scleroderma, polymyositis, dermatomyositis, spondyloarthropathies such as ankylosing spondylitis, and Sjogren's syndrome). In addition, other immune system mediated diseases, such as graft-versus-host disease and allergic disorders, are also included in the definition of immune disorders herein. Because a number of immune disorders are caused by inflammation, there is some overlap between disorders that are considered immune disorders and inflammatory disorders. For the purpose of this invention, in the case of such an overlapping disorder, it may be considered either an immune disorder or an inflammatory disorder. "Treatment of an immune disorder" herein refers to administering a compound or a composition of the invention to a subject, who has an immune disorder, a symptom of such a disease or a predisposition towards such a disease, with the purpose to cure, relieve, alter, affect, or prevent the autoimmune disorder, the symptom of it, or the predisposition towards it.

As used herein, the term "allergic disorder" means a disease, condition or disorder associated with an allergic response against normally innocuous substances. These substances may be found in the environment (such as indoor air pollutants and aeroallergens) or they may be non-environmental (such as those causing dermatological or food allergies). Allergens can enter the body through a number of routes, including by inhalation, ingestion, contact with the skin or injection (including by insect sting). Many allergic disorders are linked to atopy, a predisposition to generate the allergic antibody IgE. Because IgE is able to sensitize mast cells anywhere in the body, atopic individuals often express disease in more than one organ. For the purpose of this invention, allergic disorders include any hypersensitivity that occurs upon re-exposure to the sensitizing allergen, which in turn causes the release of inflammatory mediators. Allergic disorders include without limitation, allergic rhinitis (*e.g*., hay fever), sinusitis, rhinosinusitis, chronic or recurrent otitis media, drug reactions, insect sting reactions, latex reactions, conjunctivitis, urticaria, anaphylaxis and anaphylactoid reactions, atopic dermatitis, asthma and food allergies.

The compounds of this invention can be used to prevent or to treat subjects with inflammatory disorders. As used herein, an "inflammatory disorder" means a disease, disorder or condition characterized by inflammation of body tissue or having an inflammatory component. These include local inflammatory responses and systemic inflammation. Examples of such inflammatory disorders include: transplant rejection, including skin graft rejection; chronic inflammatory disorders of the joints, including arthritis, rheumatoid arthritis, osteoarthritis and bone diseases associated with increased bone resorption; inflammatory bowel diseases such as ileitis, ulcerative colitis, Barrett's syndrome, and Crohn's disease; inflammatory lung disorders such as asthma, adult respiratory distress syndrome, and chronic obstructive airway disease; inflammatory disorders of the eye including corneal dystrophy, trachoma, onchocerciasis, uveitis, sympathetic ophthalmitis and endophthalmitis; chronic inflammatory disorders of the gums, including gingivitis and periodontitis; tuberculosis; leprosy; inflammatory diseases of the kidney including uremic complications, glomerulonephritis and nephrosis; inflammatory disorders of the skin including sclerodermatitis, psoriasis and eczema; inflammatory diseases of the central nervous system, including chronic demyelinating diseases of the nervous system, multiple sclerosis, AIDS-related neurodegeneration and Alzheimer's disease, infectious meningitis, encephalomyelitis, Parkinson's disease, Huntington's disease, amyotrophic lateral sclerosis and viral or autoimmune encephalitis; autoimmune disorders, immune-complex vasculitis, systemic lupus and erythematodes; systemic lupus erythematosus (SLE); and inflammatory diseases of the heart such as cardiomyopathy, ischemic heart disease hypercholesterolemia, atherosclerosis); as well as various other diseases with significant inflammatory components, including preeclampsia; chronic liver failure, brain and spinal cord trauma, cancer). There may also be a systemic inflammation of the body, exemplified by gram-positive or gram negative shock, hemorrhagic or anaphylactic shock, or shock induced by cancer chemotherapy in response to pro-inflammatory cytokines, *e.g*., shock associated with pro-inflammatory cytokines. Such shock can be induced, *e.g*., by a chemotherapeutic agent used in cancer chemotherapy. "Treatment of an inflammatory disorder" herein refers to administering a compound or a composition of the invention to a subject, who has an inflammatory disorder, a symptom of such a disorder or a predisposition towards such a disorder, with the purpose to cure, relieve, alter, affect, or prevent the inflammatory disorder, the symptom of it, or the predisposition towards it.

An "effective amount" is the quantity of compound in which a beneficial outcome is achieved when the compound is administered to a subject or alternatively, the quantity of compound that possess a desired activity *in-vivo* or *in-vitro.* In the case of inflammatory disorders and autoimmune disorders, a beneficial clinical outcome includes reduction in the extent or severity of the symptoms associated with the disease or disorder and/or an increase in the longevity and/or quality of life of the subject compared with the absence of the treatment. The precise amount of compound administered to a subject will depend on the type and severity of the disease or condition and on the characteristics of the subject, such as general health, age, sex, body weight and tolerance to drugs. It will also depend on the degree, severity and type of inflammatory disorder or autoimmune disorder or the degree of immunosuppression sought. The skilled artisan will be able to determine appropriate dosages depending on these and other factors. Effective amounts of the disclosed compounds typically range between about 1 mg/m² per day and about 10 grams/m² per day, and preferably between 10 mg/m² per day and about 1 gram/m².

The compounds of the invention may contain one or more chiral centers and/or double bonds and, therefore, exist as stereoisomers, such as double-bond isomers (*i.e*., geometric isomers), enantiomers, or diastereomers. According to this invention, the chemical structures depicted herein, including the compounds of this invention, encompass all of the corresponding compounds' enantiomers and stereoisomers, that is, both the stereomerically pure form (*e.g*., geometrically pure, enantiomerically pure, or diastereomerically pure) and enantiomeric, diastereomeric, and geometric isomeric mixtures. In some cases, one enantiomer, diastereomer, or geometric isomer will possess superior activity or an improved toxicity or kinetic profile compared to others. In those cases, such enantiomers, diastereomers, and geometric isomers of a compound of this invention are preferred.

The term "inhibit production of IL-2" and like terms means inhibiting IL-2 synthesis (*e.g*. by inhibiting transcription (mRNA expression), or translation (protein expression)) and/or inhibiting IL-2 secretion in a cell that has the ability to produce and/or secrete IL-2 (*e.g*., T lymphocyte). Likewise, the term "inhibiting production of IL-4, IL-5, IL-13, GM-CSF, TNF-α or INF-γ means inhibiting the synthesis (*e.g*. by inhibiting transcription, or translation) and/or inhibiting the secretion in a cell that has the ability to produce and/or secrete these cytokines.

As used herein, a composition that "substantially" comprises a compound means that the composition contains more than about 80% by weight, more preferably more than about 90% by weight, even more preferably more than about 95% by weight, and most preferably more than about 97% by weight of the compound.

As used herein, a composition that is "substantially free" of a compound means that the composition contains less than about 20% by weight, more preferably less than about 10% by weight, even more preferably less than about 5% by weight, and most preferably less than about 3% by weight of the compound.

As used herein, a reaction that is "substantially complete" means that the reaction contains more than about 80% by weight of the desired product, more preferably more than about 90% by weight of the desired product, even more preferably more than about 95% by weight of the desired product, and most preferably more than about 97% by weight of the desired product.

As used herein, a racemic mixture means about 50% of one enantiomer and about 50% of is corresponding enantiomer relative to all chiral centers in the molecule. The invention encompasses all enantiomerically-pure, enantiomerically-enriched, diastereomerically pure, diastereomerically enriched, and racemic mixtures of the compounds of any one of formulas (I) through (IX) or Table 1.

Enantiomeric and diastereomeric mixtures can be resolved into their component enantiomers or stereoisomers by well known methods, such as chiral-phase gas chromatography, chiral-phase high performance liquid chromatography, crystallizing the compound as a chiral salt complex, or crystallizing the compound in a chiral solvent. Enantiomers and diastereomers can also be obtained from diastereomerically- or enantiomerically-pure intermediates, reagents, and catalysts by well known asymmetric synthetic methods.

When administered to a patient, *e.g*., to a non-human animal for veterinary use or for improvement of livestock, or to a human for clinical use, the compounds of the invention are typically administered in isolated form or as the isolated form in a pharmaceutical composition. As used herein, "isolated" means that the compounds of the invention are separated from other components of either (a) a natural source, such as a plant or cell, preferably bacterial culture, or (b) a synthetic organic chemical reaction mixture. Preferably, via conventional techniques, the compounds of the invention are purified. As used herein, "purified" means that when isolated, the isolate contains at least 95%, preferably at least 98%, of a single compound of the invention by weight of the isolate.

Only those choices and combinations of substituents that result in a stable structure are contemplated. Such choices and combinations will be apparent to those of ordinary skill in the art and may be determined without undue experimentation.

The invention can be understood more fully by reference to the following detailed description and illustrative examples, which are intended to exemplify non-limiting embodiments of the invention.

### SPECIFIC EMBODIMENTS

The invention relates to compounds and pharmaceutical compositions that are particularly useful for immunosuppression or to treat or prevent inflammatory conditions, immune disorders, and allergic disorders.

One embodiment of the invention relates to compounds of formula (I): 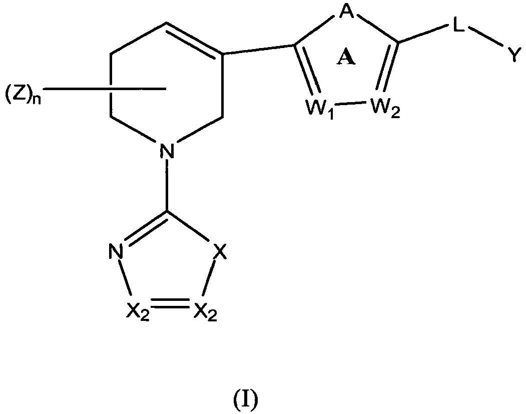 wherein A, L, Y, X, W₁, W₂, Z, X₂, and n are defined as above;
or a pharmaceutically acceptable salt, solvate, or clathrate thereof.

In some embodiments, the compound of formula (I) is not:
2,6-Difluoro-N-[4-(1-thiazol-2-yl-4-methyl-1,2,5,6-tetrahydro-pyridin-3-yl)-phenyl]-benzamide;
N-[4-(1-thiazol-2-yl-4-methyl-1,2,5,6-tetrahydro-pyridin-3-yl)-phenyl]-3-fluoro-isonicotinamide;
N-[4-(1-thiazol-2-yl-4-methyl-1,2,5,6-tetrahydro-pyridin-3-yl)-phenyl]-3-methyl-isonicotinamide;
N-[4-(1-thiazol-2-yl-4-methyl-1,2,5,6-tetrahydro-pyridin-3-yl)-phenyl]-3-fluoro-isonicotinamide;
2,6-Difluoro-N-[4-(1-oxazol-2-yl-4-methyl-1,2,5,6-tetrahydro-pyridin-3-yl)-phenyl]-benzamide;
N-[4-(1-oxazol-2-yl-4-methyl-1,2,5,6-tetrahydro-pyridin-3-yl)-phenyl]-3-fluoro-isonicotinamide;
2,6-Difluoro-N-[5-(1-thiazol-2-yl-4-methyl-1,2,5,6-tetrahydro-pyridin-3-yl)-pyridin-2-yl]-benzami de, hydrochloride;
N-[4-(1-thiazol-2-yl-4-methyl-1,2,5,6-tetrahydro-pyridin-3-yl)-phenyl]-3-fluoro-isonicotinamide, dihydrochloride;
2,6-Difluoro-N-[4-(1-thiazol-2-yl-4-methyl-1,2,5,6-tetrahydro-pyridin-3-yl)-phenyl]-benzamide, hydrochloride;
2,6-Difluoro-N-[4-(1-thiazol-2-yl-4-methyl-1,2,5,6-tetrahydro-pyridin-3-yl)-phenyl]-benzamide; 5-Methyl-pyrimidine-4-carboxylic acid
[4-(1-thiazol-2-yl-4-methyl-1,2,5,6-tetrahydro-pyridin-3-yl)-phenyl]-amide;
2,6-Difluoro-N-[4-(1-thiazol-2-yl-4-formyl-1,2,5,6-tetrahydro-pyridin-3-yl)-phenyl]-benzamide;
2,6-Difluoro-N-[4-(1-oxazol-2-yl-4-methyl-1,2,5,6-tetrahydro-pyridin-3-yl)-phenyl]-benzamide, hydrochloride;
2,6-Difluoro-N-{4-[1-(4-methyl-oxazol-2-yl)-4-methyl-1,2,5,6-tetrahydro-pyridin-3-yl]-phenyl}-benzamide;
5-Methyl-pyrimidine-4-carboxylic acid {4-[1-(4,5-dimethyl-oxazol-2-yl)-4-methyl-1,2,5,6-tetrahydro-pyridin-3-yl]-phenyl}-amide;
2,6-Difluoro-N-{4-[1-(4,5-dimethyl-oxazol-2-yl)-4-methyl-1,2,5,6-tetrahydro-pyridin-3-yl]-phenyl }-benzamide;
N-{4-[1-(1H-tetrazol-5-yl)-4-methyl-1,2,5,6-tetrahydro-pyridin-3-yl]-phenyl}-3-fluoro-isonicotinamide;
N-{4-[1-(1-methyl-1H-tetrazol-5-yl)-4-methyl-1,2,5,6-tetrahydro-pyridin-3-yl]-phenyl}-3-fluoro-isonicotinamide;
N-{4-[1-(2-methyl-2H-tetrazol-5-yl)-4-methyl-1,2,5,6-tetrahydro-pyridin-3-yl]-phenyl}-3-fluoro-isonicotinamide;
2,6-Difluoro-N-{4-[1-(2-methyl-2H-tetrazol-5-yl)-4-methyl-1,2,5,6-tetrahydro-pyridin-3-yl]-pheny 1}-benzamide;
2,6-Difluoro-N-{4-[1-(1H-tetrazol-5-yl)-4-methyl-1,2,5,6-tetrahydro-pyridin-3-yl]-phenyl}-benzamide;
2,6-Difluoro-N-{4-[1-(1-methyl-1H-tetrazol-5-yl)-4-methyl-1,2,5,6-tetrahydro-pyridin-3-yl]-pheny l}-benzamide;
2,6-Difluoro-N-[5-(1-thiazol-2-yl-4-methyl-1,2,5,6-tetrahydro-pyridin-3-yl)-pyrimidin-2-yl]-benzamide; or
2,6-Difluoro-N-[5-(1-thiazol-2-yl-4-methyl-1,2,5,6-tetrahydro-pyridin-3-yl)-pyrimidin-2-yl]-benzamide, hydrochloride.

Another embodiment of the invention relates to compounds of formula (II): 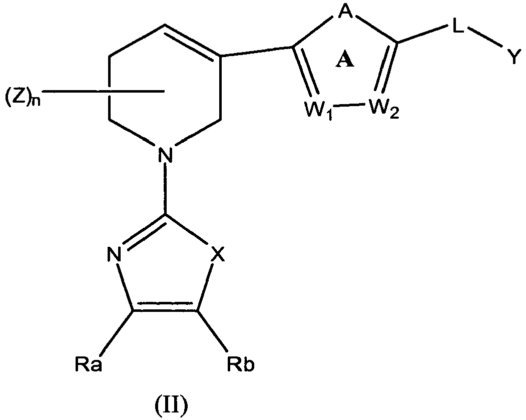 wherein A, L, Y, X, W₁, W₂, Z, Rₐ, R_{b}, and n are defined as above; or a pharmaceutically acceptable salt, solvate, or clathrate thereof.

Another embodiment of the invention relates to compounds of formula (III): 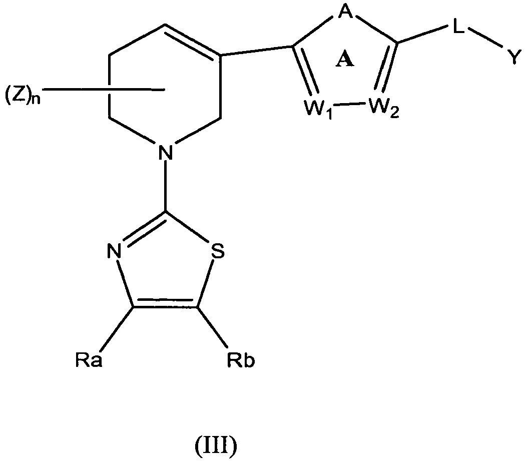 wherein A, L, Y, W₁, W₂, Z, Rₐ, R_{b}, and n are defined as above;
or a pharmaceutically acceptable salt, solvate, or clathrate thereof.

Another embodiment of the invention relates to compounds of formula (IV): 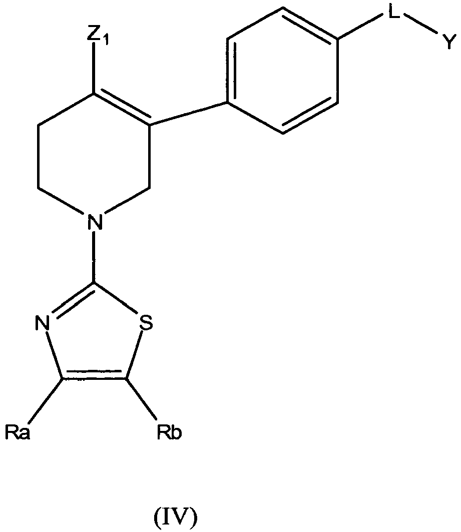 wherein L, Y, Z₁, Rₐ, and R_{b} are defined as above; or a pharmaceutically acceptable salt, solvate, or clathrate thereof.

Another embodiment of the invention relates to compounds of formula (V): 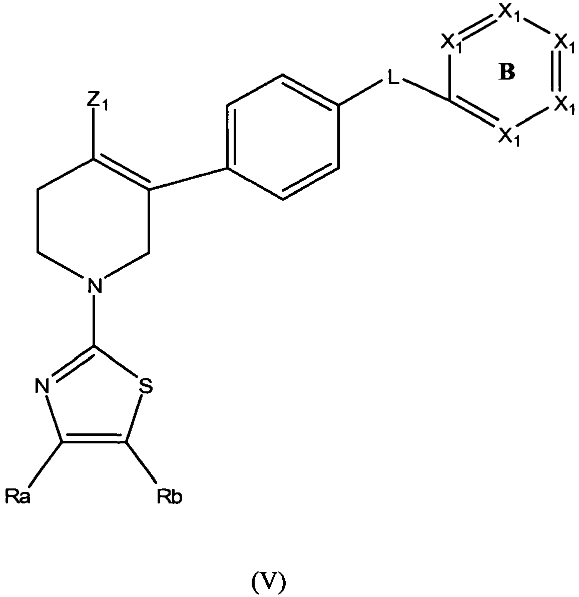 wherein L, X₁, Z₁, Rₐ, and R_{b} are defined as above; or a pharmaceutically acceptable salt, solvate, or clathrate thereof.

Another embodiment of the invention relates to compounds of formula (VI): 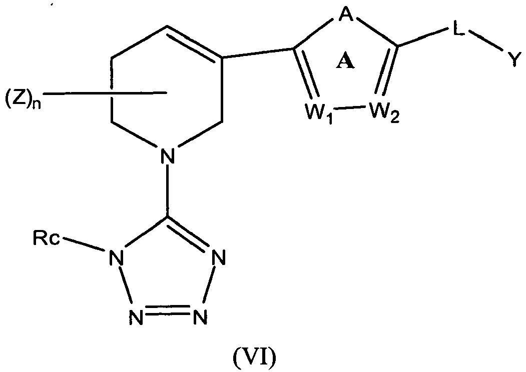 wherein A, L, Y, X, W₁, W₂, Z, R_{c} and n are defined as above; or a pharmaceutically acceptable salt, solvate, or clathrate thereof.

Another embodiment of the invention relates to compounds of formula (VII): 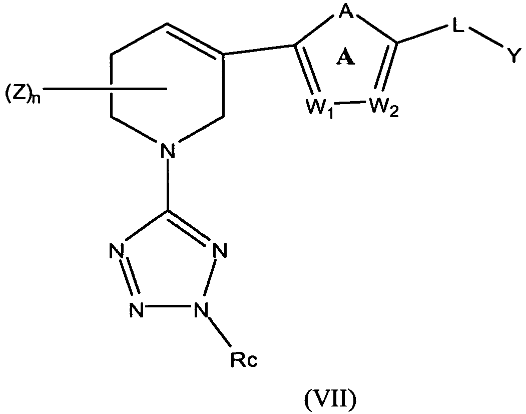 wherein A, L, Y, X, W₁, W₂, Z, R_{c}, and n are defined as above; or a pharmaceutically acceptable salt, solvate, or clathrate thereof.

Another embodiment of the invention relates to compounds of formula (VIII): 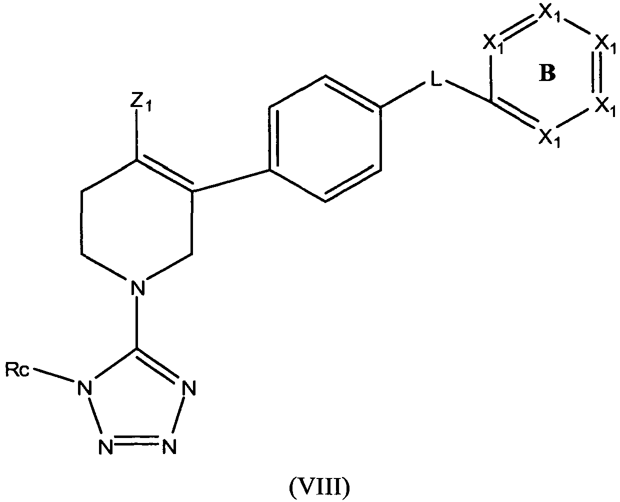 wherein L, X₁, Z₁, and R_{c} are defined as above; or a pharmaceutically acceptable salt, solvate, or clathrate thereof.

Another embodiment of the invention relates to compounds of formula (IX): 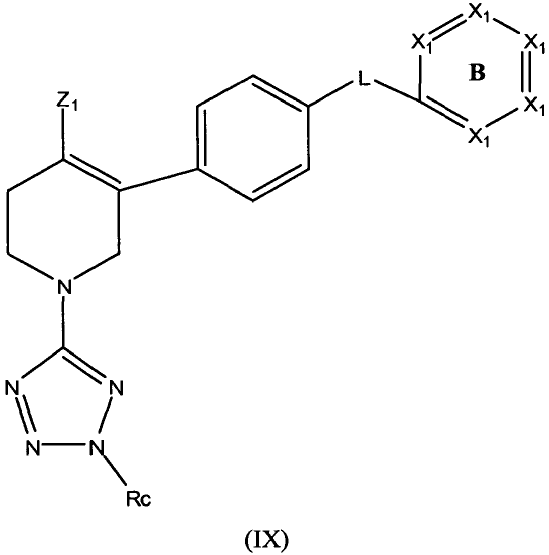 wherein L, X₁, Z₁, and R_{c} are defined as above; or a pharmaceutically acceptable salt, solvate, or clathrate thereof.

In one embodiment, in compounds represented by formula (I), (II), (III), (IV), (V), (VI), (VII), (VIII), or (IX), L is-NRCH₂-, -CH₂NR-, NR-C(O)-, or -C(O)-NR-; R, for each occurrence, is independently -H, alkyl, -C(O)-R₁₃, or -C(O)OR₁₃; and R₁₃, for each occurrence, is independently -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl.

In one aspect, in compounds represented by formula (I), (II), (III), (IV), (V), (VI), (VII), (VIII), or (IX), R is -H. In a further aspect, L is -NH-C(O)- or -C(O)-NH-. In one aspect, L is -NH-C(O)-.

In one embodiment, in compounds represented by formula (II), (III), (IV), or (V), Rₐ and R_{b} are both -H.

In one embodiment, in compounds represented by formula (II), (III), (IV), or (V), one of Rₐ or R_{b} is C₁-C₄ alkyl.

In one embodiment, in compounds represented by formula (I), (II), (III), or (IV), Y is an optionally substituted aryl or an optionally substituted heteroaryl.

In one embodiment, in compounds represented by formula (I), (II), (III), (IV), (VI), or (VII), Y is an optionally substituted phenyl, an optionally substituted oxazolyl, an optionally substituted furanyl, an optionally substitute pyrazolyl, an optionally substituted pyridinyl, an optionally substituted pyrimidinyl, an optionally substituted pyridazinyl, an optionally substituted thiadiazolyl, or an optionally substituted thiophenyl.

In one embodiment, in compounds represented by formula (I), (II), (III), (IV), (VI), or (VII), Y is an optionally substituted phenyl, an optionally substituted oxazolyl, an optionally substituted furanyl, an optionally substitute pyrazolyl, an optionally substituted pyridinyl, an optionally substituted pyridazinyl, an optionally substituted thiadiazolyl, or an optionally substituted thiophenyl. In one aspect, Y is unsubstituted. In another aspect, Y is an optionally substituted phenyl, an optionally substitute pyrimidinyl, or an optionally substituted pyridinyl. In another aspect, Y is an optionally substituted phenyl or an optionally substituted pyridinyl. In a further aspect, Y is substituted with one to two substituents. In another aspect, Y is substituted with one to two substituents and the one to two substituents are each independently a lower alkyl or a halo. In an additional aspect, Y is a difluorophenyl. In another aspect, Y is an optionally substituted thiadiazolyl. In a further aspect, Y is an optionally substituted thiophenyl. In another aspect, Y is an optionally substituted pyridazinyl. In a further aspect, Y is substituted with one methyl group.

In one embodiment, in compounds represented by formula (I), (II), (III), (IV), (VI), or (VII), Y is an optionally substituted alkyl, an optionally substituted cycloalkyl, an optionally substituted alkenyl, an optionally substituted cycloalkenyl, C(O)OR₅, or an optionally substituted heterocyclyl. In one aspect, Y is unsubstituted. In one aspect, Y is substituted with amino, lower dialkyl amino, lower alkyl amino, lower alkyl, halo, haloalkyl, nitro, lower alkoxy, or lower alkyl sulfanyl. In one aspect, Y is substituted with amino, dimethylamino, methyl, trifluoromethyl, chloro, bromo, fluoro, nitro, methoxy, or methyl sufanyl. In one aspect, Y is substituted with a halo.

In one embodiment, in compounds represented by formula (I), (II), (III), (IV), (VI), or (VII), Y is optionally substituted alkyl or optionally substituted cycloallcyl.

In one embodiment, in compounds represented by formula (I), (II), (III), (IV), (VI), or (VII), Y is optionally substituted alkenyl or optionally substituted cycloallcenyl.

In one embodiment, in compounds represented by formula (I), (II), (III), (IV), (VI), or (VII), Y is optionally substituted heterocyclyl.

In one embodiment, in compounds represented by formula (I), (II), (III), (IV), (VI), or (VII), Y is an optionally substituted C1-C6 alkyl or C3-C6 cycloalkyl. In one aspect, Y is an optionally substituted C1-C3 alkyl.

In one embodiment, in compounds represented by formula (I), (II), (III), (IV), (VI), or (VII), Y is -CH=CR₅R₅. In one aspect, each R₅ is independently -H or lower alkyl.

In one embodiment, in compounds represented by formula (I), (II), (III), (VI) or (VII), Ring A is

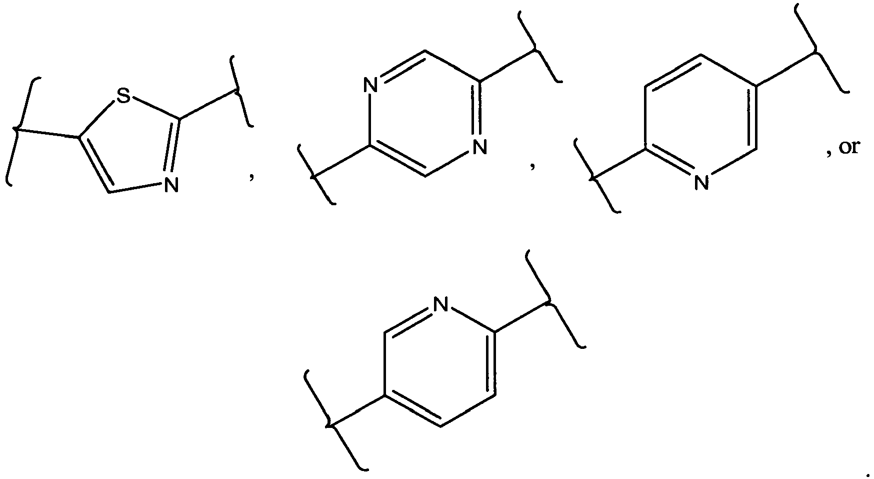

In one embodiment, in compounds represented by formula (I), (II), (III), (VI) or (VII), Z for each occurrence is, independently, an alkyl, halo, or -C(O)R₅ In one aspect, Z is an optionally substituted lower alkyl, or -C(O)R₅. In another aspect, Z is CH₃ or C(O)H.

In one embodiment, in compounds represented by formula (I), (II), (III), (VI) or (VII), n is 1.

In one embodiment, in compounds represented by formula (I), (II), (III), (VI) or (VII), n is 2.

In one embodiment, in compounds represented by formula (I) or (II), X is -O-.

In one embodiment, in compounds represented by formula (I) or (II), X is -S-.

In one embodiment, in compounds represented by formula (I) or (II), X is -NR_{c}-.

In one embodiment, in compounds represented by formula (I), each of X₂ is -N-.

In one embodiment, in compounds represented by formula (I), one of X₂ is N-.

In one embodiment, in compounds represented by formula (IV), (V), (XIII), or (IX), Z₁ for each occurrence is, independently, an optionally substituted alkyl, halo, or -C(O)R₅. In one aspect, Z₁ is an optionally substituted lower alkyl, or -C(O)R₅. In another aspect, Z₁ is CH₃ or C(O)H.

In one embodiment, in compounds represented by formula (V), (XIII), or (IX), Ring B is 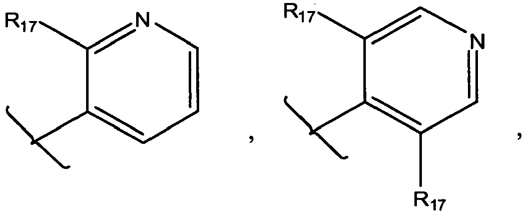 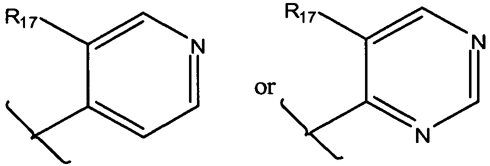 wherein R₁₇ is an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, -OR₅, -SR₅, -NR₆R₇, -C(O)NR₆R₇, -NR₅C(O)R₅, -C(O)R₅, -C(O)OR₅, -OC(O)R₅, -C(O)SR₅, -SC(O)R₅, -C(S)NR₆R₇, -NR₅C(S)R₅, -C(S)R₅, -C(S)OR₅, -OC(S)R₅, -C(S)SR₅, -SC(S)R₅, -C(NR₈)NR₆R₇, -NR₅C(NR₈)R₅, -C(NR₈)R₅, -C(NR₈)OR₅, -OC(NR₈)R₅, -C(NR₈)SR₅, -SC(NR₈)R₅, -OC(O)OR₅, -OC(O)NR₆R₇, -NR₅C(O)OR₅, -NR₅C(O)NR₆R₇, -SC(O)OR₅, -SC(O)NR₆R₇, -SC(O)SR₅, -NR₅C(O)SR₅, -OC(O)SR₅, -OC(S)OR₅, -OC(S)NR₆R₇, -NR₅C(S)OR₅, -NR₅C(S)NR₆R₇, -SC(S)OR₅, -SC(S)NR₆R₇, -SC(S)SR₅, -NR₅C(S)SR₅, -OC(S)SR₅, -OC(NR₈)OR₅, -OC(NR₈)NR₆R₇, -NR₅C(NR₈)OR₅, -NR₅C(NR₈)NR₆R₇, -SC(NR₈)OR₅, -SC(NR₈)NR₆R₇, -SC(NR₈)SR₅, -NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, -S(O)ₚOR₅, -OS(O)ₚR₅, or -OS(O)OR₅. In one aspect, R₁₇ is optionally substituted lower alkyl, halo, or -OR₅. In another aspect, R₁₇ is -CH₃ or -F.

In one embodiment, in compounds represented by formula (V), (XIII), or (IX), Ring B is 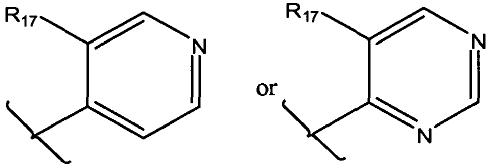 wherein R₁₇ is an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, -OR₅, -SR₅, -NR₆R₇, -C(O)NR₆R₇, -NR₅C(O)R₅, -C(O)R₅, -C(O)OR₅, -OC(O)R₅, -C(O)SR₅, -SC(O)R₅, -C(S)NR₆R₇, -NR₅C(S)R₅, -C(S)R₅, -C(S)OR₅, -OC(S)R₅, -C(S)SR₅, -SC(S)R₅, -C(NR₈)NR₆R₇, -NR₅C(NR₈)R₅, -C(NR₈)R₅, -C(NR₈)OR₅, -OC(NR₈)R₅, -C(NR₈)SR₅, -SC(NR₈)R₅, -OC(O)OR₅, -OC(O)NR₆R₇, -NR₅C(O)OR₅, -NR₅C(O)NR₆R₇, -SC(O)OR₅, -SC(O)NR₆R₇, -SC(O)SR₅, -NR₅C(O)SR₅, -OC(O)SR₅, -OC(S)OR₅, -OC(S)NR₆R₇, -NR₅C(S)OR₅, -NR₅C(S)NR₆R₇, -SC(S)OR₅, -SC(S)NR₆R₇, -SC(S)SR₅, -NR₅C(S)SR₅, -OC(S)SR₅, -OC(NR₈)OR₅, -OC(NR₈)NR₆R₇, -NR₅C(NR₈)OR₅, -NR₅C(NR₈)NR₆R₇, -SC(NR₈)OR₅, -SC(NR₈)NR₆R₇, -SC(NR₈)SR₅, -NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, -S(O)ₚOR₅, -OS(O)ₚR₅, or -OS(O)OR₅. In one aspect, R₁₇ is optionally substituted lower alkyl, halo, or -OR₅. In another aspect, R₁₇ is -CH₃ or -F.

In one embodiment, in compounds represented by formula (V), (XIII), or (IX), Ring B is 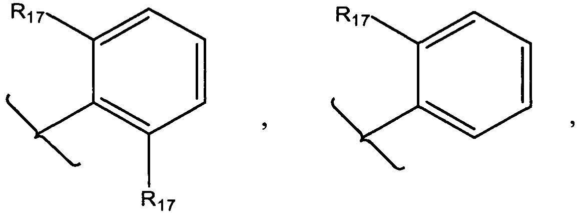 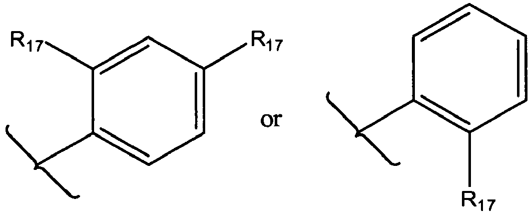 wherein each R₁₇ is independently, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, -OR₅, -SR₅, -NR₆R₇, -C(O)NR₆R₇, -NR₅C(O)R₅, -C(O)R₅, -C(O)OR₅, -OC(O)R₅, -C(O)SR₅, -SC(O)R₅, -C(S)NR₆R₇, -NR₅C(S)R₅, -C(S)R₅, -C(S)OR₅, -OC(S)R₅, -C(S)SR₅, -SC(S)R₅, -C(NR₈)NR₆R₇, -NR₅C(NR₈)R₅, -C(NR₈)R₅, -C(NR₈)OR₅, -OC(NR₈)R₅, -C(NR₈)SR₅, -SC(NR₈)R₅, -OC(O)OR₅, -OC(O)NR₆R₇, -NR₅C(O)OR₅, -NR₅C(O)NR₆R₇, -SC(O)OR₅, -SC(O)NR₆R₇, -SC(O)SR₅, -NR₅C(O)SR₅, -OC(O)SR₅, -OC(S)OR₅, -OC(S)NR₆R₇, -NR₅C(S)OR₅, -NR₅C(S)NR₆R₇, -SC(S)OR₅, -SC(S)NR₆R₇, -SC(S)SR₅, -NR₅C(S)SR₅, -OC(S)SR₅, -OC(NR₈)OR₅, -OC(NR₈)NR₆R₇, -NR₅C(NR₈)OR₅, -NR₅C(NR₈)NR₆R₇, -SC(NR₈)OR₅, -SC(NR₈)NR₆R₇, -SC(NR₈)SR₅, -NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, -S(O)ₚOR₅, -OS(O)ₚR₅, or -OS(O)OR₅.

In one embodiment, in compounds represented by formula (V), (XIII), or (IX), Ring B is 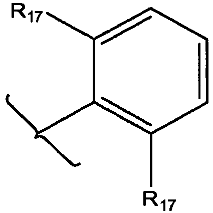 wherein each R₁₇ is independently, an optionally substituted alkyl, an optionally substituted alkenyl, an optionally substituted alkenyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, -OR₅, -SR₅, -NR₆R₇, -C(O)NR₆R₇, -NR₅C(O)R₅, -C(O)R₅, -C(O)OR₅, -OC(O)R₅, -C(O)SR₅, -SC(O)R₅, -C(S)NR₆R₇, -NR₅C(S)R₅, -C(S)R₅, -C(S)OR₅, -OC(S)R₅, -C(S)SR₅, -SC(S)R₅, -C(NR₈)NR₆R₇, -NR₅C(NR₈)R₅, -C(NR₈)R₅, -C(NR₈)OR₅, -OC(NR₈)R₅, -C(NR₈)SR₅, -SC(NR₈)R₅, -OC(O)OR₅, -OC(O)NR₆R₇, -NR₅C(O)OR₅, -NR₅C(O)NR₆R₇, -SC(O)OR₅, -SC(O)NR₆R₇, -SC(O)SR₅, -NR₅C(O)SR₅, -OC(O)SR₅, -OC(S)OR₅, -OC(S)NR₆R₇, -NR₅C(S)OR₅, -NR₅C(S)NR₆R₇, -SC(S)OR₅, -SC(S)NR₆R₇, -SC(S)SR₅, -NR₅C(S)SR₅, -OC(S)SR₅, -OC(NR₈)OR₅, -OC(NR₈)NR₆R₇, -NR₅C(NR₈)OR₅, -NR₅C(NR₈)NR₆R₇, -SC(NR₈)OR₅, -SC(NR₈)NR₆R₇, -SC(NR₈)SR₅, -NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, -S(O)ₚOR₅, -OS(O)ₚR₅, or -OS(O)OR₅.

In one aspect, each R₁₇ is independently optionally substituted lower alkyl, halo, or -OR₅. In another aspect, each R₁₇ is -F.

In one embodiment, in compounds represented by formula (VIII) or (IX), R_{c} is -H or methyl. In one aspect, R_{c} is methyl.

In one embodiment, the compound is selected from the group consisting of:
N-(4-(4-formyl-1-(thiazol-2-yl)-1,2,5,6-tetrahydropyridin-3-yl)phenyl)-5-methylpyrimidine-4-carboxamide;
2,6-difluoro-N-(5-(4-methyl-1-(2-methyl-2H-tetrazol-5-yl)-1,2,5,6-tetrahydropyridin-3-yl)py razin-2-yl)benzamide;
2,6-difluoro-N-(5-(4-methyl-1-(-methyl-1H-tetrazol-5-yl)-1,2,5,6-tetrahydropyridin-3-yl)py razin-2-yl)benzamide;
3-fluoro-N-(5-(4-methyl-1-(thiazol-2-yl)-1,2,5,6-tetrahydropyridin-3-yl)pyrazin-2-yl)isonicoti namide;
N-(2,6-difluorobenzyl)-4-(4-methyl-1-(thiazol-2-yl)-1,2,5,6-tetrahydropyridin-3 -yl)aniline;
2,6-difluoro-N-(5-(4-methyl-1-(2-methyl-2H-tetrazol-5-yl)-1,2,5,6-tetrahydropyridin-3-yl)py ridin-2-yl) benzamide;
N-(2,6-difluorobenzyl)-5-(4-methyl-1-(thiazol-2-yl)-1,2,5,6-tetrahydropyridin-3-yl)pyridin-2-amine dihydrochloride;
2-fluoro-N-(4-(4-methyl-1-(thiazol-2-yl)-1,2,5,6-tetrahydropyridin-3-yl)phenyl)-6-(trifluorom ethyl) benzamide;
N-(4-(4-methyl-1-(thiazol-2-yl)-1,2,5,6-tetrahydropyridin-3-yl)phenyl)-2-(trifluoromethyl)be nzamide;
2,6-dichloro-N-(4-(4-methyl-1-(thiazol-2-yl)-1,2,5,6-tetrahydropyridin-3-yl)phenyl)benzamid e;
2,6-difluoro-N-(6-(4-methyl-1-(thiazol-2-yl)-1,2,5,6-tetrahydropyridin-3-yl)pyridin-3-yl)benz amide;
2-fluoro-N-(4-(4-methyl-1-(thiazol-2-yl)-1,2,5,6-tetrahydropyridin-3-yl)phenyl)benzamide;
2-fluoro-6-hydroxy-N-(4-(4-methyl-1-(thiazol-2-yl)-1,2,5,6-tetrahydropyridin-3-yl)phenyl)be nzamide;
N-(4-(4-methyl-1-(thiazol-2-yl)-1,2,5,6-tetrahydropyridin-3-yl)phenyl)-2-(methylthio)nicotina mide;
2-chloro-N-(4-(4-methyl-1-(thiazol-2-yl)-1,2,5,6-tetrahydropyridin-3-yl)phenyl)butanamide;
2,4-difluoro-N-(4-(4-methyl-1-(thiazol-2-yl)-1,2,5,6-tetrahydropyridin-3-yl)phenyl)benzamid e;
N-(4-(4-methyl-1-(thiazol-2-yl)-1,2,5,6-tetrahydropyridin-3-yl)phenyl)-9H-fluorene-9-carbox amide;
N-(2'-chloro-5'-(thiazol-2-yl)biphenyl-4-yl)-2-fluoro-6-hydroxybenzamide;
2,4-difluoro-N-(4-(4-methyl-1-(thiazol-2-yl)-1,2,5,6-tetrahydropyridin-3-yl)phenyl)benzamid e hydrochloride;
2-chloro-6-fluoro-N-(5-(4-methyl-1-(thiazol-2-yl)-1,2,5,6-tetrahydropyridin-3-yl)pyrazin-2-yl )benzamide;
2,4-difluoro-N-(5-(4-methyl-1-(2-methyl-2H-tetrazol-5-yl)-1,2,5,6-tetrahydropyridin-3-yl)py ridin-2-yl)benzamide;
2,6-difluoro-N-(5-(4-methyl-1-(1-methyl-1H-imidazol-2-yl)-1,2,5,6-tetrahydropyridin-3-yl)p yridin-2-yl)benzamide;
2-chloro-6-fluoro-N-(5-(4-methyl-1-(2-methyl-2H-tetrazol-5-yl)-1,2,5,6-tetrahydropyridin-3-yl)pyridin-2-yl)benzamide;
2,4-difluoro-N-(5-(4-methyl-1-(2-methyl-2H-tetrazol-5-yl)-1,2,5,6-tetrahydropyridin-3-yl)py razin-2-yl)benzamide;
N-(5-(4-chloro-1-(2-methyl-2H-tetrazol-5-yl)-1,2,5,6-tetrahydropyridin-3-yl)pyridin-2-yl)-2, 6-difluorobenzamide;
N-(5-(4-chloro-1-(thiazol-2-yl)-1,2,5,6-tetrahydropyridin-3-yl)pyridin-2-yl)-2,6-difluorobenz amide;
2-chloro-6-fluoro-N-(5-(4-methyl-1-(2-methyl-2H-tetrazol-5-yl)-1,2,5,6-tetrahydropyridin-3-yl)pyridin-2-yl)benzamide;
3-methyl-N-(5-(4-methyl-1-(2-methyl-2H-tetrazol-5-yl)-1,2,5,6tetrahydropyridin-3-yl)pyridin -2-yl)but-2-enamide;
2-fluoro-N-(5-(4-methyl-1-(2-methyl-2H-tetrazol-5-yl)-1,2,5,6-tetrahydropyridin-3-yl)pyridin -2-yl)benzamide;
2,6-difluoro-N-(5-(4-methyl-1-(oxazol-2-yl)-1,2,5,6-tetrahydropyridin-3-yl)pyrazin-2-yl)benz amide;
2-fluoro-N-(5-(4-methyl-1-(thiazol-2-yl)-1,2,5,6-tetrahydropyridin-3-yl)pyrazin-2-yl)benzami de;
3,5-difluoro-N-(4-(4-methyl-1-(thiazol-2-yl)-1,2,5,6-tetrahydropyridin-3-yl)phenyl)isonicotin amide;
2-fluoro-N-(5-(4-methyl-1-(2-methyl-2H-tetrazol-5-yl)-1,2,5,6-tetrahydropyridin-3-yl)pyrazi n-2-yl)benzamide;
3,5-difluoro-N-(5-(4-methyl-1-(thiazol-2-yl)-1,2,5,6-tetrahydropyridin-3-yl)pyrazin-2-yl)isoni cotinamide hydrochloride;
2-chloro-6-fluoro-N-(5-(4-methyl-1-(2-methyl-2H-tetrazol-5-yl)-1,2,5,6-tetrahydropyridin-3-yl)pyrazin-2-yl)benzamide;
2-methyl-N-(4-(4-methyl-1-(thiazol-2-yl)-1,2,5,6-tetrahydropyridin-3-yl)phenyl)benzamide;
3,5-difluoro-N-(4-(4-methyl-1-(thiazol-2-yl)-1,2,5,6-tetrahydropyridin-3-yl)phenyl)isonicotin amide hydrochloride;
2,6-difluoro-N-(4-(4-isopropyl-1-(thiazol-2-yl)-1,2,5,6-tetrahydropyridin-3-yl)phenyl)benzam ide;
3,5-difluoro-N-(4-(4-isopropyl-1-(thiazol-2-yl)-1,2,5,6-tetrahydropyridin-3-yl)phenyl)isonico tinamide;
2,4-difluoro-N-(4-(4-isopropyl-1-(thiazol-2-yl)-1,2,5,6-tetrahydropyridin-3-yl)phenyl)benzam ide hydrochloride;
2,4-difluoro-N-(5-(4-methyl-1-(thiazol-2-yl)-1,2,5,6-tetrahydropyridin-3-yl)pyrazin-2-yl)benz amide;
2,4-difluoro-N-(5-(4-methyl-1-(4-methylthiazol-2-yl)-1,2,5,6-tetrahydropyridin-3-yl)pyrazin-2-yl)benzamide;
2,4-difluoro-N-(5-(4-methyl-1-(thiazol-2-yl)-1,2,5,6-tetrahydropyridin-3-yl)pyrazin-2-yl)benz amide hydrochloride;
2,6-difluoro-N-(4-(4-isopropyl-1-(thiazol-2-yl)-1,2,5,6-tetrahydropyridin-3-yl)phenyl)benzam ide hydrochloride;
2,6-difluoro-N-(5-(4-isopropyl-1-(thiazol-2-yl)-1,2,5,6-tetrahydropyridin-3-yl)pyrazin-2-yl)b enzamide;
2,6-difluoro-N-(5-(4-isopropyl-1-(2-methyl-2H-tetrazol-5-yl)-1,2,5,6-tetrahydropyridin-3-yl) pyrazin-2-yl)benzamide;
2-chloro-N-(4-(4-methyl-1-(thiazol-2-yl)-1,2,5,6-tetrahydropyridin-3-yl)phenyl)butanamide hydrochloride; or
2-chloro-N-(4-(4-methyl-1-(thiazol-2-yl)-1,2,5,6-tetrahydropyridin-3-yl)phenyl)propanamide hydrochloride;
or a pharmaceutically acceptable salt, solvate, or clathrate thereof.

In one embodiment, the compounds of the invention are not compounds disclosed in U.S. Patent Application No.11/699,002.

All of the features, specific embodiments and particular substituents disclosed herein may be combined in any combination. Each feature, embodiment or substituent disclosed in this specification may be replaced by an alternative feature, embodiment or substituent serving the same, equivalent, or similar purpose. In the case of chemical compounds, specific values for variables (*e.g*., values shown in the exemplary compounds disclosed herein) in any chemical formula disclosed herein can be combined in any combination resulting in a stable structure. Furthermore, specific values (whether preferred or not) for substituents in one type of chemical structure may be combined with values for other substituents (whether preferred or not) in the same or different type of chemical structure. Thus, unless expressly stated otherwise, each feature, embodiment or substituent disclosed is only an example of a generic series of equivalent or similar features, embodiments or substituents.

In another embodiment, the invention relates to pharmaceutical compositions that comprise a compound of any one of formulas (I) through (IX), or Table 1, or a pharmaceutically acceptable salt, solvate, or clathrate thereof, as an active ingredient, and a pharmaceutically acceptable carrier or vehicle. The compositions are useful for immunosuppression or to treat or prevent inflammatory conditions, allergic conditions and immune disorders.

In another embodiment, the invention relates to a compound represented by any one of formulas (I) through (IX), or Table 1, or a pharmaceutically acceptable salt, solvate, or clathrate thereof for use in treating or preventing inflammatory conditions, immune disorders, or allergic disorders in a patient in need thereof

In another embodiment, the invention relates to a pharmaceutical composition that comprises a compound represented by any one of formulas (I) through (IX), or in or Table 1, or a pharmaceutically acceptable salt, solvate, or clathrate thereoffor use in immunosuppression or for treating or preventing inflammatory conditions, immune disorders, or allergic disorders in a patient in need thereof.

In another embodiment, compounds of any one of formulas (I) through (IX), or Table 1, or a pharmaceutically acceptable salt, solvate, or clathrate thereof, are particularly useful inhibiting immune cell (*e.g.,* T-cells and/or B-cells) activation (*e.g*., activation in response to an antigen) and/or T cell and/or B cell proliferation. Indicators of immune cell activation include secretion of IL-2 by T cells, proliferation of T cells and/or B cells, and the like. In one embodiment, a compound of any one of formulas (I) through (IX) or Table 1, inhibits immune cell activation and/or T cell and/or B cell proliferation in a mammal (*e.g*., a human).

In another embodiment, compounds of any one of formulas (I) through (IX), or Table 1, or a pharmaceutically acceptable salt, solvate, or clathrate thereof, can inhibit the production of certain cytokines that regulate immune cell activation. For example, compounds of any one of formulas (I) through (IX), or Table 1, or a pharmaceutically acceptable salt, solvate, or clathrate thereof, can inhibit the production of IL-2, IL-4, IL-5, IL-13, GM-CSF, IFN-γ, TNF-α and combinations thereof. In one embodiment, a compound of any one of formulas (I) through (IX), or Table 1, inhibits cytokine production in a mammal (*e.g*., a human).

In another embodiment, compounds of any one of formulas (I) through (IX), or Table 1, or a pharmaceutically acceptable salt, solvate, or clathrate thereof, can modulate the activity of one or more ion channel involved in activation of immune cells, such as CRAC ion channels. In one embodiment, a compound of any one of formulas (I) through (IX) or Table 1 can inhibit the influx of calcium ions into an immune cell (*e.g*., T cells and/or B cells) by inhibiting the action of CRAC ion channels. In general, a decrease in I_{CRAC} current upon contacting a cell with a compound is one indicator that the compound inhibitions CRAC ion channels. I_{CRAC} current can be measured, for example, using a patch clamp technique, which is described in more detail in the examples below. In one embodiment, a compound of any one of formulas (I) through (IX) or Table 1 modulates an ion channel in a mammal (*e.g*., a human).

### EXEMPLARY COMPOUNDS OF THE INVENTION

Exemplary compounds of the invention are depicted in Table 1 below.

**Table 1**

| Compoun d No. | Structure | Chemical Name |
|---|---|---|
| 1 | 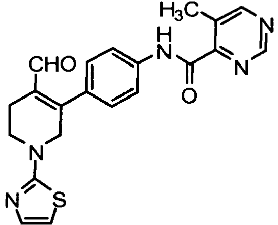 | N-(4-(4-formyl-1-(thiazol-2-yl)-1,2,5 ,6-tetrahydropyridin-3-yl)phenyl)-5-methylpyrimidine-4-carboxamide |
| 2 | 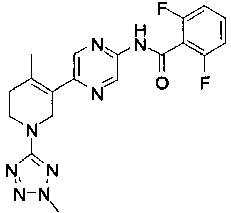 | 2,6-difluoro-N-(5-(4-methyl-1-(2-methyl-2H-tetrazol-5-yl)-1,2,5,6-tetrahydropyridin-3-yl)pyrazin-2-yl)benzamide |
| 3 | 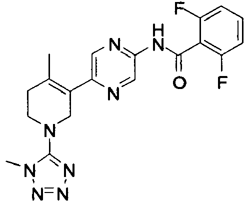 | 2,6-difluoro-N-(5-(4-methyl-1-(1-methyl-1H-tetrazol-5-yl)-1,2,5,6-tetrahydropyridin-3-yl)pyrazin-2-yl)benzamide |
| 4 | 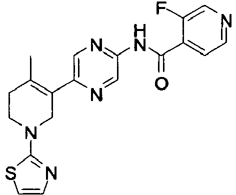 | 3-fluoro-N-(5-(4-methyl-1-(thiazol-2 -yl)-1,2,5,6-tetrahydropyridin-3-yl)pyrazin-2-yl)isonicotinamide |
| 5 | 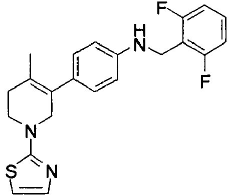 | N-(2,6-difluorobenzyl)-4-(4-methyl-1-(thiazol-2-yl)-1,2,5,6-tetrahydropy ridin-3-yl)aniline |
| 6 | 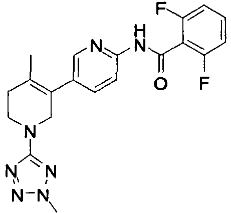 | 2,6-difluoro-N-(5-(4-methyl-1-(2-methyl-2H-tetrazol-5-yl)-1,2,5,6-tetrahydropyridin-3-yl)pyridin-2-yl) benzamide |
| 7 | 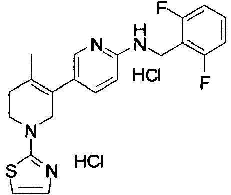 | N-(2,6-difluorobenzyl)-5-(4-methyl-1-(thiazol-2-yl)-1,2,5,6-tetrahydropy ridin-3-yl)pyridin-2-amine dihydrochloride |
| 8 | 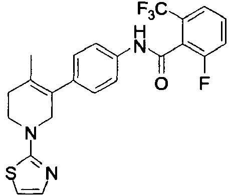 | 2-fluoro-N-(4-(4-methyl-1-(thiazol-2 -yl)-1,2,5,6-tetrahydropyridin-3-yl)phenyl)-6-(trifluoromethyl) benzamide |
| 9 | 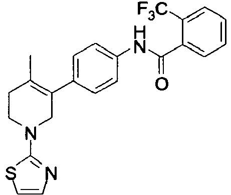 | N-(4-(4-methyl-1-(thiazol-2-yl)-1,2,5 ,6-tetrahydropyridin-3-yl)phenyl)-2-( trifluoromethyl)benzamide |
| 10 | 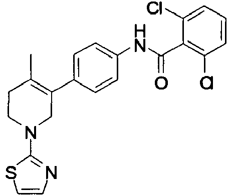 | 2,6-dichloro-N-(4-(4-methyl-1-(thiazol-2-yl)-1,2,5,6-tetrahydro pyridin-3-yl)phenyl)benzamide |
| 11 | 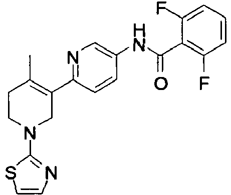 | 2,6-difluoro-N-(6-(4-methyl-1-(thiazol-2-yl)-1,2,5,6-tetrahydro pyridin-3-yl)pyridin-3-yl) benzamide |
| 12 | 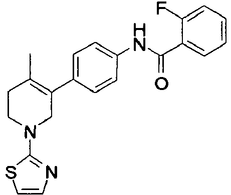 | 2-fluoro-N-(4-(4-methyl-1-(thiazol-2 -yl)-1,2,5,6-tetrahydropyridin-3-yl)phenyl)benzamide |
| 13 | 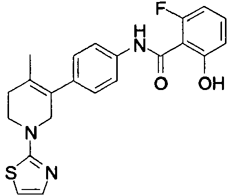 | 2-fluoro-6-hydroxy-N-(4-(4-methyl-1-(thiazol-2-yl)-1,2,5,6-tetrahydropy ridin-3-yl)phenyl)benzamide |
| 14 | 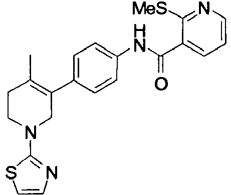 | N-(4-(4-methyl-1-(thiazol-2-yl)-1,2,5 ,6-tetrahydropyridin-3-yl)phenyl)-2-( methylthio)nicotinamide |
| 15 | 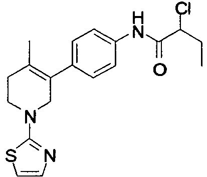 | 2-chloro-N-(4-(4-methyl-1-(thiazol-2 -yl)-1,2,5,6-tetrahydropyridin-3-yl)phenyl)butanamide |
| 16 | 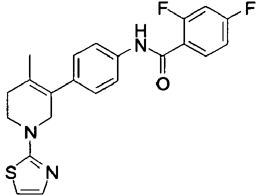 | 2,4-difluoro-N-(4-(4-methyl-1-(thiazol-2-yl)-1,2,5,6-tetrahydro pyridin-3-yl)phenyl)benzamide |
| 17 | 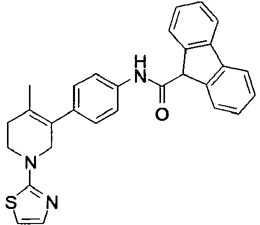 | N-(4-(4-methyl-1-(thiazol-2-yl)-1,2,5 ,6-tetrahydropyridin-3-yl)phenyl)-9H -fluorene-9-carboxamide |
| 18 | 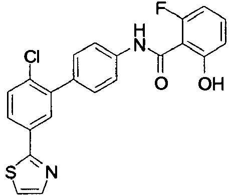 | N-(2'-chloro-5'-(thiazol-2-yl)bi phenyl-4-yl)-2-fluoro-6-hydroxy benzamide |
| 19 | 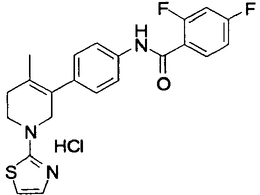 | 2,4-difluoro-N-(4-(4-methyl-1-(thiazol-2-yl)-1,2,5,6-tetrahydro pyridin-3-yl)phenyl)benzamide hydrochloride |
| 20 | 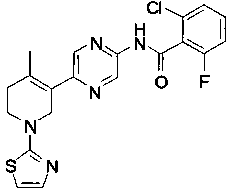 | 2-chloro-6-fluoro-N-(5-(4-methyl-1-(thiazol-2-yl)-1,2,5,6-tetrahydro pyridin-3-yl)pyrazin-2-yl) benzamide |
| 21 | 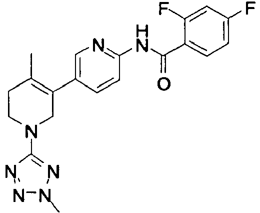 | 2,4-difluoro-N-(5-(4-methyl-1-(2-methyl-2H-tetrazol-5-yl)-1,2,5,6-tetrahydropyridin-3-yl)pyridin-2-yl)b enzamide |
| 22 | 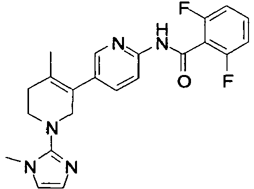 | 2,6-difluoro-N-(5-(4-methyl-1-(1-methyl-1H-imidazol-2-yl)-1,2,5,6-tetrahydropyridin-3-yl)pyridin-2-yl)b enzamide |
| 23 | 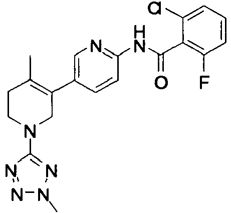 | 2-chloro-6-fluoro-N-(5-(4-methyl-1-(2-methyl-2H-tetrazol-5-yl)-1,2,5,6-t etrahydropyridin-3-yl)pyridin-2-yl)be nzamide |
| 24 | 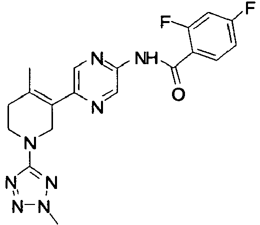 | 2,4-difluoro-N-(5-(4-methyl-1-(2-methyl-2H-tetrazol-5-yl)-1,2,5,6-tetrahydropyridin-3-yl)pyrazin-2-yl)benzamide |
| 25 | 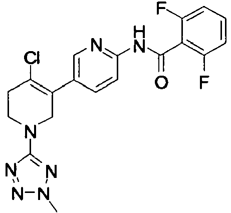 | N-(5-(4-chloro-1-(2-methyl-2H-tetrazol-5-yl)-1,2,5,6-tetrahydro pyridin-3-yl)pyridin-2-yl)-2,6-difluorobenzamide |
| 26 | 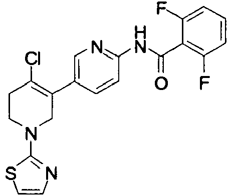 | N-(5-(4-chloro-1-(thiazol-2-yl)-1,2,5 ,6-tetrahydropyridin-3-yl)pyridin-2-yl )-2,6-difluorobenzamide |
| 27 | 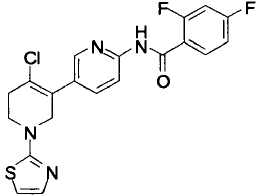 | 2-chloro-6-fluoro-N-(5-(4-methyl-1-(2-methyl-2H-tetrazol-5-yl)-1,2,5,6-t etrahydropyridin-3-yl)pyridin-2-yl)be nzamide |
| 28 | 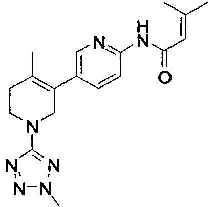 | 3-methyl-N-(5-(4-methyl-1-(2-methyl-2H-tetrazol-5-yl)-1,2,5,6 tetrahydropyridin-3-yl)pyridin-2-yl)b ut-2-enamide |
| 29 | 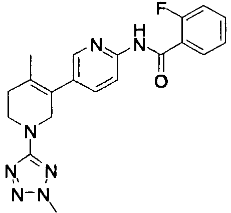 | 2-fluoro-N-(5-(4-methyl-1-(2-methyl-2H-tetrazol-5-yl)-1,2,5,6-tetrahydropyridin-3-yl)pyridin-2-yl)b enzamide |
| 30 | 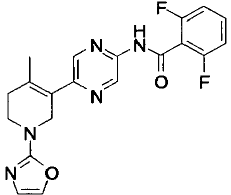 | 2,6-difluoro-N-(5-(4-methyl-1-(oxaz ol-2-yl)-1,2,5,6-tetrahydropyridin-3-yl)pyrazin-2-yl)benzamide |
| 31 | 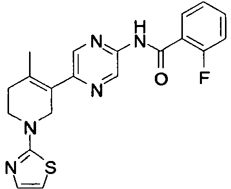 | 2-fluoro-N-(5-(4-methyl-1-(thiazol-2 -yl)-1,2,5,6-tetrahydropyridin-3-yl)p yrazin-2-yl)benzamide |
| 32 | 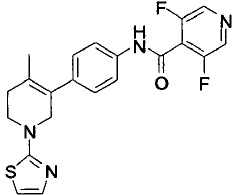 | 3,5-difluoro-N-(4-(4-methyl-1-(thiaz ol-2-yl)-1,2,5,6-tetrahydropyridin-3-yl)phenyl)isonicotinamide |
| 33 | 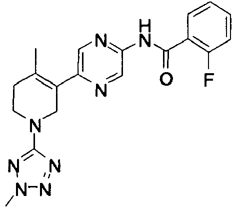 | 2-fluoro-N-(5-(4-methyl-1-(2-methyl-2H-tetrazol-5-yl)-1,2,5,6-tetr ahydropyridin-3-yl)pyrazin-2-yl)benz amide |
| 34 | 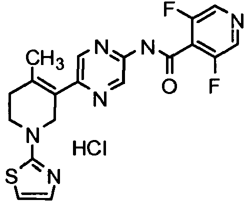 | 3,5-difluoro-N-(5-(4-methyl-l-(thiaz ol-2-yl)-1,2,5,6-tetrahydropyridin-3-yl)pyrazin-2-yl)isonicotinamide hydrochloride |
| 35 | 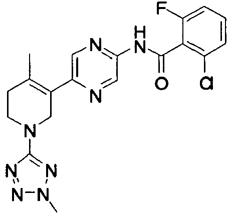 | 2-chloro-6-fluoro-N-(5-(4-methyl-1-(2-methyl-2H-tetrazol-5-yl)-1,2,5,6-t etrahydropyridin-3-yl)pyrazin-2-yl)be nzamide |
| 36 | 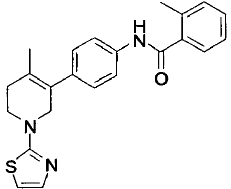 | 2-methyl-N-(4-(4-methyl-1-(thiazol-2-yl)-1,2,5,6-tetrahydropyridin-3-yl) phenyl)benzamide |
| 37 | 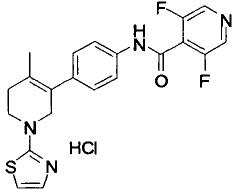 | 3,5-difluoro-N-(4-(4-methyl-1-(thiaz ol-2-yl)-1,2,5,6-tetrahydropyridin-3-yl)phenyl)isonicotinamide hydrochloride |
| 38 | 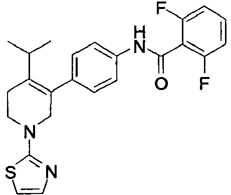 | 2,6-difluoro-N-(4-(4-isopropyl-1-(thi azol-2-yl)-1,2,5,6-tetrahydropyridin-3-yl)phenyl)benzamide |
| 39 | 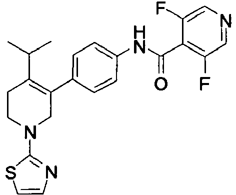 | 3,5-difluoro-N-(4-(4-isopropyl-1-(thi azol-2-yl)-1,2,5,6-tetrahydropyridin-3-yl)phenyl)isonicotinamide |
| 40 | 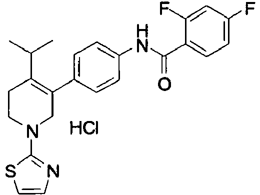 | 2,4-difluoro-N-(4-(4-isopropyl-1-(thi azol-2-yl)-1,2,5,6-tetrahydropyridin-3-yl)phenyl)benzamide hydrochloride |
| 41 | 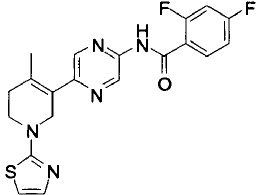 | 2,4-difluoro-N-(5-(4-methyl-1-(thiaz ol-2-yl)-1,2,5,6-tetrahydropyridin-3-yl)pyrazin-2-yl)benzamide |
| 42 | 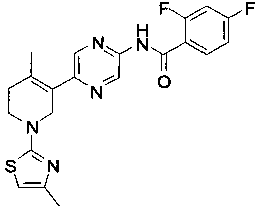 | 2,4-difluoro-N-(5-(4-methyl-1-(4-me thylthiazol-2-yl)-1,2,5,6-tetrahydrop yridin-3-yl)pyrazin-2-yl)benzamide |
| 43 | 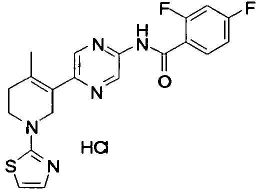 | 2,4-difluoro-N-(5-(4-methyl-1-(thiaz ol-2-yl)-1,2,5,6-tetrahydropyridin-3-yl)pyrazin-2-yl)benzamide hydrochloride |
| 44 | 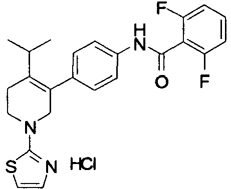 | 2,6-difluoro-N-(4-(4-isopropyl-1-(thi azol-2-yl)-1,2,5,6-tetrahydropyridin-3-yl)phenyl)benzamide hydrochloride |
| 45 | 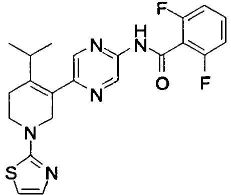 | 2,6-difluoro-N-(5-(4-isopropyl-1-(thi azol-2-yl)-1,2,5,6-tetrahydropyridin-3-yl)pyrazin-2-yl)benzamide |
| 46 | 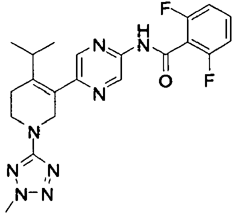 | 2,6-difluoro-N-(5-(4-isopropyl-1-(2-methyl-2H-tetrazol-5-yl)-1,2,5,6-tetr ahydropyridin-3-yl)pyrazin-2-yl)benz amide |
| 47 | 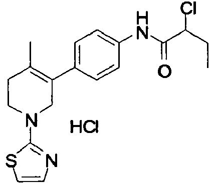 | 2-chloro-N-(4-(4-methyl-1-(thiazol-2--yl)-1,2,5,6-tetrahydropyridin-3-yl)p henyl)butanamide hydrochloride |
| 48 | 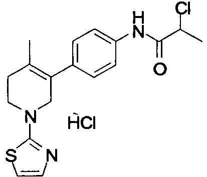 | 2-chloro-N-(4-(4-methyl-1-(thiazol-2--yl)-1,2,5,6-tetrahydropyridin-3-yl)p henyl)propanamide hydrochloride |

### MECHANISM OF ACTION

Activation of T-lymphocytes in response to an antigen is dependent on calcium ion oscillations. Calcium ion oscillations in T-lymphocytes are triggered through stimulation of the T-cell antigen receptor, and involve calcium ion influx through the stored-operated Ca²⁺-release-activated Ca²⁺ (CRAC) channel. Although the molecular structure of the CRAC ion channel has not been identified, a detailed electrophysiological profile of the channel exist. Thus, inhibition of CRAC ion channels can be measured by measuring inhibition of the I_{CRAC} current. Calcium ion oscillations in T-cells have been implicated in the activation of several transcription factors (e.g., NFAT, Oct/Oap and NFκB) which are critical for T-cell activation (Lewis, Biochemical Society Transactions (2003), 31:925-929). Without wishing to be bound by any theory, it is believed that because the compounds of the invention inhibit the activity of CRAC ion channels, they inhibit immune cell activation.

### USE OF THE INVENTIVE COMPOUNDS FOR TREATMENT AND PREVENTION

In accordance with the invention, an effective amount of a compound of any one of formulas (I) through (IX) or Table 1, or a pharmaceutically acceptable salt, solvate, and clathrate thereof, or a pharmaceutical composition comprising a compound of any one of formulas (I) through (IX) or Table 1, or a pharmaceutically acceptable salt, solvate, and clathrate thereof, is administered to a patient in need of immunosuppression or in need of treatment or prevention of an inflammatory condition, an immune disorder, or an allergic disorder. Such patients may be treatment naïve or may experience partial or no response to conventional therapies. Responsiveness of a particular inflammatory condition, immune disorder, or allergic disorder in a subject can be measured directly (*e.g.*, measuring blood levels of inflammatory cytokines (such as IL-2, IL-4, IL-5, IL-13, GM-CSF, TNF-α, IFN-γ and the like) after administration of a compound of this invention), or can be inferred based on an understanding of disease etiology and progression. The compounds of any one of formulas (I) through (IX), or Table 1, or pharmaceutically acceptable salts, solvates, and clathrates thereof can be assayed *in vitro* or *in vivo,* for the desired therapeutic or prophylactic activity, prior to use in humans. For example, known animal models of inflammatory conditions, immune disorders, or allergic disorders can be used to demonstrate the safety and efficacy of compounds of this invention.

### PHARMACEUTICAL COMPOSITIONS AND DOSAGE FORMS

Pharmaceutical compositions and dosage forms of the invention comprise one or more active ingredients in relative amounts and formulated in such a way that a given pharmaceutical composition or dosage form can be used for immunosuppression or to treat or prevent inflammatory conditions, immune disorders, and allergic disorders. Preferred pharmaceutical compositions and dosage forms comprise a compound of any one of formulas (I) through (IX), or Table 1, or a pharmaceutically acceptable salt, solvate, or clathrate thereof, optionally in combination with one or more additional active agents.

Single unit dosage forms of the invention are suitable for oral, mucosal (*e.g.*, nasal, sublingual, vaginal, buccal, or rectal), parenteral (*e.g*., subcutaneous, intravenous, bolus injection, intramuscular, or intraarterial), or transdermal administration to a patient. Examples of dosage forms include, but are not limited to: tablets; caplets; capsules, such as soft elastic gelatin capsules; cachets; troches; lozenges; dispersions; suppositories; ointments; cataplasms (poultices); pastes; powders; dressings; creams; plasters; solutions; patches; aerosols (*e.g*., nasal sprays or inhalers); gels; liquid dosage forms suitable for oral or mucosal administration to a patient, including suspensions (*e.g*., aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g*., crystalline or amorphous solids) that can be reconstituted to provide liquid dosage forms suitable for parenteral administration to a patient.

The composition, shape, and type of dosage forms of the invention will typically vary depending on their use. For example, a dosage form suitable for mucosal administration may contain a smaller amount of active ingredient(s) than an oral dosage form used to treat the same indication. This aspect of the invention will be readily apparent to those skilled in the art. *See, e.g*., Remington's Pharmaceutical Sciences (1990) 18th ed., Mack Publishing, Easton PA.

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms.

The suitability of a particular excipient may also depend on the specific active ingredients in the dosage form. For example, the decomposition of some active ingredients can be accelerated by some excipients such as lactose, or when exposed to water. Active ingredients that comprise primary or secondary amines (*e.g*., N-desmethylvenlafaxine and N,N-didesmethylvenlafaxine) are particularly susceptible to such accelerated decomposition. Consequently, this invention encompasses pharmaceutical compositions and dosage forms that contain little, if any, lactose. As used herein, the term "lactose-free" means that the amount of lactose present, if any, is insufficient to substantially increase the degradation rate of an active ingredient. Lactose-free compositions of the invention can comprise excipients that are well known in the art and are listed, for example, in the U.S. Pharmocopia (USP) SP (XXI)/NF (XVI). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

This invention further encompasses anhydrous pharmaceutical compositions and dosage forms comprising active ingredients, since water can facilitate the degradation of some compounds. For example, the addition of water (*e.g*., 5%) is widely accepted in the pharmaceutical arts as a means of simulating long-term storage in order to determine characteristics such as shelf-life or the stability of formulations over time. *See, e.g.,* Jens T. Carstensen (1995) Drug Stability: Principles & Practice, 2d. Ed., Marcel Dekker, NY, NY, 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms of the invention can be prepared using anhydrous or low moisture containing ingredients and low moisture or low humidity conditions. Pharmaceutical compositions and dosage forms that comprise lactose and at least one active ingredient that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically sealed foils, plastics, unit dose containers (*e.g*., vials), blister packs, and strip packs.

The invention further encompasses pharmaceutical compositions and dosage forms that comprise one or more compounds that reduce the rate by which an active ingredient will decompose. Such compounds, which are referred to herein as "stabilizer" include, but are not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. However, typical dosage forms of the invention comprise a compound of any one of formulas (I) through (IX), or Table 1, or a pharmaceutically acceptable salt, solvate, or clathrate thereof in an amount of from about 1 mg to about 1000 mg, preferably in an amount of from about 50 mg to about 500 mg, and most preferably in an amount of from about 75 mg to about 350 mg. The typical total daily dosage of a compound of any one of formulas (I) through (IX), or Table 1, or a pharmaceutically acceptable salt, solvate, or clathrate thereof can range from about 1 mg to about 5000 mg per day, preferably in an amount from about 50 mg to about 1500 mg per day, more preferably from about 75 mg to about 1000 mg per day. It is within the skill of the art to determine the appropriate dose and dosage form for a given patient.

### ORAL DOSAGE FORMS

Pharmaceutical compositions of the invention that are suitable for oral administration can be presented as discrete dosage forms, such as, but are not limited to, tablets (*e.g*., chewable tablets), caplets, capsules, and liquids (*e.g*., flavored syrups). Such dosage forms contain predetermined amounts of active ingredients, and may be prepared by methods of pharmacy well known to those skilled in the art. *See generally*, Remington's Pharmaceutical Sciences (1990) 18th ed., Mack Publishing, Easton PA.

Typical oral dosage forms of the invention are prepared by combining the active ingredient(s) in an admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g*., powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. If desired, tablets can be coated by standard aqueous or nonaqueous techniques. Such dosage forms can be prepared by any of the methods of pharmacy. In general, pharmaceutical compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms of the invention include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g*., ethyl cellulose, cellulose acetate, carboxymethyl cellulose calcium, sodium carboxymethyl cellulose), polyvinyl pyrrolidone, methyl cellulose, pre-gelatinized starch, hydroxypropyl methyl cellulose, (*e.g*., Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. One specific binder is a mixture of microcrystalline cellulose and sodium carboxymethyl cellulose sold as AVICEL RC-581. Suitable anhydrous or low moisture excipients or additives include AVICEL-PH-103J and Starch 1500 LM.

Examples of fillers suitable for use in the pharmaceutical compositions and dosage forms disclosed herein include, but are not limited to, talc, calcium carbonate (*e.g*., granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions of the invention is typically present in from about 50 to about 99 weight percent of the pharmaceutical composition or dosage form.

Disintegrants are used in the compositions of the invention to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms of the invention. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms of the invention include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms of the invention include, but are not limited to, calcium stearate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g*., peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL 200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

### CONTROLLED RELEASE DOSAGE FORMS

Active ingredients of the invention can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients of the invention. The invention thus encompasses single unit dosage forms suitable for oral administration such as, but not limited to, tablets, capsules, gelcaps, and caplets that are adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g*., adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

A particular extended release formulation of this invention comprises a therapeutically or prophylactically effective amount of a compound of formulas (I) through (IX), or Table 1, or a pharmaceutically acceptable salt, solvate, hydrate, or clathrate thereof, in spheroids which further comprise microcrystalline cellulose and, optionally, hydroxypropylmethyl-cellulose coated with a mixture of ethyl cellulose and hydroxypropylmethylcellulose. Such extended release formulations can be prepared according to U.S. Patent No. 6,274,171.

A specific controlled-release formulation of this invention comprises from about 6% to about 40% a compound of any one of formulas (I) through (IX), or Table 1 by weight, about 50% to about 94% microcrystalline cellulose, NF, by weight, and optionally from about 0.25% to about 1% by weight of hydroxypropyl-methylcellulose, USP, wherein the spheroids are coated with a film coating composition comprised of ethyl cellulose and hydroxypropylmethylcellulose.

### PARENTERAL DOSAGE FORMS

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but are not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms of the invention are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients disclosed herein can also be incorporated into the parenteral dosage forms of the invention.

### TRANSDERMAL, TOPICAL, AND MUCOSAL DOSAGE FORMS

Transdermal, topical, and mucosal dosage forms of the invention include, but are not limited to, ophthalmic solutions, sprays, aerosols, creams, lotions, ointments, gels, solutions, emulsions, suspensions, or other forms known to one of skill in the art. *See, e.g.,* Remington's Pharmaceutical Sciences (1980 & 1990) 16h and 18th eds., Mack Publishing, Easton PA and Introduction to Pharmaceutical Dosage Forms (1985) 4th ed., Lea & Febiger, Philadelphia. Dosage forms suitable for treating mucosal tissues within the oral cavity can be formulated as mouthwashes or as oral gels. Further, transdermal dosage forms include "reservoir type" or "matrix type" patches, which can be applied to the skin and worn for a specific period of time to permit the penetration of a desired amount of active ingredients.

Suitable excipients (*e.g*., carriers and diluents) and other materials that can be used to provide transdermal, topical, and mucosal dosage forms encompassed by this invention are well known to those skilled in the pharmaceutical arts, and depend on the particular tissue to which a given pharmaceutical composition or dosage form will be applied. With that fact in mind, typical excipients include, but are not limited to, water, acetone, ethanol, ethylene glycol, propylene glycol, butane-1,3-diol, isopropyl myristate, isopropyl palmitate, mineral oil, and mixtures thereof to form lotions, tinctures, creams, emulsions, gels or ointments, which are non-toxic and pharmaceutically acceptable. Moisturizers or humectants can also be added to pharmaceutical compositions and dosage forms if desired. Examples of such additional ingredients are well known in the art. *See, e.g.,* Remington's Pharmaceutical Sciences (1980 & 1990) 16h and 18th eds., Mack Publishing, Easton PA.

Depending on the specific tissue to be treated, additional components may be used prior to, in conjunction with, or subsequent to treatment with active ingredients of the invention. For example, penetration enhancers can be used to assist in delivering the active ingredients to the tissue. Suitable penetration enhancers include, but are not limited to: acetone; various alcohols such as ethanol, oleyl, and tetrahydrofuryl; alkyl sulfoxides such as dimethyl sulfoxide; dimethyl acetamide; dimethyl formamide; polyethylene glycol; pyrrolidones such as polyvinylpyrrolidone; Kollidon grades (Povidone, Polyvidone); urea; and various water-soluble or insoluble sugar esters such as Tween 80 (polysorbate 80) and Span 60 (sorbitan monostearate).

The pH of a pharmaceutical composition or dosage form, or of the tissue to which the pharmaceutical composition or dosage form is applied, may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts, hydrates or solvates of the active ingredients can be used to further adjust the properties of the resulting composition.

### COMBINATION THERAPY

The compounds of the invention may be used for immunosuppression or for treating or preventing inflammatory conditions and immune disorders in a patient in need thereof. This can further comprise administering to the patient being administered a compound of this invention, an effective amount of one or more other active agents. Such active agents may include those used conventionally for immunosuppression or for inflammatory conditions or immune disorders. These other active agents may also be those that provide other benefits when administered in combination with the compounds of this invention. For example, other therapeutic agents may include, without limitation, steroids, non-steroidal anti-inflammatory agents, antihistamines, analgesics, immunosuppressive agents and suitable mixtures thereof. In such combination therapy treatment, both the compounds of this invention and the other drug agent(s) are administered to a subject (*e.g*., humans, male or female) by conventional methods. The agents may be administered in a single dosage form or in separate dosage forms. Effective amounts of the other therapeutic agents and dosage forms are well known to those skilled in the art. It is well within the skilled artisan's purview to determine the other therapeutic agent's optimal effective-amount range.

In one embodiment of the invention where another therapeutic agent is administered to a subject, the effective amount of the compound of this invention is less than its effective amount when the other therapeutic agent is not administered. In another embodiment, the effective amount of the conventional agent is less than its effective amount when the compound of this invention is not administered. In this way, undesired side effects associated with high doses of either agent may be minimized. Other potential advantages (including without limitation improved dosing regimens and/or reduced drug cost) will be apparent to those of skill in the art.

In one embodiment relating to autoimmune and inflammatory conditions, the other therapeutic agent may be a steroid or a non-steroidal anti-inflammatory agent. Particularly useful non-steroidal anti-inflammatory agents, include, but are not limited to, aspirin, ibuprofen, diclofenac, naproxen, benoxaprofen, flurbiprofen, fenoprofen, flubufen, ketoprofen, indoprofen, piroprofen, carprofen, oxaprozin, pramoprofen, muroprofen, trioxaprofen, suprofen, aminoprofen, tiaprofenic acid, fluprofen, bucloxic acid, indomethacin, sulindac, tolmetin, zomepirac, tiopinac, zidometacin, acemetacin, fentiazac, clidanac, oxpinac, mefenamic acid, meclofenamic acid, flufenamic acid, niflumic acid, tolfenamic acid, diflurisal, flufenisal, piroxicam, sudoxicam, isoxicam; salicylic acid derivatives, including aspirin, sodium salicylate, choline magnesium trisalicylate, salsalate, diflunisal, salicylsalicylic acid, sulfasalazine, and olsalazin; para-aminophennol derivatives including acetaminophen and phenacetin; indole and indene acetic acids, including indomethacin, sulindac, and etodolac; heteroaryl acetic acids, including tolmetin, diclofenac, and ketorolac; anthranilic acids (fenamates), including mefenamic acid, and meclofenamic acid; enolic acids, including oxicams (piroxicam, tenoxicam), and pyrazolidinediones (phenylbutazone, oxyphenthartazone); and alkanones, including nabumetone and pharmaceutically acceptable salts thereof and mixtures thereof. For a more detailed description of the NSAIDs, *see* Paul A. Insel, Analgesic-Antipyretic and Antiinflammatory Agents and Drugs Employed in the Treatment of Gout, in Goodrnan & Gilman's The Pharmacological Basis of Therapeutics 617-57 (Perry B. Molinhoff and Raymond W. Ruddon eds., 9th ed 1996) and Glen R. Hanson, Analgesic, Antipyretic and Anti-Inflammatory Drugs in Remington: The Science and Practice of Pharmacy Vol II 1196-1221 (A.R. Gennaro ed. 19th ed. 1995).

Of particular relevance to allergic disorders, the other therapeutic agent may be an antihistamine. Useful antihistamines include, but are not limited to, loratadine, cetirizine, fexofenadine, desloratadine, diphenhydramine, chlorpheniramine, chlorcyclizine, pyrilamine, promethazine, terfenadine, doxepin, carbinoxamine, clemastine, tripelennamine, brompheniramine, hydroxyzine, cyclizine, meclizine, cyproheptadine, phenindamine, acrivastine, azelastine, levocabastine, and mixtures thereof. For a more detailed description of anthihistamines, *see* Goodman & Gilman's The Pharmacological Basis of Therapeutics (2001) 651-57, 10th ed).

Immunosuppressive agents include glucocorticoids, corticosteroids (such as Prednisone or Solumedrol), T cell blockers (such as cyclosporin A and FK506), purine analogs (such as azathioprine (Imuran)), pyrimidine analogs (such as cytosine arabinoside), alkylating agents (such as nitrogen mustard, phenylalanine mustard, buslfan, and cyclophosphamide), folic acid antagonists (such as aminopterin and methotrexate), antibiotics (such as rapamycin, actinomycin D, mitomycin C, puramycin, and chloramphenicol), human IgG, antilymphocyte globulin (ALG), and antibodies (such as anti-CD3 (OKT3), anti-CD4 (OKT4), anti-CD5, anti-CD7, anti-IL-2 receptor, anti-alpha/beta TCR, anti-ICAM-1, anti-CD20 (Rituxan), anti-IL-12 and antibodies to immunotoxins).

The foregoing and other useful combination therapies will be understood and appreciated by those of skill in the art. Potential advantages of such combination therapies include a different efficacy profile, the ability to use less of each of the individual active ingredients to minimize toxic side effects, synergistic improvements in efficacy, improved ease of administration or use and/or reduced overall expense of compound preparation or formulation.

### OTHER EMBODIMENTS

The compounds of this invention may be used as research tools (for example, as a positive control for evaluating other potential CRAC inhibitors, or IL-2, IL-4, IL-5, IL-13, GM-CSF, TNF-α, and/or INF- γ inhibitors).

The invention is further defined by reference to the following examples describing in detail the preparation of compounds of the invention

### EXAMPLES

### EXPERIMENTAL RATIONALE

Without wishing to be bound by theory, it is believed that the compounds of this invention inhibit CRAC ion channels, thereby inhibiting production of IL-2 and other key cytokines involved with inflammatory and immune responses. The examples that follow demonstrate these properties.

### MATERIALS AND GENERAL METHODS

Reagents and solvents used below can be obtained from commercial sources such as Aldrich Chemical Co. (Milwaukee, Wisconsin, USA). ¹H-NMR and ¹³C-NMR spectra were recorded on a Varian 300MHz NMR spectrometer. Significant peaks are tabulated in the order: δ (ppm): chemical shift, multiplicity (s, singlet; d, doublet; t, triplet; q, quartet; m, multiplet; br s, broad singlet),coupling constant(s) in Hertz (Hz) and number of protons.

Patch clamp experiments were performed in the tight-seal whole-cell configuration at 21-25°C. High resolution current recordings were acquired by a computer-based patch clamp amplifier system (EPC-9, HEKA, Lambrecht, Germany). Patch pipettes had resistances between 2-4 MΩ after filling with the standard intracellular solution. Immediately following establishment of the whole-cell configuration, voltage ramps of 50-200 ms duration spanning the voltage range of -100 to +100 mV were delivered at a rate of 0.5 Hz over a period of 300-400 seconds. All voltages were corrected for a liquid junction potential of 10 mV between external and internal solutions when using glutamate as the intracellular anion. Currents were filtered at 2.9 kHz and digitized at 10 µs intervals. Capacitive currents and series resistance were determined and corrected before each voltage ramp using the automatic capacitance compensation of the EPC-9. The low resolution temporal development of membrane currents was assessed by extracting the current amplitude at -80 mV or +80 mV from individual ramp current records.

Compounds of the invention can also be prepared as in U.S. Application No. 10/897,681, filed July 22, 2004 and U.S. Application No. 11/326,872, filed January 6, 2006.

### EXAMPLE 1: SYNTHESIS OF REPRESENTATIVE EXEMPLARY COMPOUNDS OF THIS INVENTION

### 3-fluoro-N-(4-(4-methyl-1-(thiazol-2-yl)-1,2,5,6-tetrahydropyridin-3-yl)phenyl)isonicotinami de dihydrochloride:

### Method 1:

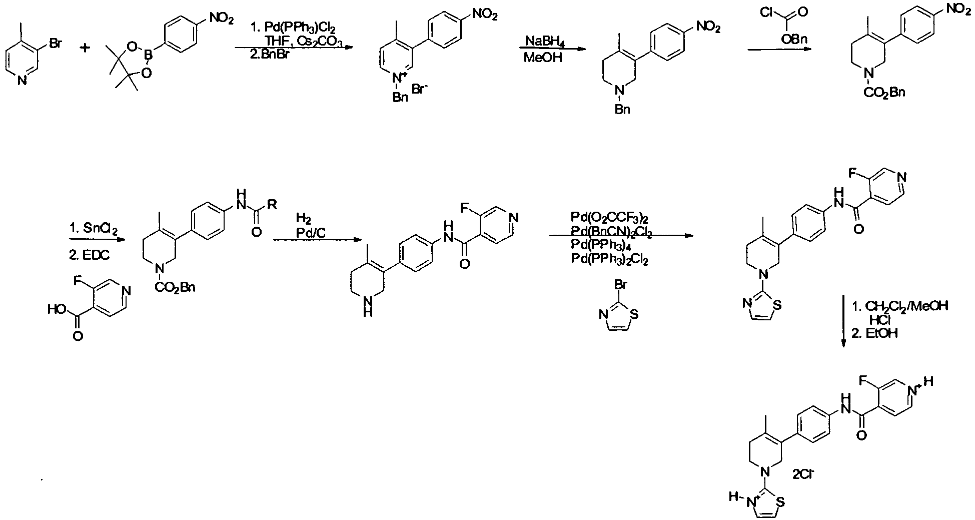

### Method 2:

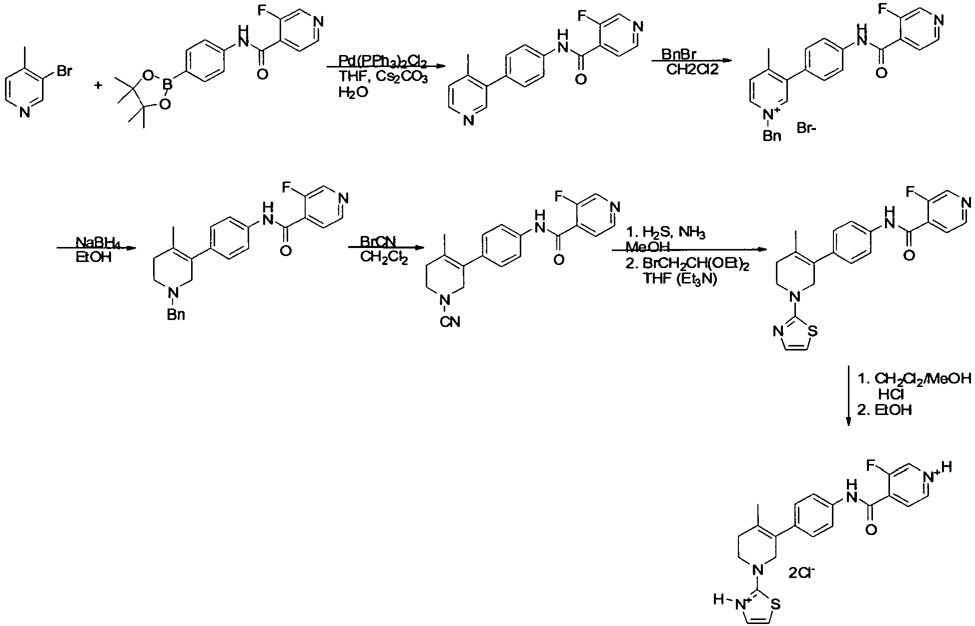

### Coupling of Bromopicoline:

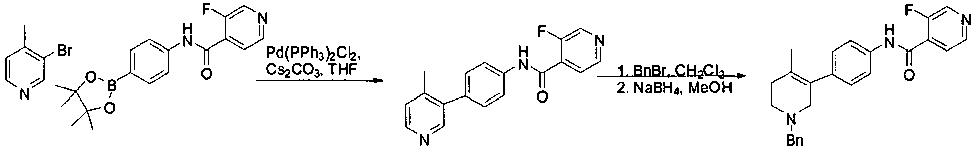

A solution of boronic ester(1g, 1eq), bromopicoline (1eq) and cesium carbonate (1.5 eq) was made in a THF 10 ml in a microwave vial. The palladium was added along with a couple drops of water, then sealed. The vial was heated to 170 °C for 1 hour. Water was added along with ethyl acetate. After extraction, the organics were dried with sodium sulfate. A column was performed in ethyl acetate / hexane. After drying, a 78% yield of the desired product was recovered as an off-white solid.

3-fluoro-N-(4-(4-methylpyridin-3-yl)phenyl)isonicotinamide
ESMS calcd. (C₁₈H₁₄FN₃O) 307.1; found: 308.0 (M⁺1).

### Pyridinium Salt Formation:

A solution of the pyridine (200mg, 0.62mmol) was dissolved in dichloromethane(1.5ml). Benzyl bromide (3eq) was added and the solution was stirred at room temperature for 24 hours. The solid was filtered and washed with cold dichloromethane to give pure pyridinium salt. This solid was dried and carried on to next step.

1-benzyl-3-(4-(3-fluoroisonicotinamido)phenyl)-4-methylpyridinium bromide
ESMS calcd. (C₁₈H₁₄FN₃O) 398.2; found: 398.5 (M⁺).

### Reduction of Pyridinium Salt:

The pyridinium salt (crude) was dissolved in methanol and sodium borohydride (113mg, 3eq) was added. This solution was warmed to 35 °C for 5 h then cooled to room temperature. The methanol was removed, water and methylene chloride added, and extracted. The 150mg (70%yield) of tetrahydropyridine was produced after column chromatography.

N-(4-(1-benzyl-4-methyl-1,2,5,6-tetrahydropyridin-3-yl)phenyl)-3-fluoroiso nicotinamide
¹H NMR (300 MHz, CDCl₃) δ 8.66 (d, *J*=2.7Hz, 1H), 8.63 (dd, *J*=1.5, 5.1Hz, 1H), 8.37 (broad d, *J*=13.5Hz, 1H), 8.03 (dd, *J*=3.6, 6.6Hz, 1H), 7.60 (d, *J*=8.7Hz, 2H), 7.39-7.18 (m, 5H), 7.20 (d, *J*=9Hz, 2H), 3.63 (s, 2H), 3.20-3.15(m, 2H), 2.64 (t, *J*=5.7, 2H), 2.24-2.21 (m, 2H), 1.62 (s, 3H) ppm. ESMS calcd. (C25H24FN30 401.2; found: 402.2 (M⁺1).

### Cyano transfer

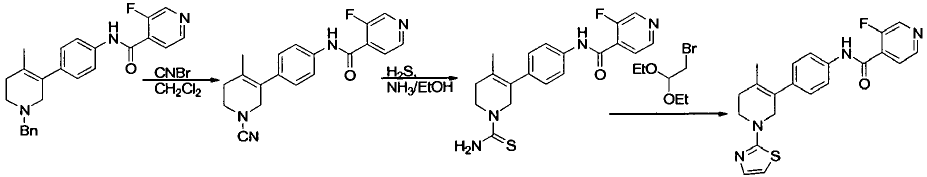

The benzamine(477mg, 1.19mmol) was dissolve in methylene chloride (15ml) at room temperature. Cyanogen bromide(2.4mmol) was added to the reaction mixture and it was allowed to stir at room temperature for 1 h. This methylene chloride solution was added directly to the head of the column and purified. Once pure compound was in hand it was dissolved in EtOH(2M ammonia) and hydrogen sulfide was bubbled through the reaction for half hour. The bubbling was then ceased and the vessel was capped and stirred for 3 h more. Once the reaction was complete the solvent was removed, first by bubbling air through, then by high-vac. Ethyl acetate was added and the reaction was triturated. The product was collected by filtration.

N-(4-(1-cyano-4-methyl-1,2,5,6-tetrahydropyridin-3-yl)phenyl)-3-fluoroiso nicotinamide
¹H NMR (300 MHz, CDCl₃) δ 8.67 (d, *J*=2.41Hz, 1H), 8.65 (dd, *J*=1.5, 5.1Hz, 1H), 8.44 (broad d, *J*=13.5Hz, 1H), 8.03 (dd, *J*=5.1, 6.6Hz, 1H), 7.69-7.64(m, 2H), 7.20-7.16 (m, 2H), 3.87-3.84 (m, 2H), 3.40 (t, *J*=5.7, 2H), 2.34-2.31 (m, 2H), 1.65 (s, 3H) ppm. ESMS calcd.
(C₁₉H₁₇FN₄O) 466.1; found: 467.1 (M⁺¹).

N-(4-(1-carbamothioyl-4-methyl-1,2,5,6-tetrahydropyridin-3-yl)phenyl)-3-fluoroiso nicotinamide
¹H NMR (300 MHz, CDCl₃) 8.68 (d, *J*=2.7Hz, 1H), 8.66 (d, *J*=5.4Hz, 1H), 8.40 (d, *J*=12.9Hz, 1H), 8.04 (t, *J*=6.3Hz, 1H), 7.67 (d, *J*=8.7Hz, 2H), 7.24-7.22 (m, 2H), 5.71 (s, 2H), 4.35-4.28 (m, 2H), 4.10-4.03 (m, 2H), 2.30-2.39 (m, 2H), 1.69 (s, 3H) ppm
ESMS calcd. (C₁₉H₁₉FN₄OS 370.1; found: 371.4 (M⁺¹).

### General Procedure for Tetrahydropyridine deprotection

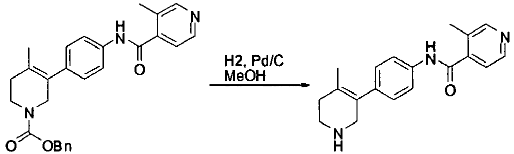

10% Palladium on carbon (10mg, 10%w/w) was added to a methanolic solution (1.5ml) of benzylcarbamate (109mg, 0.247mmol). A balloon of hydrogen was added to the top of the flask and thoroughly stirred for 1 hour. After filtration completely pure free amine was recovered (73mg, 96%yield). No further purification required.

### General Procedure for thiazole amine formation.

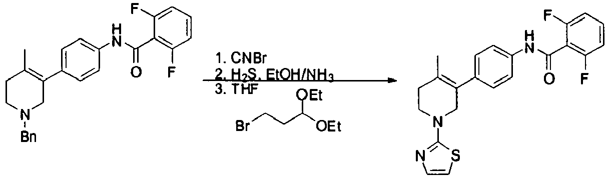

The amine (725mg, 1.73mmol) was dissolved in methylene chloride (5ml) and cyanogen bromide (549mg, 5.19mmol) was added. This reaction was stirred at room temperature for 1 hour then purified by flash chromatography. Pure cyanamide (415mg, 68% yield) was isolated as a white solid.

The cyanamide (55mg, .01556mmol) was dissolved in ethanol/ammonia (2ml, 2M). Hydrogen sulfide gas was then bubbled through the reaction solution for 20minutes at which time the bubbling was siesed and the reaction was capped and stirred for 5hours at room temperature. After completion was solvent was removed and the thiourea was recrystallized from ethanol to remove yellow color.

The thiourea was then dissolved in anhydrous THF (5ml) and the bromide (140mg, 0.709mmol) was added and refluxed for 5 hours. The rxn mixture was then quenched with sodium bicarbonate solution and diluted with ethylacetate. After drying over magnesium sulfate, the compound was purified by flash chromotagraphy yielding 39mg of pure thiazole product. The compound was then dissolved in ethanol and excess HCl in ether was added to form the salt. Solid precipitated out of solution at -40°C and was filtered.

### Thiazole Cyclization:

The thiourea was dissolved in dry THF(15ml) and bromoacetaldehyde diethylether(479ul, 3eq)was added to the reaction at reflux. After refluxing for 3 h the reaction was cooled and THF was diluted with methylene chloride and NaHCO₃ solution. After acid base work-up, pure thiazole amine was isolated. This compound was dissolved in methylene chloride and a solution of HCl in ether was added, rotovaped and recrystallized to get pure thiazole disalt. (254mg, 49% from benzamine)

3-fluoro-N-(4-(4-methyl-1-(thiazol-2-yl)-1,2,5,6-tetrahydropyridin-3-yl)phenyl)isonicotinami de
¹H NMR (300 MHz, DMSO) δ 8.68 (d, *J*=2.7Hz, 1H), 8.66 (dd, *J*=1.2, 5.1Hz, 1H), 8.15 (d, *J*=13.1Hz, 1H), 8.05 (dd, *J*=4.8, 6.6Hz, 1H), 7.67 (d, *J*=8.4Hz, 2H), 7.29-7.22(m, 3H), 6.58 (d, *J*=3.9Hz, 1H), 4.10-4.08 (m, 2H), 3.75 (t, *J*=6.0Hz, 2H), 2.39-2.36(m, 2H), 1.68 (s, 3H)ppm
ESMS calcd. (C₂₁H₁₉FN₄OS 394.1; found: 395.2 (M⁺¹).

3-fluoro-N-(4-(4-methyl-1-(thiazol-2-yl)-1,2,5,6-tetrahydropyridin-3-yl)phenyl)isonicotinami de
¹HNMR(300 MHz, DMSO) δ 10.85 (s, 1H), 8.79 (s, 1H), 8.61 (d, *J*=5.1Hz, 1H), 7.78-7.70 (m, H), 7.48(dd, *J*=1.2, 3.9Hz, 1H), 7.33 (d, *J*=7.5Hz, 2H), 7.12 (dd, *J*=1.5, 4.2Hz, 1H), 4.40 (broad s, 2H), 4.26 (s, 2H), 3.81-3.78 (m, 2H), 2.41 (s, 2H), 1.69 (s, 3H) ppm. ESMS calcd. (C₂₁H₂₁F₂N₄OS 466.1; found: 396.2 (M^{+ -2Cl}).

### 3-methyl-N-(4-(4-methyl-1-(thiazol-2-yl)-1,2,5,6-tetrahydropyridin-3-yl)phenyl)isonicotinami de:

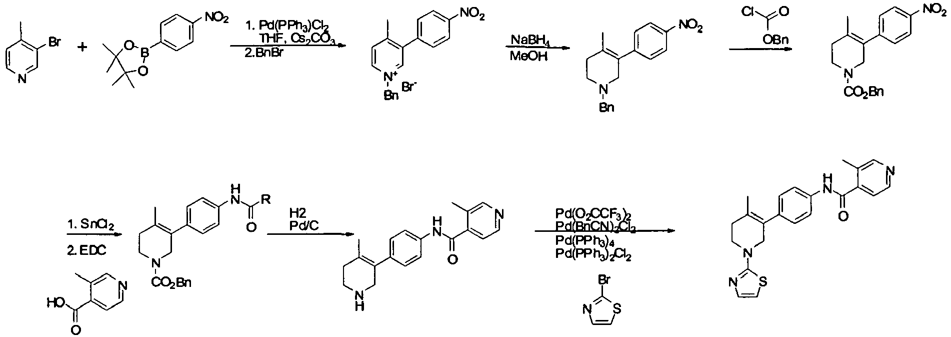

### 2,6-difluoro-N-(4-(4-methyl-1-(thiazol-2-yl)-1,2,5,6-tetrahydropyridin-3-yl)phenyl)benzamid e hydrochloride:

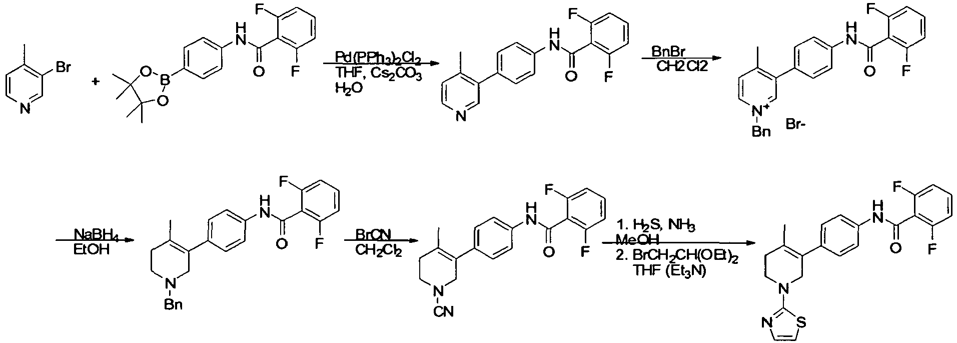

### 2,6-difluoro-N-(4-(4-formyl-1-(thiazol-2-yl)-1,2,5,6-tetrahyrdropyridin-3-yl)phenyl)benzamid e:

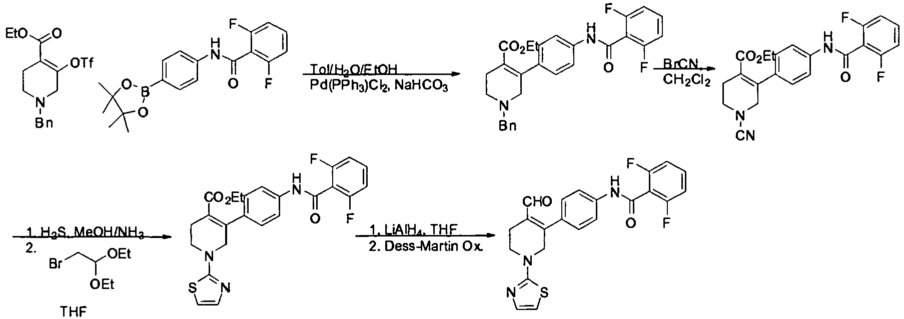

**Procedure**: The triflate (ethyl 1-benzyl-5-(trifluoromethylsulfonyloxy)-1,2,3,6-tetrahydropyridine-4-carboxylate) (1.095g, 2.784mmol), boronic ester(1.00g, 2.78mmol), Pd(PPh₃)₂Cl₂(19.6mg,0.028 mmol), NaHCO₃(213.5mg, 2.78mmol), were all added to a microwave vial as a solid. 13ml of toluene, 2ml of water and 0.5ml of ethanol were added to the vial and it was sealed and heated for 1 hour. The rxn mixture was dumped into water and extracted 3x with methylene chloride. Purified by column chromatography to produce 600mg of pure product in a 45% yield.

Pure
ethyl-1-benzyl-5-(4-(2,6-difluorobenzamido)phenyl)-1,2,3,6-tetrahydropyridine-4-carboxylat e was dissolved in methylene chloride. Cyanogen bromide was added at room temperature and the reaction was stirred for 20minutes. After quenching the reaction with a saturated sodium bicarbonate solution and extracting with methylene chloride, the product was purified by column chromatography using methylene chloride/methanol as the mobile phase. A 92% yield (475mg) of desired cyanamide was isolated as a light yellow solid.

The
ethyl-11-cyano-5-(4-(2,6-difluorobenzamido)phenyl)-1,2,3,6-tetrahydropyridine-4-carboxylat e was dissolved in methanol/ 7N ammonia solution (10ml) at room temperature. Hydrogen disulfide was bubbled through the reaction for 30minutes. After capping, the reaction was stirred for an additional 6 hours, after which, the solvent was evaporated. The thiourea product was dissolved in dry THF and 2-bromoacetaldehyde diethylether (3eq) was added and the reaction was refluxed for 5 hours. After quenching with a saturated sodium bicarbonate solution, the aqueous phase was extracted with ethylacetate 3 times and dried over magnesium sulfate. After purification by column chromatography, 205mg of pure product was isolated in a 35% yield for two steps.

205mg of ethyl
5-(4-(2,6-difluorobenzamido)phenyl)-1-(thiazol-2-yl)-1,2,3,6-tetrahydropyridine-4-carboxylat e was dissolved in THF(5ml) and lithium aluminum hydride (50mg, 1.31mmol)was added and stirred for 15minutes when it was quenched with water followed by magnesium sulfate to dry. After filtering through celite, the corresponding allylic alcohol was dissolved in methylene chloride and where excess (>3eq) Dess-Martin reagent was added at room temperature. After 30 minutes the reaction was quenched and extracted with methylene chloride. Purified by column chromatography to produce 63mg of pure aldehyde. 34% yield.
ESMS calcd. (C₂₂H₁₇F₂N₃O₂S): 425.1; found: 426.2(M + H).

### 2,6-difluoro-N-(4-(4-methyl-1-(oxazol-2-yl)-1,2,5,6-tetrahydropyridin-3-yl)phenyl)benzamid e:

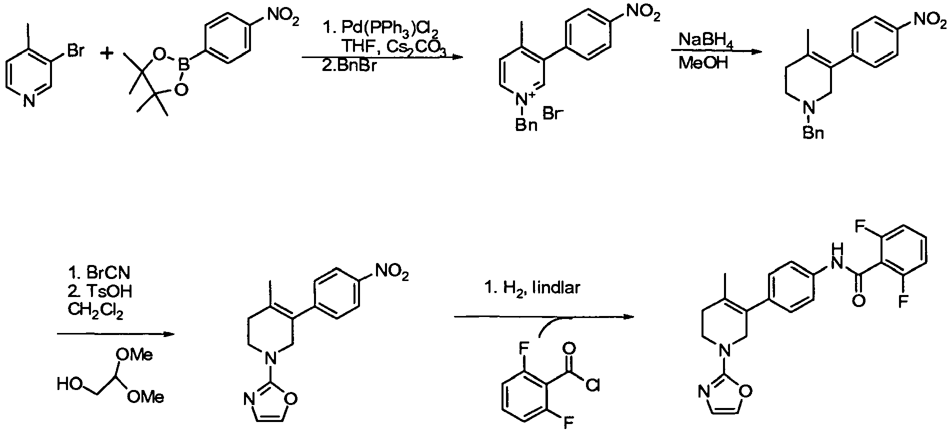

### Compound 1: N-(4-(4-formyl-1-(thiazol-2-yl)-1,2,5,6-tetrahydropyridin-3-yl)phenyl)-5-methylpyrimidine-4-carboxamide

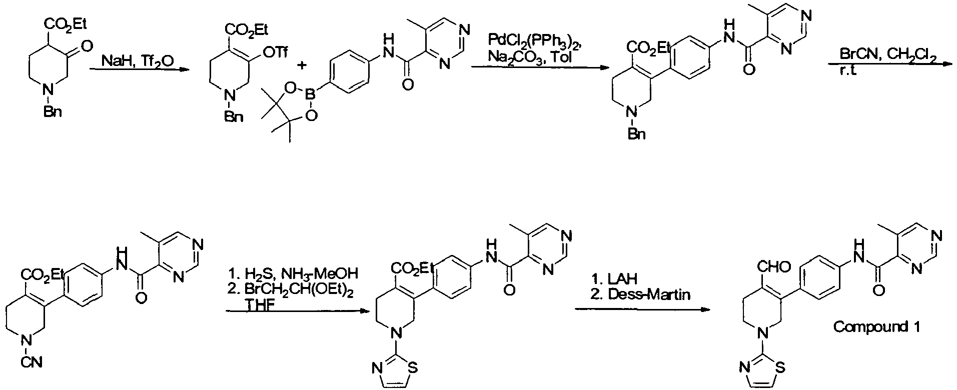

### Compounds 2 and 3:

### Tetrazole synthesis

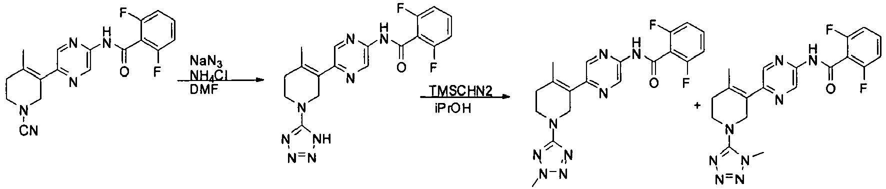

### Procedure:

A solution of DMF(2ml) containing cyanamide(30mg, 0.084mmol), ammonium chloride(16mg, 0.252mmol) and sodium azide(13.5mg, 0.252mmol) was heated to 100°C for 2 hours. The solution was then cooled to room temperature and the DMF was removed on rotary evaporator. Methylene chloride was added followed by water and aqueous workup. After drying with Na₂SO₄ and removing methylene chloride, the residue was dissolved in isopropanol, excess TMS-diazomethane was added and stirred for 12 minutes. The reaction mixture was purified by flash chromatography. Received a 6:1 mixture of isomers. Overall (29mg, 60% yield)

### 2,6-difluoro-N-(5-(4-methyl-1-(2-methyl-2H-tetrazol-5-yl)-1,2,5,6-tetrahydropyridin-3-yl)py razin-2-yl)benzamide (Compound 2)

¹H NMR (300 MHz, CDCl₃) δ 9.67(s, 1H), 8.39 (s, 1H), 8.23-8.25 (m, 1H), 7.52-7.42 (m, 1H), 7.08-7.00 (m, 2H), 4.30-4.20(m 2H), 4.18 (s, 3H), 3.75 (t, *J*=5.7Hz, 2H), 2.40-2.36(m, 2H), 1.82 (s, 3H) ppm
ESMS calcd. (C₁₉H₁₈F₂N₈O): 412.2; found: 413.2(M + H).

### 2,6-difluoro-N-(5-(4-methyl-1-(1-methyl-1H-tetrazol-5-yl)-1,2,5,6-tetrahydropyridin-3-yl)py razin-2-yl)benzamide (Compound 3)

ESMS calcd. (C₁₉H₁₈F₂N₈O): 412.2; found: 413.2(M + H).

### Alternative procedure:

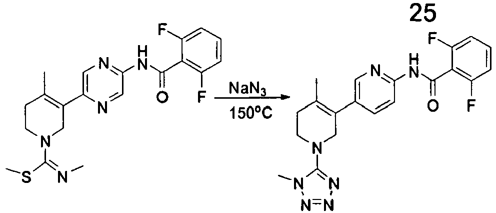

(E)-methyl 3-(5-(2,6-difluorobenzamido)pyrazin-2-yl)-N,4-dimethyl-5,6-dihydropyridine-1(2H)-carbimidothioate was made using same procedure as in Compound 22 (below). 25mg, 0.06mmol was dissolved in isopropanol (1.5ml), sodium azide( 8mg, 0.12mmol) was added to the mixture and heated to 150°C for 1 hour. After cooling, the solvent was removed and the methyl tetrazole was purified via flash chromatography.

### Compound 4: 3-fluoro-N-(5-(4-methyl-1-(thiazol-2-yl)-1,2,5,6-tetrahydropyridin-3-yl) pyrazin-2-yl)isonicotinamide

ESMS calcd. (C₁₉H₁₇FN₆OS): 396.1; found: 397.1(M + H).

**Procedure:** General Procedure for thiazole amine formation from N-(5-(1-cyano-4-methyl-1,2,5,6-tetrahydropyridin-3-yl)pyrazin-2-yl)-3-fluoroisonicotinamide

### Compound 5: N-(2,6-difluorobenzyl)-4-(4-methyl-1-(thiazol-2-yl)-1,2,5,6-tetrahydropyridin-3-yl)aniline

### Procedure for reductive amination

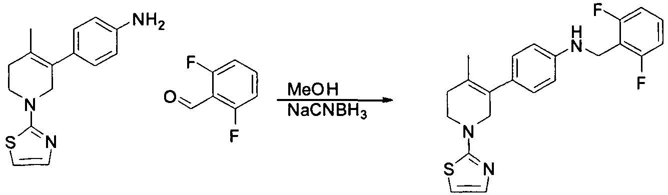

**Procedure:** The aryl amine (35mg, 0.129mmol) was dissolved in 2ml of methanol. At room temperature, (20.2mg, 0.142mmol) was added to the reaction and stirred for 1.5 hours. 13mg, of sodium borohydride was then added and stirred for 15minutes to reduce the imine. The methanol was removed and the compound loaded onto silica gel with methylene chloride. After flash column, 32mg of product was recovered in a 63% yield.
ESMS calcd. (C₂₂H₂₁F₂N₃S): 397.1; found: 398.1(M + H).

### Compound 6: 2,6-difluoro-N-(5-(4-methyl-1-(2-methyl-2H-tetrazol-5-yl)-1,2,5,6-tetrahydropyridin-3-yl)pyridin-2-yl)benzamide

¹H NMR (300 MHz, CDCl₃) δ 9.29 (s, 1H), 8.36 (d, *J*=8.4Hz, 1H), 7.93-7.90 (m, 1H), 7.63 (dd, *J*=4.2, 8.7Hz, 1H), 7.48-7.38 (m, 1H), 7.03-6.95 (m, 2H), 4.20-4.19 (m, 3H), 4.01-4.04 (m, 2H), 3.73 (t, *J*=5.4Hz, 2H) 2.35-2.31 (m, 2H), 1.64 (s, 3H) ppm ESMS calcd. (C₂₀H₁₉F₂N₇O): 411.2; found: 412.2(M + H).
**Procedure:** Performed as with Compound 2 tetrazole synthesis using N-(5-(1-cyano-4-methyl-1,2,5,6-tetrahydropyridin-3-yl)pyridin-2-yl)-2,6-difluorobenzamide.

### Compound 7: N-(2,6-difluorobenzyl)-5-(4-methyl-1-(thiazol-2-yl)-1,2,5,6-tetrahydropyridin-3-yl)pyridin-2-amine dihydrochloride

ESMS calcd. (C₂₁H₂₂Cl₂F₂N₄S): 470.1; found: 399.1(M - 2Cl-H).
**Procedure:** See Procedure for reductive amination (Compound 5) beginning with 5-(4-methyl-1-(thiazol-2-yl)-1,2,5,6-tetrahydropyridin-3-yl)pyridin-2-amine.

### Compound 8: 2-fluoro-N-(4-(4-methyl-1-(thiazol-2-yl)-1,2,5,6-tetrahydropyridin-3-yl)phenyl)-6-(trifluoromethyl)benzamide

ESMS calcd. (C₂₃H₁₉F₄N₃OS): 461.1; found :462.1 (M + H).
**Procedure:** See general procedure for amide formation using EDC and 2-fluoro-6-(trifluoromethyl)benzoic acid.

### Compound 9: N-(4-(4-methyl-1-(thiazol-2-yl)-1,2,5,6-tetrahydropyridin-3-yl)phenyl)-2-(trifluoromethyl)benzamide

ESMS calcd. (C₂₃H₂₀F₃N₃OS): 443.1; found: 444.1 (M + H).
**Procedure:** See general procedure for amide formation using EDC and 2-(trifluoromethyl)benzoic acid

### Compound 10: 2,6-dichloro-N-(4-(4-methyl-1-(thiazol-2-yl)-1,2,5,6-tetrahydropyridin-3-yl)phenyl)benzamide

ESMS calcd. (C₂₂H₁₉Cl₂F₂N₃OS): 443.1; found: 444.1(M + H).
**Procedure:** See general procedure for amide formation using EDC and 2,6-dichlorobenzoic acid.

### Compound 11: 2,6-difluoro-N-(6-(4-methyl-1-(thiazol-2-yl)-1,2,5,6-tetrahydropyridin-3-yl) pyridin-3-yl)benzamide

ESMS calcd. (C₂₁H₁₈F₂N₄OS): 412.1; found: 413.1 (M + H).
**Procedure:** See general procedure for amide formation using 2,6-difluorobenzoyl chloride.

### Compound 12: 2-fluoro-N-(4-(4-methyl-1-(thiazol-2-yl)-1,2,5,6-tetrahydropyridin-3-yl) phenyl)benzamide

ESMS calcd. (C₂₂H₂₀FN₃OS): 393.1; found: 394.1(M + H).

**Procedure:** See general procedure for amide formation using EDC and 2-flurobenzoyl chloride.

### Compound 13:

2-fluoro-6-hydroxy-N-(4-(4-methyl-1-(thiazol-2-yl)-1,2,5,6-tetrahydropyridin-3-yl)phenyl)benzamide

ESMS calcd. (C₂₂H₂₀FN3OS): 409.1; found: 410.1(M + H).
**Procedure:** See general procedure for amide formation using EDC and 2-fluoro-6-hydroxybenzoic acid.

### Compound 14: N-(4-(4-methyl-1-(thiazol-2-yl)-1,2,5,6-tetrahydropyridin-3-yl)phenyl)-2-(methylthio)nicotinamide

ESMS calcd. (C₂₂H₂₂N4OS): 422.1; found: 423.1 (M + H).
**Procedure:** See general procedure for amide formation using EDC and 2-(methylthio)nicotinic acid.

### Compound 15: 2-chloro-N-(4-(4-methyl-1-(thiazol-2-yl)-1,2,5,6-tetrahydropyridin-3-yl)phenyl)butanamide

ESMS calcd. (C₁₉H₂₂ClN₃OS): 375.1; found: 376.1(M + H).
**Procedure:** See general procedure for amide formation using EDC and 2-chlorobutanoic acid.

### Compound 16: 2,4-difluoro-N-(4-(4-methyl-1-(thiazol-2-yl)-1,2,5,6-tetrahydropyridin-3-yl)phenyl)benzamide

ESMS calcd. (C₂₂H₁₉F₂N₃OS): 411.1; found: 412.1(M + H).
**Procedure:** See general procedure for amide formation using EDC and 2,4-difluorobenzoic acid.

### Compound 17: N-(4-(4-methyl-1-(thiazol-2-yl)-1,2,5,6-tetrahydropyridin-3-yl)phenyl)-9H-fluorene-9-carboxamide

ESMS calcd. (C₂₉H₂₅N₃OS): 463.2; found: 464.2.(M + H).
**Procedure:** See general procedure for amide formation using EDC and 9H-fluorene-9-carboxylic acid.

### Compound 18: N-(2'-chloro-5'-(thiazol-2-yl)biphenyl-4-yl)-2-fluoro-6-hydroxybenzamide

ESMS calcd. (C₂₂H₁₄ClFN₂O₂S): 424.0; found: 425.1(M + H).
**Procedure:** See general coupling procedure for boronic esters, and general amide formation using EDC and 2-fluoro-6-hydroxybenzoic acid.

### Compound 19: 2,4-difluoro-N-(4-(4-methyl-1-(thiazol-2-yl)-1,2,5,6-tetrahydropyridin-3-yl)phenyl)benzamide hydrochloride

ESMS calcd. (C₂₂H₂₀ClF₂N₃O): 447.1; found: 412.5(M - Cl).
**Procedure:** See general procedure for amide formation using acid chloride of 2,4-difluorobenzoic acid followed by salt formation as for 3-fluoro-N-(4-(4-methyl-1-(thiazol-2-yl)-1,2,5,6-tetrahydropyridin-3-yl)phenyl)isonicotinamide dihydrochloride.

### Compound 20: 2-chloro-6-fluoro-N-(5-(4-methyl-1-(thiazol-2-yl)-1,2,5,6-tetrahydropyridin-3-yl)pyrazin-2-yl)benzamide

ESMS calcd. (C₂₀H₁₇ClFN₅OS): 429.1; found:430.1 (M + H).
**Procedure:** General thiazole formation beginning from 2-chloro-N-(5-(1-cyano-4-methyl-1,2,5,6-tetrahydropyridin-3-yl)pyrazin-2-yl)-6-fluorobenza mide.

### Compound 21: 2,4-difluoro-N-(5-(4-methyl-1-(2-methyl-2H-tetrazol-5-yl)-1,2,5,6-tetrahydropyridin-3-yl)pyridin-2-yl)benzamide

ESMS calcd. (C₂₀H₁₉F₂N₇O): 411.2; found: 412.2(M + H).
**Procedure:** See (Compound 2) thiazole formation using N-(5-(1-cyano-4-methyl-1,2,5,6-tetrahydropyridin-3-yl)pyrazin-2-yl)-2,4-difluorobenzamide.

### Compound 22:2,6-difluoro-N-(5-(4-methyl-1-(1-methyl-1H-imidazol-2-yl)-1,2,5,6-tetrahydropyridin-3-yl)pyridin-2-yl)benzamide

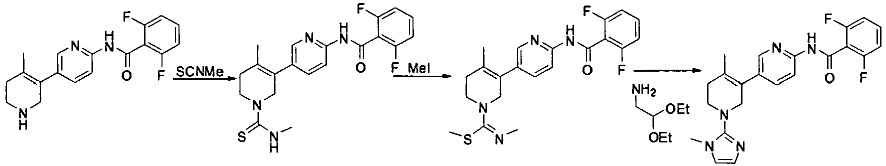

The amine was dissolved in methylene chloride and stirred overnight in the presence of methyl isothiocyanate. The corresponding thiourea was purified by a short column chromotagraphy. Then dissolved in methylene chloride again and stirred with methyl iodide for 3 hours. The methyl iodide was removed along with solvent and isopropanol was added. To this reaction mixture in isopropananol was added the 2-aminoacetaldehyde diethyl acetal. After warming to 35°C for 4 hours, the reaction solvent was removed and the residue was purified by column chromatography.
ESMS calcd. (C₂₂H₂₁F₂N₅O): 409.2; found: 410.2(M + H).

### Compound 23: 2-chloro-6-fluoro-N-(5-(4-methyl-1-(2-methyl-2H-tetrazol-5-yl)-1,2,5,6-tetrahydropyridin-3-yl)pyridin-2-yl)benzamide

### Procedure

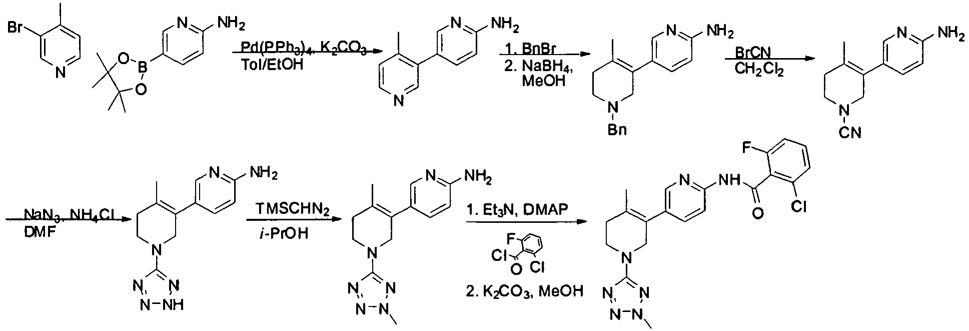

5-(4,4,5,5-tetramethyl-1,3,2-dioxaborolan-2-yl)pyridin-2-amine (1g, 4.55mmol), 3-bromo-4-methylpyridine(782mg, 4.55mmol), Pd₂(dba)₃ (200mg, 4.8%) triphenyl phosphine (229mg,0.874mmol), and potassium carbonate (1.57g, 11.37mmol) were all added to a microwave tube and 15ml of toluene and 1.5ml of ethanol were added and stirred vigorously. The tube was sealed and heated to 150°C for 15min. The potassium carbonate was filtered off and the solvent was removed. The oily product was dissolved in methylene chloride and purified by column chromatography. Isolated 660mg 78%yield.

The pure 4'-methyl-3,3'-bipyridin-6-amine was dissolved in methylene chloride 25ml and benzyl bromide(1.22g, 7.14mmol) was added. After stirring overnight at room temperature the methylene chloride was removed and the remaining mass was stirred in hexanes/diethyl ether and filtered to remove all excess benzyl bromide. This solid was redissolved in methanol and sodium borohydride(340mg, 8.55mmol) was slowly added. After stirring for 1 hour the solvent was removed and methylene chloride was added and stirred. After filtering, it was purified by flash chromatography through a basic alumina column.

5-(1-benzyl-4-methyl-1,2,5,6-tetrahydropyridin-3-yl)pyridin-2-amine (1.5g, 5mmol) was dissolved in methylene chloride(30ml) and cooled to 0°C in an ice bath. Solid cyanogen bromide was added and stirred for 30 minutes. Saturated sodium bicarbonate was added and the methylene chloride was dried with sodium sulfate. Purified with flash chromatography to produce 815mg, 71%yield as a viscous oil.

### (763.2mg, 3.56mmol)

3-(6-aminopyridin-3-yl)-4-methyl-5,6-dihydropyridine-1(2H)-carbonitrile was dissolved in 5 ml of DMF. Sodium azide(695mg, 10.7mmol), and ammonium chloride(566mg, 10.7mmol) were added as a solid and the reaction was heated to 100°C for 1 hour. The DMF was removed and isopropanol was added followed by TMS diazomethane(10.7mmol). The reaction was stirred for 30min then the isopropanol was removed and the product was purified by column chromatography. 400mg(42% 2 step yield) was recovered as a white solid.

5-(4-methyl-1-(2-methyl-2H-tetrazol-5-yl)-1,2,5,6-tetrahydropyridin-3-yl)pyridin-2-amine (31mg, 0.114mmol) was dissolved in 2ml of methylene chloride, Triethylamine(48µl, 0.342mmol), DMAP (3mg, 10%w/w), and 2-chloro-6-fluorobenzoyl chloride(44.1mg, 0.229mmol) were then added to the flask at room temperature and stirred for 1hour. Quenched with sodium bicarbonate(sat) solution. The methylene chloride layer was then separated and 10ml of methanol plus 150ml of potassium carbonate were added. The reaction was heated to 60°C for 15min. The methanol was then removed and the compound was purified by flashed column chromatography. Isolated 30mg as a 61% yield.
ESMS calcd. (C₂₀H₁₉ClFN₇O): 427.1; found: 428.1(M + H).

### Compound 24: 2,4-difluoro-N-(5-(4-methyl-1-(2-methyl-2H-tetrazol-5-yl)-1,2,5,6-tetrahydropyridin-3-yl)pyrazin-2-yl)benzamide

### Procedure

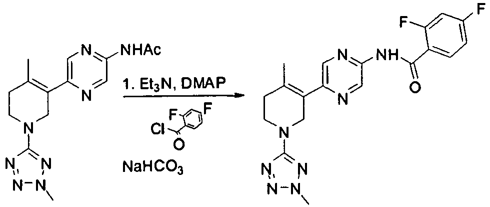

N-(5-(4-methyl-1-(2-methyl-2H-tetrazol-5-yl)-1,2,5,6-tetrahydropyridin-3-yl)pyrazin-2-yl)ac etamide (203mg, 0.645mmol) was dissolved in 5ml of methylene chloride. Triethylamine (272µl, 1.94mmol) and DMAP (7mg, 0.065mmol) were added, followed by 2,4-diflurobenzoyl chloride(227mmg, 1.29mmol). After 35min at room temperature, the reaction was quenched with sodium bicarbonate solution (saturated) and stirred overnight. Purified by column chromatography to isolate 200mg 75% yield of pure amide.
ESMS calcd. (C₁₉H₁₈F₂N₈O): 412.2; found: 413.3(M + H).

### Compound 25: N-(5-(4-chloro-1-(2-methyl-2H-tetrazol-5-yl)-1,2,5,6-tetrahydropyridin-3-yl)pyridin-2-yl)-2,6-difluorobenzamide

ESMS calcd. (C₁₉H₁₆ClF₂N₇O): 431.1; found: 432.1(M + H).
**Procedure:** Same as for Compound 23 using 3-bromo-4-chloropyridine for initial step.

### Compound 26: N-(5-(4-chloro-1-(thiazol-2-yl)-1,2,5,6-tetrahydropyridin-3-yl) pyridin-2-yl)-2,6-difluorobenzamide

ESMS calcd. (C₂₀H₁₅ClF₂N₄OS): 432.1; found: 433.1M + H).
**Procedure:** Same general thiazole synthesis using
N-(5-(4-chloro-1-cyano-1,2,5,6-tetrahydropyridin-3-yl)pyridin-2-yl)-2,6-difluorobenzamide

### Compound 27: 2-chloro-6-fluoro-N-(5-(4-methyl-1-(2-methyl-2H-tetrazol-5-yl)-1,2,5,6-tetrahydropyridin-3-yl)pyridin-2-yl)benzamide

ESMS calcd. (C₂₀H₁₅ClF₂N₄OS): 432.1; found: 433.1(M + H).
**Procedure:** Same general thiazole synthesis using
N-(5-(4-chloro-1-cyano-1,2,5,6-tetrahydropyridin-3-yl)pyridin-2-yl)-2,4-difluorobenzamide

### Compound 28: 3-methyl-N-(5-(4-methyl-1-(2-methyl-2H-tetrazol-5-yl)-1,2,5,6-tetrahydropyridin-3-yl)pyridin-2-yl)but-2-enamide

ESMS calcd. (C₁₈H₂₃N₇O): 353.2; found:354.2 (M + H).
**Procedure:** Same as Compound 23 using 3-methylbut-2-enoyl chloride.

### Compound 29:

2-fluoro-N-(5-(4-methyl-1-(2-methyl-2H-tetrazol-5-yl)-1,2,5,6-tetrahydropyridin-3-yl)pyridin-2-yl)benzamide
ESMS calcd. (C₂₀H₂₀FN₇O): 393.2; found: 394.2(M + H).
**Procedure:** Same as Compound 23 using 2-fluorobenzoyl chloride.

### Compound 30: 2,6-difluoro-N-(5-(4-methyl-1-(oxazol-2-yl)-1,2,5,6-tetrahydropyridin-3-yl)pyrazin-2-yl)benzamide

ESMS calcd. (C₂₀H₁₇F₂N₅O₂): 397.1; found:398.1 (M + H).
**Procedure:** See general synthesis for oxazole amine using
N-(5-(1-cyano-4-methyl-1,2,5,6-tetrahydropyridin-3-yl)pyrazin-2-yl)-2,6-difluorobenzamide

### Compound 31: 2-fluoro-N-(5-(4-methyl-1-(thiazol-2-yl)-1,2,5,6-tetrahydropyridin-3-yl)pyrazin-2-yl)benzamide

ESMS calcd. (C₂₀H₁₈FN₅OS):395.1; found: 396.1(M + H).
**Procedure:** See general synthesis for thiazoleamine using
N-(5-(1-cyano-4-methyl-1,2,5,6-tetrahydropyridin-3-yl)pyrazin-2-yl)-2-fluorobenzamide.

### Compound 32:3,5-difluoro-N-(4-(4-methyl-1-(thiazol-2-yl)-1,2,5,6-tetrahydropyridin-3-yl)phenyl)isonicotinamide

ESMS calcd. (C₂₁H₁₉F₂N₄OS): 412.1; found: 413.1(M + H).
**Procedure:** See amide formation using EDC procedure using 3,5-difluoroisonicotinoyl chloride.

### Compound 33:

2-fluoro-N-(5-(4-methyl-1-(2-methyl-2H-tetrazol-5-yl)-1,2,5,6-tetrahydropyridin-3-yl)pyrazin-2-yl)benzamide
ESMS calcd. (C₁₉H₁₉FN₈O): 394.2; found: 395.2(M + H).
**Procedure:** See Compound 2 tetrazole procedure using
N-(5-(1-cyano-4-methyl-1,2,5,6-tetrahydropyridin-3-yl)pyrazin-2-yl)-2-fluorobenzamide.

### Compound 34: 3,5-difluoro-N-(5-(4-methyl-1-(thiazol-2-yl)-1,2,5,6-tetrahydropyridin-3-yl) pyrazin-2-yl)isonicotinamide hydrochloride

ESMS calcd. (C₁₉H₁₇ClF₂N₆OS): 450.1; found: 414.11(M - Cl).
**Procedure:** The procedure for the synthesis of
3-fluoro-N-(4-(4-methyl-1-(thiazol-2-yl)-1,2,5,6-tetrahydropyridin-3-yl)phenyl)isonicotinamide dihydrochloride was followed.

### Compound 35: 2-chloro-6-fluoro-N-(5-(4-methyl-1-(2-methyl-2H-tetrazol-5-yl)-1,2,5,6-tetrahydropyridin-3-yl)pyrazin-2-yl)benzamide

¹H NMR (300 MHz, CDCl₃) δ 9.69 (s, 1H), 8.31 (s, 1H), 8.21 (s, 1H), 7.39-7.45 (m, 1H), 7.30 (d, *J*=6.0Hz, 1H), 7.14 (t, *J*=6.3Hz, 1H), 4.28-4.31 (m, 2H), 4.19 (s, 3H), 3.76 (t, *J*=4.5Hz, 2H), 2.35-2.43 (m, 2H), 1.82 (s, 3H)ppm
ESMS calcd. (C₁₉H₁₈ClFN₈O): 428.1; found: 429.(M +H).

### Procedure

See Tetrazole synthesis (Compounds 2 and 3)

### Compound 36: 2-methyl-N-(4-(4-methyl-1-(thiazol-2-yl)-1,2,5,6-tetrahydropyridin-3-yl)phenyl)benzamide

¹H NMR (300 MHz, CDCl₃) δ
ESMS (C₂₃H₂₃N₃OS): calcd. 389.2; found 399.2 (M+H)
**Procedure:** See procedure for 2,6-difluoro-N-(4-(4-methyl-1-(thiazol-2-yl)-1,2,5,6-tetrahydropyridin-3-yl)phenyl)benzamide hydrochloride

### Compound 37: 3,5-difluoro-N-(4-(4-methyl-1-(thiazol-2-yl)-1,2,5,6-tetrahydropyridin-3-yl)phenyl)isonicotinamide hydrochloride

¹H NMR (300 MHz, CDCl₃) δ 8.94 (s, 1H), 8.39 (s, 2H), 7.65 (d, *J*=6.3Hz, 2H), 7.17 (d, *J*=6.3Hz, 2H), 7.09 (d, *J*=3Hz, 1H), 6.48 (d, *J*=3Hz, 1H), 4.06-4.09 (m, 2H), 3.65 (t, *J*=4.5Hz, 2H), 2.30-2.40 (m, 2H), 1.66 (s, 3H)ppm
ESMS calcd. (C₂₁H₁₉ClF₂N₄OS) 448.1; found: 413.1(M-Cl).
**Procedure** See procedure for 2,6-difluoro-N-(4-(4-methyl-1-(thiazol-2-yl)-1,2,5,6-tetrahydropyridin-3-yl)phenyl)benzamide hydrochloride

### Compound 38: 2,6-difluoro-N-(4-(4-isopropyl-1-(thiazol-2-yl)-1,2,5,6-tetrahydropyridin-3-yl)phenyl)benzamide

¹H NMR (300 MHz, CDCl₃) δ 7.70 (bs, 1H), 7.64 (d, *J*=6.3Hz, 2H), 7.40-7.47(m, 1H), 7.20-7.22 (m, 3H), 7.01 (t, *J*=6Hz, 2H), 6.54 (d, *J*=2.7Hz, 1H), 4.01 (s, 2H), 3.73 (t, *J*=4.5Hz, 2H), 2.61-2.68 (m, 1H), 2.32-2.33 (m, 2H), 0.94 (d, *J*=5.1Hz, 6H) ppm
ESMS (C₂₄H₂₃F₂N₃OS): calcd. 439.2; found 440.2 (M+H)
**Procedure:** See procedure for 2,6-difluoro-N-(4-(4-methyl-1-(thiazol-2-yl)-1,2,5,6-tetrahydropyridin-3-yl)phenyl)benzamide hydrochloride beginning from 3-bromo-4-isopropyl pyridine.

### Compound 39: 3,5-difluoro-N-(4-(4-isopropyl-1-(thiazol-2-yl)-1,2,5,6-tetrahydropyridin-3-yl)phenyl)isonicotinamide

ESMS (C₂₃H₂₂F₂N₄OS): calcd. 440.2; found 441.1 (M+H)
**Procedure:** See procedure for 2,6-difluoro-N-(4-(4-methyl-1-(thiazol-2-yl)-1,2,5,6-tetrahydropyridin-3-yl)phenyl)benzamide hydrochloride beginning from 3-bromo-4-isopropyl pyridine.

### Compound 40: 2,4-difluoro-N-(4-(4-isopropyl-1-(thiazol-2-yl)-1,2,5,6-tetrahydropyridin-3-yl)phenyl)benzamide hydrochloride

ESMS (C₂₄H₂₄ClF₂N₃OS): calcd. 475.1; found 440.2 (M-Cl)
**Procedure:** See procedure for 2,6-difluoro-N-(4-(4-methyl-1-(thiazol-2-yl)-1,2,5,6-tetrahydropyridin-3-yl)phenyl)benzamide hydrochloride beginning from 3-bromo-4-isopropyl pyridine.

### Compound 41: 2,4-difluoro-N-(5-(4-methyl-1-(thiazol-2-yl)-1,2,5,6-tetrahydropyridin-3-yl)pyrazin-2-yl)benzamide

¹H NMR (300 MHz, CDCl₃) δ 9.68 (d, *J*=0.6Hz, 1H), 8.96 (bd, *J*=10.8Hz, 1H), 8.30 (d, *J*= 0.6Hz, 1H), 8.22-8.28 (m, 1H), 7.23 (d, *J*=2.4Hz, 1H), 7.10 (dd, *J*=1.8, 6.2 Hz, 1H), 6.99 (dd, *J*=1.8, 6.3Hz, 1H), 6.58 (d, *J*=2.7Hz, 1H), 4.27-4.32 (m, 2H), 3.78 (t, 4.2Hz, 2H), 2.40-2.45 (m, 2H), 1.89 (s, 3H)ppm
ESMS (C₂₀H₁₇F₂N₅OS): calcd. 413.1; found 414.2 (M+H)
**Procedure:** See procedure for 3-fluoro-N-(4-(4-methyl-1-(thiazol-2-yl)-1,2,5,6-tetrahydropyridin-3-yl)phenyl)isonicotinamide dihydrochloride.

### Compound 42:

2,4-difluoro-N-(5-(4-methyl-1-(4-methylthiazol-2-yl)-1,2,5,6-tetrahydropyridin-3-yl)pyrazin-2-yl)benzamide
ESMS (C₂₁H₁₉F₂N₅OS): calcd. 427.1; found 428.2 (M+H)
**Procedure:** See procedure for 3-fluoro-N-(4-(4-methyl-1-(thiazol-2-yl)-1,2,5,6-tetrahydropyridin-3-yl)phenyl)isonicotinamide dihydrochloride. Final step uses chloroacetone instead of bromoacetaldehyde diethyl acetal.

### Compound 43: 2,4-difluoro-N-(5-(4-methyl-1-(thiazol-2-yl)-1,2,5,6-tetrahydropyridin-3-yl)pyrazin-2-yl)benzamide hydrochloride

ESMS (C₂₀H₁₈ClF₂N₅OS): calcd. 449.09; found 414.1 (M-Cl)
**Procedure:** See procedure for 3-fluoro-N-(4-(4-methyl-1-(thiazol-2-yl)-1,2,5,6-tetrahydropyridin-3-yl)phenyl)isonicotinamide dihydrochloride

### Compound 44: 2,6-difluoro-N-(4-(4-isopropyl-1-(thiazol-2-yl)-1,2,5,6-tetrahydropyridin-3-yl)phenyl)benzamide hydrochloride

ESMS (C₂₄H₂₄ClF₂N₃O): calcd. 475.1; found 440.2 (M-Cl)
**Procedure:** See procedure for 2,6-difluoro-N-(4-(4-methyl-1-(thiazol-2-yl)-1,2,5,6-tetrahydropyridin-3-yl)phenyl)benzamide hydrochloride

### Compound 45: 2,6-difluoro-N-(5-(4-isopropyl-1-(thiazol-2-yl)-1,2,5,6-tetrahydropyridin-3-yl)pyrazin-2-yl)benzamide

¹H NMR (300 MHz, CDCl₃) δ 9.68 (s, 1H), 8.35-8.36 (m, 1H), 8.24 (s, 1H), 7.46-7.53 (m, 1H), 7.21-7.22(m, 1H), 7.06 (t, *J*= 6.3Hz 2H), 6.56 (d, *J*=2.7Hz, 1H), 4.22(s, 2H), 3.77 (t, *J*=4.2Hz, 2H), 2.70-2.77 (m, 1H), 2.41-2.43 (m, 2H), 1.03 (d, *J*=5.1Hz, 6H) ppm. ESMS calcd. (C₂₂H₂₁F₂N₅OS) 441.1; found: 442.2(M +H).

### Procedure.

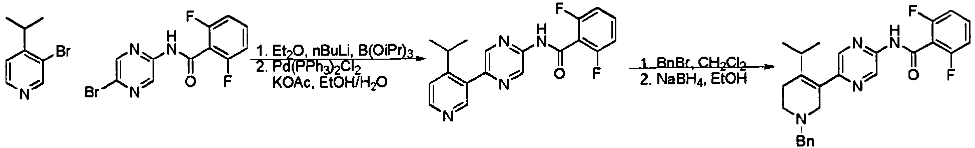

The 3-bromo-4-isopropylpyridine (500mg, 2.5mmol) was dissolved in dithethylether (2ml) and cooled to -78°C, n-BuLi (2.5mmol in hex) was slowly added and stirred for 15minutes. After which, isopropyl borate (577ul, 2.5mmol) was added and warmed to room temperature for 30 minutes and water(1ml) was added and the ether was evaporated off. LCMS shows good conversion to the boronic acid. The aqueous solution was transferred to a microwave vial and (5ml) ethanol was added, followed by palladium bistriphenylphoshine dichloride (123mg, 0.175mmol), the bromide (550mg, 0.175mmol) and potassium acetate (400mg, 5mmol)). The vessel was sealed and heated in microwave for 5000 seconds at 80°C. After cooling the ethanol was evaporated and the residue was extracted with methylene chloride. Purified by column choromtagraphy using methylene chloride/ethyl acetate 25%. Isolated 454mg of coupled product.

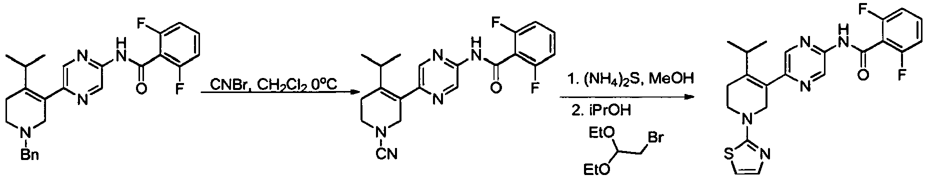

The benzyl amine (140mg, 0.312mmol) was dissolved in 2ml methylene chloride and cooled to 0°C. Cyanogen bromde (66mg, 0.624mmol) was added as a solid and stirred for an additional 15minutes. The reaction was quenched with a saturated solution of sodium bicarbonated and purified by column chromatography. Recovered 119mg of pure cyanamide (100%). In a sealed tube, the cyanamide(90mg, 0.24mmol) in 2ml of ethanol was headed to 35°C for 1hour and cooled. The amount of ethanol was reduced and distilled water added. The solid was filtered off and dried. This solid was sealed in a microwave tube with 2ml of isopropanol and bromoacetaldehyde diethyl acetal (1.06ml, 0.71mmol) and heated to 100°C for 30 minutes. The reaction was cooled and neutralized with saturated sodium bicarbonate solution. After extraction with methylene chloride and drying, the residue was purified by column chromatography. 59mg of pure product was recovered (57%yield for two step)

### Compound 46: 2,6-difluoro-N-(5-(4-isopropyl-1-(2-methyl-2H-tetrazol-5-yl)-1,2,5,6-tetrahydropyridin-3-yl)pyrazin-2-yl)benzamide

¹H NMR (300 MHz, CDCl₃) δ 8.44 (s, 1H), 8.21 (s, 1H), 7.45-7.53 (m, 1H), 7.05 (t, *J*=6Hz, 2H), 4.22-4.24(m, 2H), 4.18 (s, 3H), 3.75 (t, *J*=4.2Hz, 2H), 2.67-2.73(m, 1H), 2.36-2.39 (m, 2H), 1.01(d, *J*= 5.Hz, 6H) ppm. ESMS calcd. (C₂₁H₂₂F₂N₈O) 440.2; found: 441.2(M +H).

### Procedure.

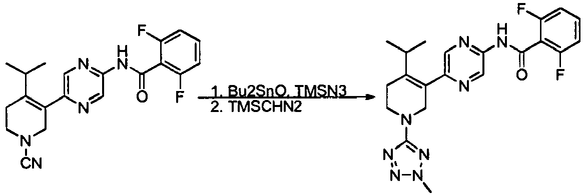

The cyanamide was dissolved in THF and trimethylsilylazide was added followed by a catalytic amount of dibutyltin oxide. After warming to 30°C for 2 hours the reaction was completed. After base/acid extraction, the tetrazole-H was dissolved in isopropanol and TMS diazomethane was added at room temp. The reaction was stirred for 30minutes and the solvent was evaporated. Purified by column chromatography. Isolated 2 isomers in a 5:1 ratio. Major product was isolated in a 50%yield.

### Compound 47: 2-chloro-N-(4-(4-methyl-1-(thiazol-2-yl)-1,2,5,6-tetrahydropyridin-3-yl)phenyl)butanamide hydrochloride

¹H NMR (300 MHz, CDCl₃) δ 8.70 (s, 1H), 7.63(d, *J*= 6.0Hz, 2H), 7.33 (d, *J*=3.3Hz, 1H), 7.23 (d, *J*=6Hz, 2H), 6.66(d, *J*=3.3Hz, 1H), 4.52-4.55 (m, 1H), 4.09-4.29 (m, 4H), 2.45-254(m, 2H), 2.01-2.25 (m, 2H), 1.70 (s, 3H), 1.11 (t, *J*=5.4Hz, 3H)ppm ESMS calcd. (C₁₉H₂₃Cl₂N₃OS) 411.1; found: 376.3(M -Cl).

### Procedure

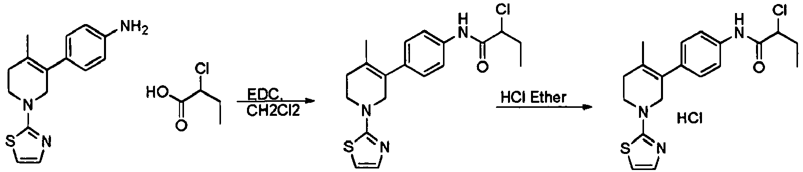

The phenyl amine (200mg, 0.737mmol) was dissolved in methylene chloride (10ml) along with (282.5mag, 1.47mmol) EDC, and alpha-chlorobutryic acid (180.6mg, 1.47mmol). Stirred for 4 hours then quenched with sodium bicarbonated aqueous solution. Purified by column chromotography to produce a colorless oil. The oil was dissolved in ether and HCl in ether was added. The precep was dried on vacuum. Isolated 251mg of pure salt in a 83% yield.

### Compound 48: 2-chloro-N-(4-(4-methyl-1-(thiazol-2-yl)-1,2,5,6-tetrahydropyridin-3-yl)phenyl)propanamide hydrochloride

¹H NMR (300 MHz, CDCl₃) δ 8.29 (s, 1H), 7.55 (d, *J*= 6.3Hz, 2H), 7.21-7.23 (m, 3H), 6.55 (d, *J*=2.4Hz, 1H), 4.57 (q, *J*=5.4Hz, 1H), 4.06 (s, 2H), 3.73 (t,*J*= 4.3Hz, 2H), 2.32-2.38 (m, 2H), 1.84 (d, *J*=5.4Hz, 3H), 1.65 (s, 3H)ppm
ESMS calcd. (C₁₈H₂₁Cl₂N₃OS) 397.1; found: 362.3(M -Cl).
**Procedure** is the same as Compound 47.

### EXAMPLE 2: INHIBITION OF IL-2 PRODUCTION

Jurkat cells were placed in a 96 well plate (0.5 million cells per well in 1% FBS medium) then a test compound of this invention was added at different concentrations. After 10 minutes, the cells were activated with PHA (final concentration 2.5 µg/mL) and incubated for 20 hours at 37°C under CO₂. The final volume was 200 µL. Following incubation, the cells were centrifuged and the supernatants collected and stored at -70°C prior to assaying for IL-2 production. A commercial ELISA kit (IL-2 Eli-pair, Diaclone Research, Besancon, France) was used to detect production of IL-2, from which dose response curves were obtained. The IC₅₀ value was calculated as the concentration at which 50% of maximum IL-2 production after stimulation was inhibited versus a non-stimulation control.

Inhibition of other cytokines, such as IL-4, IL-5, IL-13, GM-CSF, TNF-α, and INF- γ, can be tested in a similar manner using a commercially available ELISA kit for each cytokine.

| Compound # | IC₅₀ (nM) |
|---|---|
| 4, 5, 6, 12, 15, 16, 19, 20, 28, 25, 26, 32 | ≤ 35 |
| 2, 7, 9, 29, 30, 31 | 35 < and ≤ 75 |
| 1, 8, 10, 11, 18, 24, 27, 33 | 75 < and ≤ 150 |
| 13, 14, 17 | 150< and < 1000 |
| 3, 22, 23, 28 | > 1000 |

### EXAMPLE 3: PATCH CLAMP STUDIES OF INHIBITION OF I_{CRAC} CURRENT IN RBL CELLS, JURKAT CELLS, AND PRIMARY T CELLS

In general, a whole cell patch clamp method is used to examine the effects of a compound of the invention on a channel that mediates I_{crac}. In such experiments, a baseline measurement is established for a patched cell. Then a compound to be tested is perfused (or puffed) to cells in the external solution and the effect of the compound on I_{crac} is measured. A compound that modulates I_{crac} (e.g., inhibits) is a compound that is useful in the invention for modulating CRAC ion channel activity.

### 1) RBL cells

### Cells

Rat basophilic leukemia cells (RBL-2H3) were grown in DMEM media supplemented with 10% fetal bovine serum in an atmosphere of 95% air/5% CO₂. Cells were seeded on glass coverslips 1-3 days before use.

### Recording Conditions

Membrane currents of individual cells were recorded using the whole-cell configuration of the patch clamp technique with an EPC10 (HEKA Electronik, Lambrecht, Germany). Electrodes (2-5 MΩ□ in resistance) were fashioned from borosilicate glass capillary tubes (Sutter Instruments, Novato, Ca). The recordings were done at room temperature.

### Intracellular pipette solution

The intracellular pipette solution contained Cs-Glutamate 120mM; CsCl 20mM; CsBAPTA 10mM; CsHEPES 10mM; NaCl 8mM; MgCl₂ 1mM; IP3 0.02mM; pH=7.4 adjusted with CsOH. The solution was kept on ice and shielded from light before the experiment was preformed.

### Extracellular solution

The extracellular solution contained NaCl 138mM; NaHEPES, 10mM; CsCl 10MM; CaCl₂ 10mM; Glucose 5.5mM; KCl 5.4mM; KH₂PO₄ 0.4mM; Na₂HPO₄˙H₂O 0.3mM at pH=7.4 adjusted with NaOH.

### Compound treatment

Each compound was diluted from a 10 mM stock in series using DMSO. The final DMSO concentration was always kept at 0.1 %.

### Experimental procedure

I_{CRAC} currents were monitored every 2 seconds using a 50 msec protocol, where the voltage was ramped from -100 mV to +100 mV. The membrane potential was held at 0 mV between the test ramps. In a typical experiment, the peak inward currents would develop within 50-100 seconds. Once the I_{CRAC} currents were stabilized, the cells were perfused with a test compound in the extracellular solution. At the end of an experiment, the remaining I_{CRAC} currents were then challenged with a control compound (SKF96365, 10 µM) to ensure that the current could still be inhibited.

### Data analysis

The I_{CRAC} current level was determined by measuring the inward current amplitude at -80 mV of the voltage ramp in an off-line analysis using MATLAB. The I_{CRAC} current inhibition for each concentration was calculated using peak amplitude in the beginning of the experiment from the same cell. The IC₅₀ value and Hill coefficient for each compound was estimated by fitting all the individual data points to a single Hill equation.

### Results

Compound 1 had an IC₅₀ of 150 nM.

### 2) Jurkat cells

### Cells

Jurkat T cells are grown on glass coverslips, transferred to the recording chamber and kept in a standard modified Ringer's solution of the following composition: NaCl 145mM, KCl 2.8mM, CsCl 10mM, CaCl₂ 10mM, MgCl₂ 2mM, glucose 10mM, HEPES-NaOH 10mM, pH 7.2.

### Extracellular Solution

The external solution contains 10 mM CaNaR, 11.5 mM glucose and a test compound at various concentrations.

### Intracellular Pipette Solution

The standard intracellular pipette solution contains: Cs-glutamate 145 mM, NaCl 8 mM, MgCl₂ 1 mM, ATP 0.5 mM, GTP 0.3 mM, pH 7.2 adjusted with CsOH. The solution is supplemented with a mixture of 10 mM Cs-BAPTA and 4.3-5.3 mM CaCl₂ to buffer [Ca²⁺]i to resting levels of 100-150 nM.

### Patch-clamp recordings

Patch-clamp experiments are performed in the tight-seal whole-cell configuration at 21-25 °C. High-resolution current recordings are acquired by a computer-based patch-clamp amplifier system (EPC-9, HEKA, Lambrecht, Germany). Sylgard®- coated patch pipettes have resistances between 2-4 MΩ after filling with the standard intracellular solution. Immediately following establishment of the whole-cell configuration, voltage ramps of 50 ms duration spanning the voltage range of-100 to +100 mV are delivered from a holding potential of 0 mV at a rate of 0.5 Hz over a period of 300 to 400 seconds. All voltages are corrected for a liquid junction potential of 10 mV between external and internal solutions. Currents are filtered at 2.3 kHz and digitized at 100 µs intervals. Capacitive currents and series resistance are determined and corrected before each voltage ramp using the automatic capacitance compensation of the EPC-9.

### Data analysis

The very first ramps before activation of I_{CRAC} (usually 1 to 3) are digitally filtered at 2 kHz, pooled and used for leak-subtraction of all subsequent current records. The low-resolution temporal development of inward currents is extracted from the leak-corrected individual ramp current records by measuring the current amplitude at -80 mV or a voltage of choice.

### 3) Primary T Cells

### Preparation of Primary T Cells

Primary T cells are obtained from human whole blood samples by adding 100µL of RosetteSep® human T cell enrichment cocktail to 2 mL of whole blood. The mixture is incubated for 20 minutes at room temperature, then diluted with an equal volume of PBS containing 2% FBS. The mixture is layered on top of RosetteSep® DM-L density medium and then centrifuged for 20 minutes at 1200 g at room temperature. The enriched T cells are recovered from the plasma/density medium interface, then washed with PBS containing 2% FBS twice, and used in patch clamp experiments following the procedure described for RBL cells.

### EXAMPLE 4: ORAL BIOAVAILABILITY

The pharmacokinetic profile of 3-fluoro-N-(4-(4-methyl-1-(thiazol-2-yl)-1,2,5,6-tetrahydro pyridin-3-yl)phenyl)isonicotinamide dihydrochloride was studied in male SD rats. Rats were bled at 5 min (IV only), 15 min, 30 min, 1 h, 2 h, 4 h, 6 h, 8 h and 24 h. Samples were prepared by mixing 50 µL of plasma, 5 µL, DMSO, and 250 µL of ACN, followed by shaking for 5 min, centrifuging at 4400 rpm for 15 min at 8 °C. 225 µL of supernatant was vacuum dried, reconstituted with 180 µL of 20% ACN (10mM NH₄OAc), shaken for ∼2 min and transferred to 96MT plate. Samples were analyzed by HPLC, Phenomenex Onyx Monolithic C18, 4.6x100 mm column eluted with 30-95% ACN (10mM NH₄OAc) gradient. Detection was at 280nm.

### Summary

The following table summarizes the data obtained for
3-fluoro-N-(4-(4-methyl-1-(thiazol-2-yl)-1,2,5,6-tetrahydropyridin-3-yl)phenyl)isonicotinami de dihydrochloride:

| **Route** | **Dose (mg/kg)** | **Formulation** | **t_{1/2} (hr)** | **Tₘₐₓ (hr)** | **Cₘₐₓ (µmol/L)** | **AUCₗₐₛₜ (hr*µmol/L)** | **AUC_{inf} (hr*µmol/L)** | **%F** |
|---|---|---|---|---|---|---|---|---|
| IV | 5 | DRD | 1.0 | 0.083 | 34.6 | 37.5 | 37.6 | -- |
| PO | 50 | 0.5% MC | 3.2 | 2.0 | 43.5 | 348 | 351 | 93.4 % |

3-fluoro-N-(4-(4-methyl-1-(thiazol-2-yl)-1,2,5,6-tetrahydropyridin-3-yl)phenyl)isonicotinami de dihydrochloride showed high oral bioavailability in 0.5% MC (93.4%).

### EXAMPLE 5: SOLUBILITY

3-fluoro-N-(4-(4-methyl-1-(thiazol-2-yl)-1,2,5,6-tetrahydropyridin-3-yl)phenyl)isonicotinami de dihydrochloride had a solubility of > 12 mg/kg. The following table shows the solubility of other salts of
3-fluoro-N-(4-(4-methyl-1-(thiazol-2-yl)-1,2,5,6-tetrahydropyridin-3-yl)phenyl)isonicotinami de.

| **Salt** | **Water Solubility (mg/mL)** |
|---|---|
| HBr | 11.56 |
| H₂SO₄ | 12.69 |
| Phosphoric Acid | 3.21 |
| Succinate | 0.36 |
| Citrate | 1.19 |
| Tartrate | 1.34 |
| Methanesulfonic Acid | 14.01 |

### EXAMPLE 6: SOLUBILITY in SIMULATED GASTRIC FLUIDS

| **Compound** | **Solubility (µM)** |
|---|---|
| 23, 31, 34, 35 | 25 |
| 2, 4, 5, 6, 10, 12, 13, 24, 25, 27, 28, 29 | 50 |
| 7, 11, 26 | 100 |
| 15, 16, 19, 20, 21, 30, 32, 33 | 150 |

### EXAMPLE 7: INHIBITION OF MULTIPLE CYTOKINES IN PRIMARY HUMAN PBMCs

Peripheral blood mononuclear cells (PBMCs) are stimulated with phytohemagglutinin (PHA) in the presence of varying concentrations of compounds of the invention or cyclosporine A (CsA), a known inhibitor of cytokine production. Cytokine production is measured using commercially available human ELISA assay kits (from Cell Science, Inc.) following the manufacturers instructions.

The compounds of the invention are expected to be potent inhibitors of IL-2, IL-4, IL-5, IL-13, GM-CSF, INF-α and TNF- γ in primary human PBM cells. In addition, compounds of the invention are not expected to inhibit the anti-inflammatory cytokine, IL-10.

### EXAMPLE 8: INHIBITION OF DEGRANULATION IN RBL CELLS

### Procedure:

The day before the assay is performed, RBL cells, that have been grown to confluence in a 96 well plate, are incubated at 37°C for at least 2 hours. The medium is replaced in each well with 100 µL of fresh medium containing 2µLg/mL of anti-DNP IgE.

On the following day, the cells are washed once with PRS (2.6 mM glucose and 0.1% BSA) and 160µL of PRS is added to each well. A test compound is added to a well in a 20µL solution at 10X of the desired concentration and incubated for 20 to 40 minutes at 37°C. 20µL of 10X mouse anti-IgE (10µL/mL) is added. Maximum degranulation occurs between 15 to 40 minutes after addition of anti-IgE.

Compounds of the invention are expected to inhibit degranulation.

### EXAMPLE 9: INHIBITION OF CHEMOTAXIS IN T CELLS

### T-cell isolation:

Twenty ml aliquots of heparinized whole blood (2 pig, 1 human) are subjected to density gradient centrifugation on Ficoll Hypaque. The buffy coat layers representing peripheral blood mononuclear cells (PBMCs) containing lymphocytes and monocytes are washed once, resuspended in 12 ml of incomplete RPMI 1640 and then placed in gelatin-coated T75 culture flasks for 1 hr at 37°C. The non-adherent cells, representing peripheral blood lymphocytes (PBLs) depleted of monocytes, are resuspended in complete RPMI media and placed in loosely packed activated nylon wool columns that have been equilibrated with warm media. After 1 hr at 37°C, the non-adherent T cell populations are eluted by washing of the columns with additional media. The T cell preparations are centrifuged, resuspended in 5 ml of incomplete RPMI, and counted using a hemocytometer.

### Cell migration assay:

Aliquots of each T cell preparation are labeled with Calcien AM (TefLabs) and suspended at a concentration of 2.4 x10⁶/ml in HEPES-buffered Hank's Balanced Salt Solution containing 1.83 mM CaCl₂ and 0.8 mM Much, pH 7.4 (HHBSS). An equal volume of HHBSS containing 0, 20 nM, 200 nM or 2000 nM of compound 1 or 20 nM EDTA is then added and the cells incubated for 30 min at 37 °C. Fifty µl aliquots of the cell suspensions (60,000 cells) are placed on the membrane (pore size 5 µm) of a Neuroprobe ChemoTx 96 well chemotaxis unit that have been affixed over wells containing 10 ng/ml MIP-1α in HHBSS. The T cells are allowed to migrate for 2 hr at 37 °C, after which the apical surface of the membrane is wiped clean of cells. The chemotaxis units are then placed in a CytoFlour 4000 (PerSeptive BioSystems) and the fluorescence of each well measured (excitation and emission wavelengths of 450 and 530 nm, respectively). The number of migrating cells in each well is determined from a standard curve generated from measuring the fluorescence of serial two-fold dilutions of the labeled cells placed in the lower wells of the chemotaxis unit prior to affixing the membrane.

Compounds of the invention are expected to inhibit chemotactic response of T cells.

## Claims

1. A compound represented by formula (I): 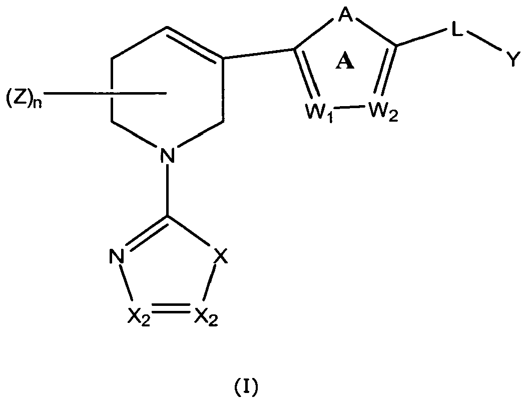 or a pharmaceutically acceptable salt, solvate, or clathrate thereof; wherein:
A is -CR,=CR₂-, -N=CR₁-, or -CR₁=N-;
W₁ and W₂ are each, independently, CR₁ or N;
X is -S-, -O-, or -NR_{c}-;
each of X₂ are independently selected from N or CR_{d};
Y is an optionally substituted aryl, an optionally substituted heteroaryl, an optionally substituted alkyl, an optionally substituted cycloalkyl, or an optionally substituted alkenyl;
L is -NRCH₂-, -CH₂NR-, -NR-C(O)-, or -C(O)-NR-;
Z is alkyl, halo, or -C(O)R₅;
R, for each occurrence, is independently -H, alkyl, -C(O)-R₁₃, or -C(O)OR₁₃;
R_{c} is -H or C₁-C₄ alkyl;
R_{d} for each occurrence is independently -H or C₁-C₄ alkyl;
R₁ and R₂, for each occurrence, are independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, - OR₅, -SR₅, -NR₆R₇, -C(O)NR₆R₇, -NR₅C(O)R₅, -C(O)R₅, -C(O)OR₅, -OC(O)R₅, -C(O)SR₅, - SC(O)R₅, -C(S)NR₆R₇, -NR₅C(S)R₅, -C(S)R₅, -C(S)OR₅, -OC(S)R₅, -C(S)SR₅, -SC(S)R₅, - C(NR₈)NR₆R₇, -NR₅C(NR₈)R₅, -C(NR₈)R₅, -C(NR₈)OR₅, -OCCNR₈)R₅ -C(NR₈)SR₅, - SC(NR₈)R₅, -OC(O)OR₅, -OC(O)NR₆R₇, -NR₅C(O)OR₅, -NR₅C(O)NR₆R₇, -SC(O)OR₅, - SC(O)NR₆R₇, -SC(O)SR₅, -NR₅C(O)SR₅, -OC(O)SR₅, -OC(S)OR₅, -OC(S)NR₆R₇, - NR₅C(S)OR₅, -NR₅C(S)NR₆R₇, -SC(S)OR₅, -SC(S)NR₆R₇, -SC(S)SR₅, -NR₅C(S)SR₅, - OC(S)SR₅, -OC(NR₈)OR₅, -OC(NR₈)NR₆R₇, -NR₅C(NR₈)OR₅, -NR₅C(NR₈)NR₆R₇, - SC(NR₈)OR₅, -SC(NR₈)NR₆R₇, -SC(NR₈)SR₅, -NR₅C(NR₈)SR₅, -OC(NR₈)SR₅, -S(O)ₚR₅, - S(O)ₚNR₆R₇, -NR₅S(O)ₚR₅, -NR₅S(O)NR₆R₇, -S(O)ₚOR₅, -OS(O)ₚR₅, or -OS(O)OR₅;
R₅, for each occurrence, is independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
R₆ and R₇, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₆ and R₇ taken together with the nitrogen to which they are attached are an optionally substituted heterocyclyl or optionally substituted heteroaryl;
R₈, for each occurrence, is independently -H, a halo, an alkyl, -OR₅, -NR₆R₇, -C(O)R₅, -C(O)OR₅, or -C(O)NR₆R₇;
R₁₁ is H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, -OR₅, -SR₅, -NR₆R₇, -C(O)NR₆R₇, -C(O)R₅, -C(O)OR₅, -C(O)SR₅, -C(S)NR₆R₇, -C(S)R₅, -C(S)OR₅, -C(S)SR₅, -C(NR₈)NR₆R₇, -C(NR₈)R₅, -C(NR₈)OR₅, or - C(NR₈)SR₅;
R₁₃, for each occurrence, is independently -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 1;
p is 1 or 2;
wherein the term "optionally substituted" refers to optional substitution with one or more substituents selected from the group consisting of alkyl, alkoxy, alkylsulfanyl, alkylamino, dialkylamino, alkenyl, alkynyl, cycloalkyl, cycloalkenyl, heterocyclyl, aryl, heteroaryl, aralkyl, heteraralkyl, haloalkyl, -C(O)NR₁₃R₁₄, -NR₁₅C(O)R₁₆, halo, -OR₁₅, cyano, nitro, haloalkoxy, -C(O)R₁₅, -NR₁₃R₁₄, -SR₁₅ -C(O)OR₁₅, -OC(O)R₁₅, - NR₁₅C(O)NR₁₃R₁₄, -OC(O)NR₁₃R₁₄, -NR₁₅C(O)OR₁₆, -S(O)pR₁₅, or -S(O)_{P}NR₁₃R₁₄; wherein R₁₃ and R₁₄, for each occurrence are, independently, H, alkyl, alkenyl, alkynyl, cycloalkyl, cycloalkenyl, heterocyclyl, aryl, heteroaryl, aralkyl, or heteraralkyl; or R₁₃ and R₁₄ taken together with the nitrogen to which they are attached is heterocyclyl or heteroaryl; and R₁₅ and R₁₆ for each occurrence are, independently, H, alkyl, alkenyl, alkynyl, cycloalkyl, cycloalkenyl, heterocyclyl, aryl, heteroaryl, aralkyl, or heteraralkyl; and
with the proviso that the compound is not:
2,6-Difluoro-N-[4-(1-thiazol-2-yl-4-methyl-1,2,5,6-tetrahydro-pyridin-3-yl)-phenyl]-benzamide;
N-[4-(1-thiazol-2-yl-4-methyl-1,2,5,6-tetrahydro-pyridin-3-yl)-phenyl]-3-fluoro-isonicotinamide;
N-[4-(1-thiazol-2-yl-4-methyl-1,2,5,6-tetrahydro-pyridin-3-yl)-phenyl]-3-methyl-isonicotinamide;
N-[4-(1-thiazol-2-yl-4-methyl-1,2,5,6-tetrahydro-pyridin-3-yl)-phenyl]-3-fluoro-isonicotinamide;
2,6-Difluoro-N-[4-(1-oxazol-2-yl-4-methyl-1,2,5,6-tetrahydro-pyridin-3-yl)-phenyl]-benzamide;
N-[4-(1-oxazol-2-yl-4-methyl-1,2,5,6-tetrahydro-pyridin-3-yl)-phenyl]-3-fluoro-isonicotinamide;
2,6-Difluoro-N-[5-(1-thiazol-2-yl-4-methyl-1,2,5,6-tetrahydro-pyridin-3-yl)-pyridin-2-yl]-benzamide, hydrochloride;
N-[4-(1-thiazol-2-yl-4-methyl-1,2,5,6-tetrahydro-pyridin-3-yl)-phenyl]-3-fluoro-isonicotinamide, dihydrochloride;
2,6-Difluoro-N-[4-(1-thiazol-2-yl-4-methyl-1,2,5,6-tetrahydro-pyridin-3-yl)-phenyl]-benzamide, hydrochloride;
2,6-Difluoro-N-[4-(1-thiazol-2-yl-4-methyl-1,2,5,6-tetrahydro-pyridin-3-yl)-phenyl]-benzamide;
5-Methyl-pyrimidine-4-carboxylic acid [4-(1-thiazol-2-yl-4-methyl-1,2,5,6-tetrahydro-pyridin-3-yl)-phenyl]-amide;
2,6-Difluoro-N-[4-(1-thiazol-2-yl-4-formyl-1,2,5,6-tetrahydro-pyridin-3-yl)-phenyl]-benzamide;
2,6-Difluoro-N-[4-(1-oxazol-2-yl-4-methyl-1,2,5,6-tetrahydro-pyridin-3-yl)-phenyl]-benzamide, hydrochloride;
2,6-Difluoro-N-{4-[1-(4-methyl-oxazol-2-yl)-4-methyl-1,2,5,6-tetrahydro-pyridin-3-yl]-phenyl}-benzamide;
5-Methyl-pyrimidine-4-carboxylic acid {4-[1-(4,5-dimethyl-oxazol-2-yl)-4-methyl-1,2,5,6-tetrahydro-pyridin-3-yl]-phenyl}-amide;
2,6-Difluoro-N-{4-[1-(4,5-dimethyl-oxazol-2-yl)-4-methyl-1,2,5,6-tetrahydro-pyridin-3-yl]-phenyl}-benzamide;
N-(4-[1-(1H-tetrazol-5-yl)-4-methyl-1,2,5,6-tetrahydro-pyridin-3-yl]-phenyl)-3-fluoro-isonicotinamide;
N-{4-[1-(1-methyl-1H-tetrazol-5-yl)-4-methyl-1,2,5,6-tetrahydro-pyridin-3-yl]-phenyl}-3-fluoro-isonicotinamide;
N-{4-[1-(2-methyl-2H-tetrazol-5-yl)-4-methyl-1,2,5,6-tetrahydro-pyridin-3-yl]-phenyl}-3-fluoro-isonicotinamide;
2,6-Difluoro-N-{4-[1-(2-methyl-2H-tetrazol-5-yl)-4-methyl-1,2,5,6-tetrahydro-pyridin-3-yl]-phenyl}-benzamide;
2,6-Difluoro-N-{4-[1-(1H-tetrazol-5-yl)-4-methyl-1,2,5,6-tetrahydro-pyridin-3-yl]-phenyl}-benzamide;
2,6-Difluoro-N-{4-[1-(1-methyl-1H-tetrazol-5-yl)-4-methyl-1,2,5,6-tetrahydro-pyridin-3-yl]-phenyl}-benzamide;
2,6-Difluoro-N-[5-(1-thiazol-2-yl-4-methyl-1,2,5,6-tetrahydro-pyridin-3-yl)-pyrimidin-2-yl]-benzamide; or
2,6-Difluoro-N-[5-(1-thiazol-2-yl-4-methyl-1,2,5,6-tetrahydro-pyridin-3-yl)-pyrimidin-2-yl]-benzamide, hydrochloride.

2. The compound of Claim 1, wherein L is -NR-C(O)- or -C(O)-NR-.

3. The compound of Claim 2, wherein L is -NH-C(O)- or -C(O)-NH-.

4. The compound of Claim 1, wherein Y is an optionally substituted phenyl, an optionally substituted oxazolyl, an optionally substituted furanyl, an optionally substitute pyrazolyl, an optionally substituted pyrimidinyl, an optionally substituted pyridinyl, an optionally substituted pyridazinyl, an optionally substituted thiadiazolyl, or an optionally substituted thiophenyl.

5. The compound of Claim 4, wherein Y is an optionally substituted phenyl, an optionally substituted pyrimidinyl, or an optionally substituted pyridinyl.

6. The compound of Claim 5, wherein Y is substituted with one to two substituents.

7. The compound of Claim 6, wherein the one to two substituents are each independently a C₁-C₄-alkyl or a halo.

8. The compound of Claim 7, wherein Y is a difluorophenyl.

9. The compound of Claim 1, wherein Z is methyl.

10. The compound of Claim 1, wherein the compound is represented by formula (II): 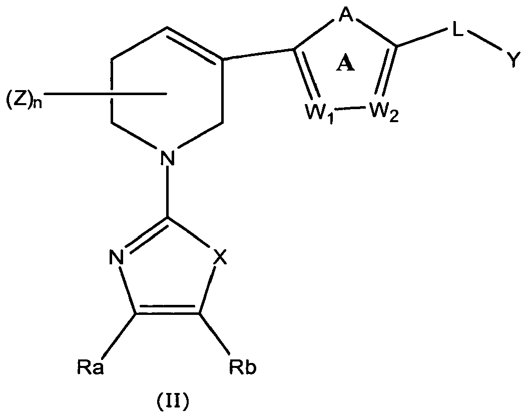 wherein Rₐ and R_{b} are each, independently, -H or C₁-C₄ alkyl.

11. The compound of Claim 10, wherein the compound is represented by formula (III):

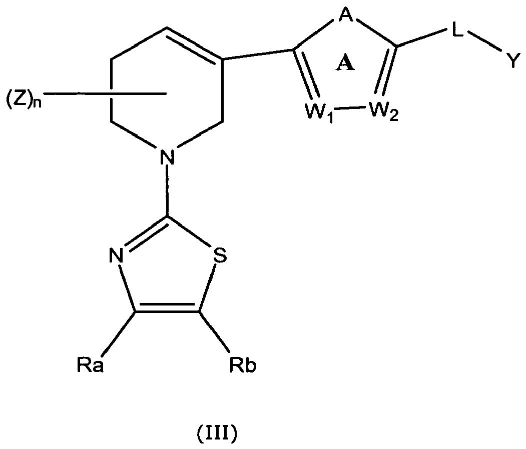

12. The compound of any one of Claims 10 or 11, wherein:
L is -NHCH₂-, -CH₂NH-, -NH-C(O)-, or -C(O)-NH-; and
Y is an optionally substituted phenyl, an optionally substitute pyrimidinyl, or an optionally substituted pyridinyl.

13. The compound of Claim 11, wherein the compound is a compound of formula (IV): 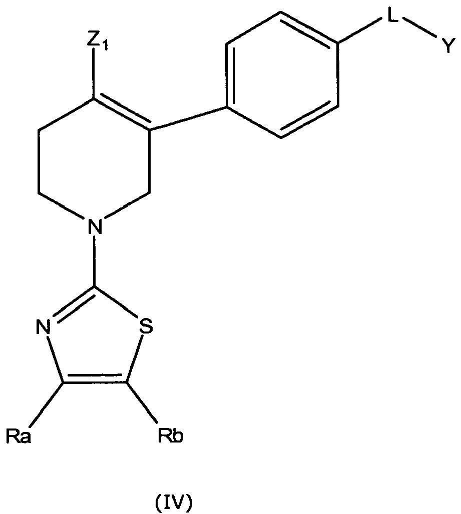 wherein Z₁ is alkyl, halo, or -C(O)R₅.

14. The compound of Claim 13, wherein:
L is-NR-C(O)- or -C(O)-NR.

15. The compound of Claim 14, wherein the compound is a compound of formula (V): 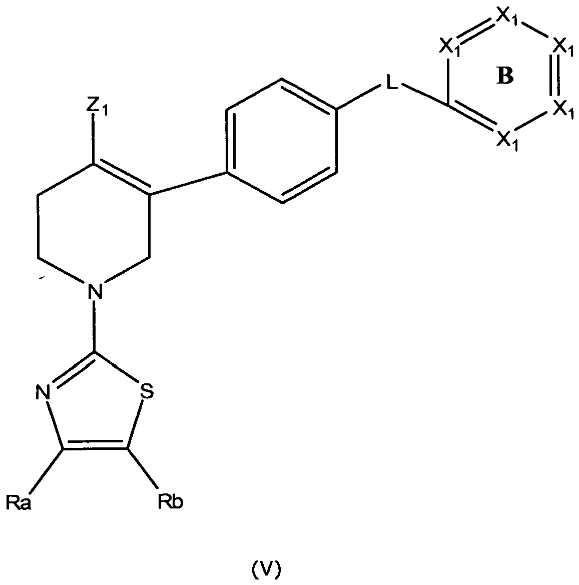 wherein each X₁ is independently N or CR, and at least two X₁ groups are CR₁.

16. The compound of Claim 15, wherein:
L is-NR-C(O)- or -C(O)-NR-.

17. The compound of Claim 1, wherein the compound is selected from

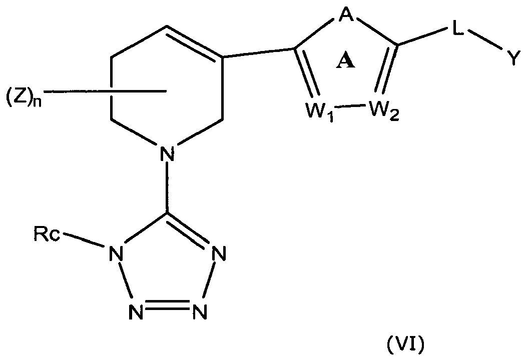

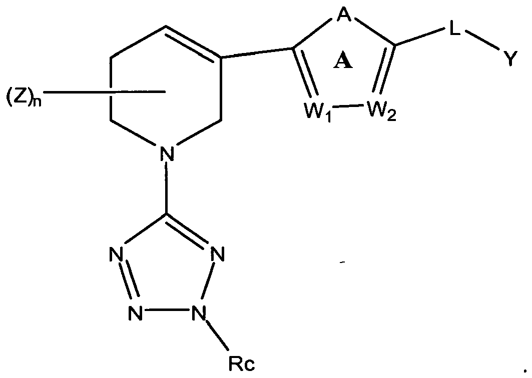

18. A compound of formula (VII): 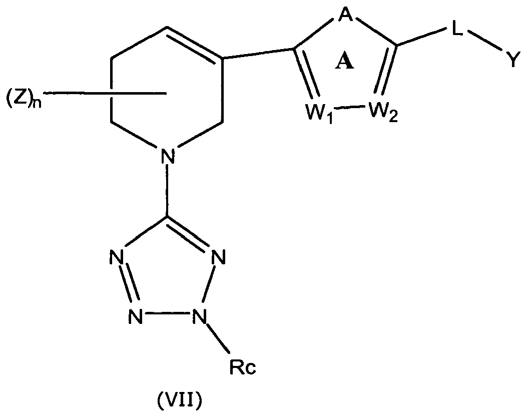 or a pharmaceutically acceptable salt, solvate, or clathrate thereof; wherein:
A is -CR₁=CR₂-, -N=CR₁-, or -CR₁=N-;
W₁ and W₂ are each, independently, CR, or N;
Y is an optionally substituted aryl, an optionally substituted heteroaryl, an optionally substituted alkyl, an optionally substituted cycloalkyl, or an optionally substituted alkenyl;
L is -NRCH₂-, -CH₂NR-, -NR-C(O)-, or -C(O)-NR-;
Z is alkyl, halo, or -C(O)R₅;
R, for each occurrence, is independently -H, alkyl, -C(O)-R₁₃, or -C(O)OR₁₃;
R_{c} is -H or C₁-C₄ alkyl;
R₁ and R₂, for each occurrence, are independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, cyano, nitro, halo, - OR₅, -SR₅, -NR₆R₇, -C(O)NR₆R₇, -NR₅C(O)R₅, -C(O)R₅, -C(O)OR₅, -OC(O)R₅, -C(O)SR₅, - SC(O)R₅, -C(S)NR₆R₇, -NR₅C(S)R₅, -C(S)R₅, -C(S)OR₅, -OC(S)R₅, -C(S)SR₅, -SC(S)R₅, - C(NR₈)NR₆R₇, -NR₅C(NR₈)R₅, -C(NR₈)R₅, -C(NR₈)OR₅, -OC(NR₈)R₅, -C(NR₈)SR₅, - SC(NR₈)R₅, -OC(O)OR₅, -OC(O)NR₆R₇, -NR₅C(O)OR₅, -NR₅C(O)NR₆R₇, -SC(O)OR₅, - SC(O)NR₆R₇, -SC(O)SR₅, -NR₅C(O)SR₅, -OC(O)SR₅, -OC(S)OR₅, -OC(S)NR₆R₇, - NR₅C(S)OR₅, -NR₅C(S)NR₆R₇, -SC(S)OR₅, -SC(S)NR₆R₇, -SC(S)SR₅, -NR₅C(S)SR₅, - OC(S)SR₅, -OC(NR₈)OR₅, -OC(NR₈)NR₆R₇, -NR₅C(NR₈)OR₅, -NR₅C(NR₈)NR₆R₇, - SC(NR₈)OR₅, -SC(NR₈)NR₆R₇, -SC(NR₈)SR₅, -NR₅C(NR₈)SR₅, -OC(NR₈)SR₅, -S(O)ₚR₅, - S(O)ₚNR₆R₇, -NR₅S(O)pR₅, -NR₅S(O)NR₆R₇, -S(O)pOR₅, -OS(O)ₚR₅, or -OS(O)OR₅;
R₅, for each occurrence, is independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
R₆ and R₇, for each occurrence are, independently, H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl; or R₆ and R₇ taken together with the nitrogen to which they are attached are an optionally substituted heterocyclyl or optionally substituted heteroaryl;
R₈, for each occurrence, is independently -H, a halo, an alkyl, -OR₅, -NR₆R₇, -C(O)R₅, -C(O)OR₅, or -C(O)NR₆R₇;
R₁₁ is H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, an optionally substituted heteraralkyl, -OR₅, -SR₅, -NR₆R₇, -C(O)NR₆R₇, -C(O)R₅, -C(O)OR₅, -C(O)SR₅, -C(S)NR₆R₇, -C(S)R₅, -C(S)OR₅, -C(S)SR₅, -C(NR₈)NR₆R₇, -C(NR₈)R₅, -C(NR₈)OR₅, or - C(NR₈)SR₅;
R₁₃, for each occurrence, is independently -H, an optionally substituted alkyl, an optionally substituted alkenyl, an optionally substituted alkynyl, an optionally substituted cycloalkyl, an optionally substituted cycloalkenyl, an optionally substituted heterocyclyl, an optionally substituted aryl, an optionally substituted heteroaryl, an optionally substituted aralkyl, or an optionally substituted heteraralkyl;
n is 1;
p is 1 or 2;
wherein the term "optionally substituted" refers to optional substitution with one or more substituents selected from the group consisting of alkyl, alkoxy, alkylsulfanyl, alkylamino, dialkylamino, alkenyl, alkynyl, cycloalkyl, cycloalkenyl, heterocyclyl, aryl, heteroaryl, aralkyl, heteraralkyl, haloalkyl, -C(O)NR₁₃R₁₄, -NR₁₅C(O)R₁₆, halo, -OR₁₅, cyano, nitro, haloalkoxy, -C(O)R₁₅, -NR₁₃R₁₄, -SR₁₅, -C(O)OR₁₅, -OC(O)R₁₅, - NR₁₅C(O)NR₁₃R₁₄, -OC(O)NR₁₃R₁₄, -NR₁₅C(O)OR₁₆, -S(O)pR₁₅, or -S(O)ₚNR₁₃R₁₄; wherein R₁₃ and R₁₄, for each occurrence are, independently, H, alkyl, alkenyl, alkynyl, cycloalkyl, cycloalkenyl, heterocyclyl, aryl, heteroaryl, aralkyl, or heteraralkyl; or R₁₃ and R₁₄ taken together with the nitrogen to which they are attached is heterocyclyl or heteroaryl; and R₁₅ and R₁₆ for each occurrence are, independently, H, alkyl, alkenyl, alkynyl, cycloalkyl, cycloalkenyl, heterocyclyl, aryl, heteroaryl, aralkyl, or heteraralkyl; and
with the proviso that the compound is not:
N-{4-[1-(2-methyl-2H-tetrazol-5-yl)-4-methyl-1,2,5,6-tetrahydro-pyridin-3-yl]-phenyl}-3-fluoro-isonicotinamide or
2,6-Difluoro-N-{4-[1-(2-methyl-2H-tetrazol-5-yl)-4-methyl-1,2,5,6-tetrahydro-pyridin-3-yl]-phenyl}-benzamide.

19. A pharmaceutical composition, comprising a pharmaceutically acceptable carrier and a compound of any one of Claims 1 through 18.

20. The pharmaceutical composition of Claim 19, further comprising one or more additional therapeutic agents.

21. The pharmaceutical composition according to Claim 20, wherein the additional therapeutic agent is selected from the group consisting of immunosuppressive agents, anti-inflammatory agents and suitable mixtures thereof; or the additional therapeutic agent is selected from the group consisting of steroids, non-steroidal anti-inflammatory agents, antihistamines, analgesics, and suitable mixtures thereof.

22. Use of a compound of any one of Claims 1 through 18 for the manufacture of a medicament for treating or preventing an immune disorder, or treating or preventing an inflammatory condition, or suppressing the immune system, or treating or preventing an allergic disorder in a subject in need thereof.

23. The use of Claim 22, wherein the disorder is selected from the group consisting of multiple sclerosis, myasthenia gravis, Guillain-Barré, autoimmune uveitis, autoimmune hemolytic anemia, pernicious anemia, autoimmune thrombocytopenia, temporal arteritis, anti-phospholipid syndrome, vasculitides such as Wegener's granulomatosis, Behcet's disease, psoriasis, dermatitis herpetiformis, pemphigus vulgaris, vitiligo, Crohn's disease, ulcerative colitis, primary biliary cirrhosis, autoimmune hepatitis, Type 1 or immune-mediated diabetes mellitus, Grave's disease, Hashimoto's thyroiditis, autoimmune oophoritis and orchitis, autoimmune disorder of the adrenal gland, rheumatoid arthritis, systemic lupus erythematosus, scleroderma, polymyositis, dermatomyositis, ankylosing spondylitis, Sjogren's syndrome, transplant rejection, skin graft rejection, arthritis, rheumatoid arthritis, osteoarthritis and bone diseases associated with increased bone resorption; inflammatory bowel disease, ileitis, ulcerative colitis, Barrett's syndrome, Crohn's disease; asthma, adult respiratory distress syndrome, chronic obstructive airway disease; corneal dystrophy, trachoma, onchocerciasis, uveitis, sympathetic ophthalmitis, endophthalmitis; gingivitis, periodontitis; tuberculosis; leprosy; uremic complications, glomerulonephritis, nephrosis; sclerodermatitis, psoriasis, eczema; chronic demyelinating diseases of the nervous system, multiple sclerosis, AIDS-related neurodegeneration, Alzheimer's disease, infectious meningitis, encephalomyelitis, Parkinson's disease, Huntington's disease, amyotrophic lateral sclerosis viral or autoimmune encephalitis; autoimmune disorders, immune-complex vasculitis, systemic lupus and erythematodes; systemic lupus erythematosus (SLE); cardiomyopathy, ischemic heart disease hypercholesterolemia, atherosclerosis, preeclampsia; chronic liver failure, brain and spinal cord trauma, cancer, allergic rhinitis, sinusitis, rhinosinusitis, chronic otitis media, recurrent otitis media, drug reactions, insect sting reactions, latex reactions, conjunctivitis, urticaria, anaphylaxis reactions, anaphylactoid reactions, atopic dermatitis, asthma, or food allergies.

## Patentansprüche

1. Eine Verbindung, die durch Formel (I) repräsentiert wird: 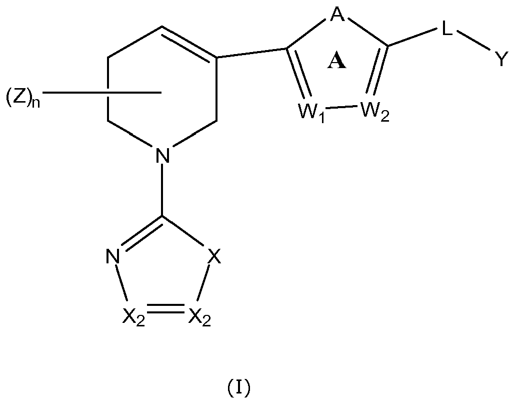 oder ein pharmazeutisch geeignetes Salz, Solvat oder Clathrat, wobei:
A -CR₁=CR₂-, -N=CR₁-, oder -CR₁ =N- ist;
W₁ und W₂ jeweils unabhängig CR₁ oder N sind;
X -S-, -O- oder -NR_{c} ist;
jedes X₂ unabhängig aus N oder CR_{d} ausgewählt wird;
Y ein optional substituiertes Aryl, ein optional substituiertes Heteroaryl, ein optional substituiertes Alkyl, ein optional substituiertes Cycloalkyl oder ein optional substituiertes Alkenyl ist;
L -NRCH₂-, -CH₂NR-, -NR-C(O)- oder -C(O)-NR- ist;
Z Alkyl, Halo oder -C(O)R₅ ist;
R, bei jedem Auftreten, unabhängig -H, Alkyl, -C(O)-R₁₃ oder -C(O)OR₁₃ ist;
R_{c} -H oder C₁-C₄ Alkyl ist;
R_{d} bei jedem Auftreten unabhängig -H oder C₁-C₄ Alkyl ist;
R₁ und R₂, bei jedem Auftreten, unabhängig H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteraralkyl, Cyano, Nitro, Halo, -OR₅, -SR₅, -NR₆R₇, -C(O)NR₆R₇, -NR₅C(O)R₅, -C(O)R₅, -C(O)OR₅, - OC(O)R₅, -C(O)SR₅, -SC(O)R₅, -C(S)NR₆R₇, -NR₅C(S)R₅, -C(S)R₅, -C(S)OR₅, -OC(S)R₅, - C(S)SR_{S}, -SC(S)R₅, -C(NR₈)NR₆R₇, -NR₅C(NR₈)R₅, -C(NR₈)R₅, -C(NR₈)OR₅, -OC(NR₈)R₅, - C(NR₈)SR₅, -SC(NR₈)R₅, -OC(O)OR₅, -OC(O)NR₆R₇, -NR₅C(O)OR₅, -NR₅C(O)NR₆R₇, - SC(O)OR₅, -SC(O)NR₆R₇, -SC(O)SR₅, -NR₅C(O)SR₅, -OC(O)SR₅, -OC(S)OR₅, - OC(S)NR₆R₇, -NR₅C(S)OR₅, -NR₅C(S)NR₆R₇, -SC(S)OR₅, -SC(S)NR₆R₇, -SC(S)SR₅, - NR₅C(S)SR₅, -OC(S)SR₅, -OC(NR₈)OR₅, -OC(NR₈)NR₆R₇, -NR₅C(NR₈)OR₅, - NR₅C(NR₈)NR₆R₇, -SC(NR₈)OR₅, -SC(NR₈)NR₆R₇, -SC(NR₈)SR₅, -NR₅C(NR₈)SR₅, - OC(NR₈)SR₅, -S(O)ₚR₅, -S(O)ₚNR₆R₇, -NR₅S(O)ₚR₅, -NR₅S(O)NR₆R₇, -S(O)ₚOR₅, - OS(O)ₚR₅ oder -OS(O)OR₅ ist;
R₅, bei jedem Auftreten, unabhängig H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteraralkyl ist;
R₆ und R₇, bei jedem Auftreten, unabhängig H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteraralkyl sind; oder R₆ und R₇ zusammen mit dem Sauerstoff, an das sie gebunden sind, ein optional substituiertes Heterocyclyl oder optional substituiertes Heteroaryl sind;
R₈, bei jedem Auftreten, unabhängig -H, ein Halogen, ein Alkyl, -OR₅, -NR₆R₇, -C(O)R₅, -C(O)OR₅ oder -C(O)NR₆R₇ ist;
R₁₁ H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteraralkyl, -OR₅, -SR₅, -NR₆R₇, -C(O)NR₆R₇, - C(O)R₅, -C(O)OR₅, -C(O)SR₅, -C(S)NR₆R₇, -C(S)R₅, -C(S)OR₅, -C(S)SR₅, -C(NR₈)NR₆R₇, - C(NR₈)R₅, -C(NR₈)OR₅ oder -C(NR₈)SR₅ ist;
R₁₃, bei jedem Auftreten, unabhängig -H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteraralkyl ist;
n 1 ist;
p 1 oder 2 ist;
wobei der Begriff "optional substituiert" sich auf eine optionale Substitution mit einem oder mehreren Substituenten bezieht, die aus der Gruppe, die aus Alkyl, Alkoxy, Alkylsulfanyl, Alkylamino, Dialkylamino, Alkenyl, Alkynyl, Cycloalkyl, Cycloalkenyl, Heterocyclyl, Aryl, Heteroaryl, Aralkyl, Heteraralkyl, Haloalkyl, -C(O)NR₁₃R₁₄, - NR₁₅C(O)R₁₆, Halogen, -OR₁₅, Cyano, Nitro, Haloalkoxy, -C(O)R₁₅, -NR₁₃R₁₄, -SR₁₅, - C(O)OR₁₅, -OC(O)R₁₅, -NR₁₅C(O)NR₁₃R₁₄, -OC(O)NR₁₃R₁₄, -NR₁₅C(O)OR₁₆, -S(O)ₚR₁₅ oder -S(O)ₚNR₁₃R₁₄ besteht, ausgewählt werden; wobei R₁₃ und R₁₄, bei jedem Auftreten, unabhängig H, Alkyl, Alkenyl, Alkynyl, Cycloalkyl, Cycloalkenyl, Heterocyclyl, Aryl, Heteroaryl, Aralkyl oder Heteraralkyl sind; oder R₁₃ und R₁₄ zusammen mit dem Stickstoff, an das sie gebunden sind, Heterocyclyl oder Heteroaryl sind; und R₁₅ und R₁₆ bei jedem Auftreten unabhängig H, Alkyl, Alkenyl, Alkynyl, Cycloalkyl, Cycloalkenyl, Heterocyclyl, Aryl, Heteroaryl, Aralkyl oder Heteraralkyl sind; und
mit der Maßgabe, dass die Verbindung nicht ist:
2,6-Difluoro-N-[4-(1-thiazol-2-yl-4-methyl-1,2,5,6-tetrahydro-pyridin-3-yl)-phenyl]-benzamid;
N-[4-(1-Thiazol-2-yl-4-methyl-1,2,5,6-tetrahydro-pyridin-3-yl)-phenyl]-3-fluoro-isonicotinamid;
N-[4-(1-Thiazol-2-yl-4-methyl-1,2,5,6-tetrahydro-pyridin-3-yl)-phenyl]-3-methyl-isonicotinamid;
N-[4-(1-Thiazol-2-yl-4-methyl-1,2,5,6-tetrahydro-pyridin-3-yl)-phenyl]-3-fluoro-isonicotinamid;
2,6-Difluoro-N-[4-(1-oxazol-2-yl-4-methyl-1,2,5,6-tetrahydro-pyridin-3-yl)-phenyl]-benzamid;
N-[4-(1-Oxazol-2-yl-4-methyl-1,2,5,6-tetrahydro-pyridin-3-yl)-phenyl]-3-fluoro-isonicotinamid;
2,6-Difluoro-N-[5-(1-thiazol-2-yl-4-methyl-1,2,5,6-tetrahydro-pyridin-3-yl)-pyridin-2-yl]-benzamid, Hydrochlorid;
N-[4-(1-Thiazol-2-yl-4-methyl-1,2,5,6-tetrahydro-pyridin-3-yl)-phenyl]-3-fluoro-isonicotinamid, dihydrochlorid;
2,6-Difluoro-N-[4-(1-thiazol-2-yl-4-methyl-1,2,5,6-tetrahydro-pyridin-3-yl)-phenyl]-benzamid, Hydrochlorid;
2,6-Difluoro-N-[4-(1-thiazol-2-yl-4-methyl-1,2,5,6-tetrahydro-pyridin-3-yl)-phenyl]-benzamid;
5-Methyl-pyrimidin-4-carboxylic acid [4-(1-thiazol-2-yl-4-methyl-1,2,5,6-tetrahydro-pyridin-3-yl)-phenyl]-amid;
2,6-Difluoro-N-[4-(1-thiazol-2-yl-4-formyl-1,2,5,6-tetrahydro-pyridin-3-yl)-phenyl]-benzamid;
2,6-Difluoro-N-[4-(1-oxazol-2-yl-4-methyl-1,2,5,6-tetrahydro-pyridin-3-yl)-phenyl]-benzamid, hydrochlorid;
2,6-Difluoro-N-{4-[1-(4-methyl-oxazol-2-yl)-4-methyl-1,2,5,6-tetrahydro-pyridin-3-yl]-phenyl}-benzamid;
5-Methyl-pyrimidin-4-carboxylic acid {4-[1-(4,5-dimethyl-oxazol-2-yl)-4-methyl-1,2,5,6-tetrahydro-pyridin-3-yl]-phenyl}-amid;
2,6-Difluoro-N-{4-[1-(4,5-dimethyl-oxazol-2-yl)-4-methyl-1,2,5,6-tetrahydro-pyridin-3-yl]-phenyl}-benzamid;
N-{4-[1-(1H-Tetrazol-5-yl)-4-methyl-1,2,5,6-tetrahydro-pyridin-3-yl]-phenyl}-3-fluoro-isonicotinamid;
N-{4-[1-(1-Methyl-1H-tetrazol-5-yl)-4-methyl-1,2,5,6-tetrahydro-pyridin-3-yl]-phenyl}-3-fluoro-isonicotinamid;
N-{4-[1-(2-Methyl-2H-tetrazol-5-yl)-4-methyl-1,2,5,6-tetrahydro-pyridin-3-yl]-phenyl}-3-fluoro-isonicotinamid;
2,6-Difluoro-N-{4-[1-(2-methyl-2H-tetrazol-5-yl)-4-methyl-1,2,5,6-tetrahydro-pyridin-3-yl]-phenyl}-benzamid;
2,6-Difluoro-N-{4-[1-(1H-tetrazol-5-yl)-4-methyl-1,2,5,6-tetrahydro-pyridin-3-yl]-phenyl}-benzamid;
2,6-Difluoro-N-{4-[1-(1-methyl-1H-tetrazol-5-yl)-4-methyl-1,2,5,6-tetrahydro-pyridin-3-yl]-phenyl}-benzamid;
2,6-Difluoro-N-[5-(1-thiazol-2-yl-4-methyl-1,2,5,6-tetrahydro-pyridin-3-yl)-pyrimidin-2-yl]-benzamid; oder
2,6-Difluoro-N-[5-(1-thiazol-2-yl-4-methyl-1,2,5,6-tetrahydro-pyridin-3-yl)-pyrimidin-2-yl]-benzamid, hydrochlorid.

2. Die Verbindung nach Anspruch 1, wobei L -NR-C(O)- oder -C(O)-NR- ist.

3. Die Verbindung nach Anspruch 2, wobei L -NH-C(O)- oder -C(O)-NH- ist.

4. Die Verbindung nach Anspruch 1, wobei Y ein optional substituiertes Phenyl, ein optional substituiertes Oxazolyl, ein optional substituiertes Furanyl, an optional substituiertes Pyrazolyl, ein optional substituiertes Pyrimidinyl, ein optional substituiertes Pyridinyl, ein optional substituiertes Pyridazinyl, ein optional substituiertes Thiadiazolyl oder ein optional substituiertes Thiophenyl ist.

5. Die Verbindung nach Anspruch 4, wobei Y ein optional substituiertes Phenyl, ein optional substituiertes Pyrimidinyl oder ein optional substituiertes Pyridinyl ist.

6. Die Verbindung nach Anspruch 5, wobei Y mit einem oder zwei Substituenten substituiert ist.

7. Die Verbindung nach Anspruch 6, wobei der eine oder die zwei Substituenten jeweils unabhängig C₁-C₄-Alkyl oder ein Halogen sind.

8. Die Verbindung nach Anspruch 7, wobei Y ein Difluorophenyl ist.

9. Die Verbindung nach Anspruch 1, wobei Z Methyl ist.

10. Die Verbindung nach Anspruch 1, wobei die Verbindung durch Formel (II) repräsentiert wird: 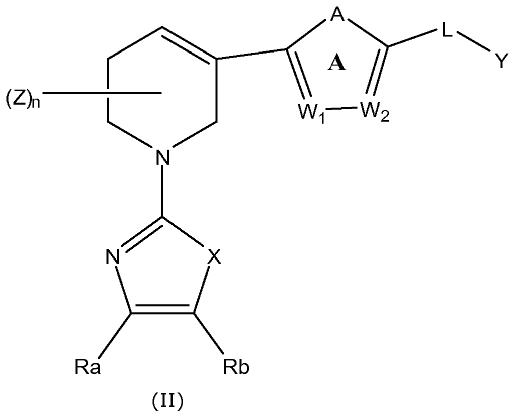 wobei Rₐ und R_{b} jeweils unabhängig -H oder C₁-C₄ Alkyl sind.

11. Die Verbindung nach Anspruch 10, wobei die Verbindung durch Formel (III) repräsentiert wird:

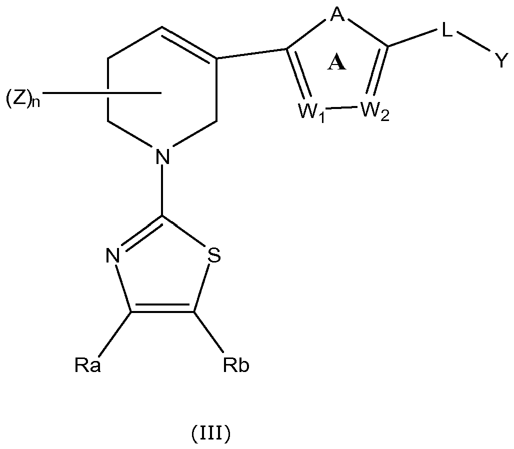

12. Die Verbindung nach einem der Ansprüche 10 oder 11, wobei:
L -NHCH₂-, -CH₂NH-, -NH-C(O)- oder -C(O)-NH- ist; und
Y ein optional substituiertes Phenyl, an optional substituiertes Pyrimidinyl oder ein optional substituiertes Pyridinyl ist.

13. Die Verbindung nach Anspruch 11, wobei die Verbindung eine Verbindung der Formel (IV) ist: 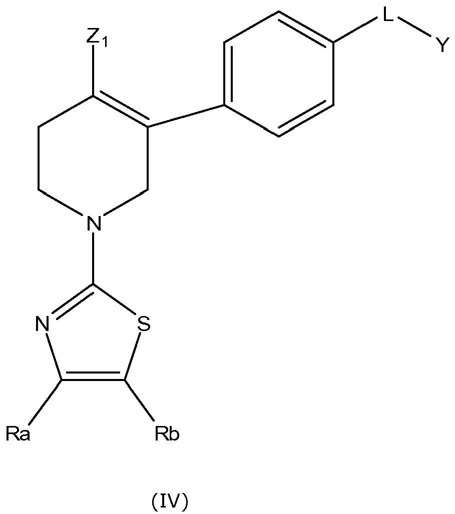 wobei Z₁ Alkyl, Halogen oder -C(O)R₅ ist.

14. Die Verbindung nach Anspruch 13, wobei:
L-NR-C(O)- oder -C(O)-NR ist.

15. Die Verbindung nach Anspruch 14, wobei die Verbindung eine Verbindung nach Formel (V) ist: 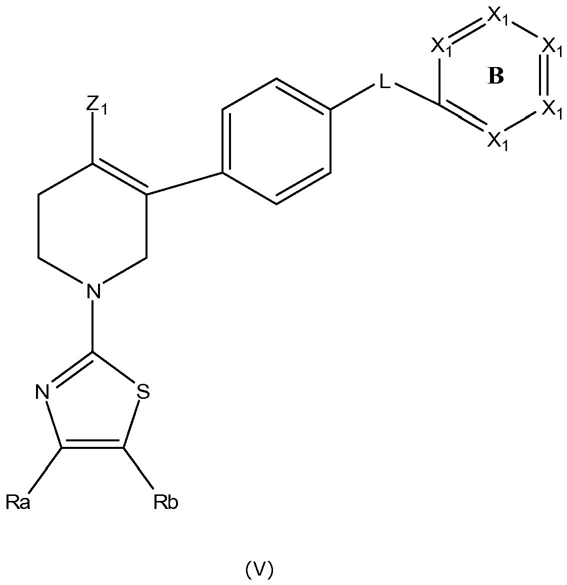 wobei jedes X₁ unabhängig N oder CR₁ ist und mindestens zwei X₁ Gruppen CR₁ sind.

16. Die Verbindung nach Anspruch 15, wobei:
L-NR-C(O)- oder -C(O)-NR- ist.

17. Die Verbindung nach Anspruch 1, wobei die Verbindung ausgewählt wird aus

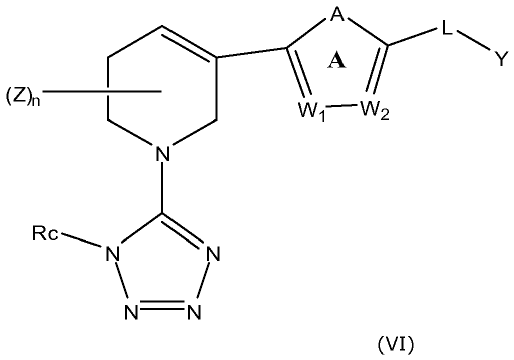

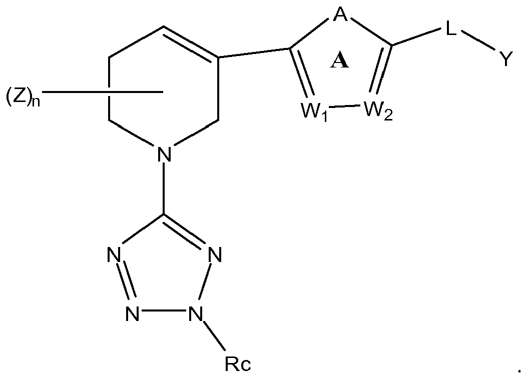

18. Eine Verbindung nach Formel (VII): 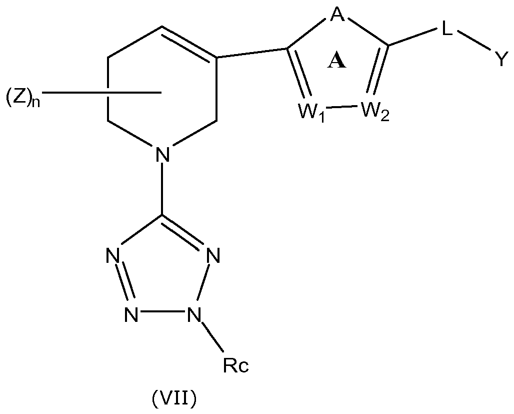 oder ein pharmazeutisch geeignetes Salz, Solvat oder Clathrat davon; wobei:
A -CR₁=CR₂-, -N=CR₁- oder -CR₁=N- ist;
W₁ und W₂ jeweils unabhängig CR₁ oder N sind;
Y ein optional substituiertes Aryl, ein optional substituiertes Heteroaryl, ein optional substituiertes Alkyl, ein optional substituiertes Cycloalkyl oder ein optional substituiertes Alkenyl ist;
L -NRCH₂-, -CH₂NR-, -NR-C(O)-, oder -C(O)-NR- ist;
Z Alkyl, Halogen oder -C(O)R₅ ist;
R, bei jedem Auftreten, unabhängig -H, Alkyl, -C(O)-R₁₃ oder -C(O)OR₁₃ ist;
R_{c} -H oder C₁-C₄ Alkyl ist;
R₁ und R₂, bei jedem Auftreten, unabhängig H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteraralkyl, Cyano, Nitro, Halogen, -OR₅, -SR₅, -NR₆R₇, -C(O)NR₆R₇, -NR₅C(O)R₅, -C(O)R₅, - C(O)OR₅, -OC(O)R₅, -C(O)SR₅, -SC(O)R₅, -C(S)NR₆R₇, -NR₅C(S)R₅, -C(S)R₅, -C(S)OR₅, - OC(S)R₅, -C(S)SR₅, -SC(S)R₅, -C(NR₈)NR₆R₇, -NR₅C(NR₈)R₅, -C(NR₈)R₅, -C(NR₈)OR₅, - OC(NR₈)R₅, -C(NR₈)SR₅, -SC(NR₈)R₅, -OC(O)OR₅, -OC(O)NR₆R₇, -NR_{S}C(O)OR₅, - NR₅C(O)NR₆R₇, -SC(O)OR₅, -SC(O)NR₆R₇, -SC(O)SR₅, -NR₅C(O)SR₅, -OC(O)SR₅, - OC(S)OR₅, -OC(S)NR₆R₇, -NR₅C(S)OR₅, -NR₅C(S)NR₆R₇, -SC(S)OR₅, -SC(S)NR₆R₇, - SC(S)SR₅, -NR_{S}C(S)SR₅, -OC(S)SR₅, -OC(NR₈)OR₅, -OC(NR₈)NR₆R₇, -NR₅C(NR₈)OR₅, - NR₅C(NR₈)NR₆R₇, -SC(NR₈)OR₅, -SC(NR₈)NR₆R₇, -SC(NR₈)SR₅, -NR₅C(NR₈)SR₅, - OC(NR₈)SR₅, -S(O)ₚR₅, -S(O)ₚNR₆R₇, -NR₅S(O)ₚR₅, -NR₅S(O)NR₆R₇, -S(O)ₚOR₅, - OS(O)ₚR₅ oder -OS(O)OR₅ sind;
R₅, bei jedem Auftreten, unabhängig H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteraralkyl ist;
R₆ und R₇, bei jedem Auftreten, unabhängig H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteraralkyl sind; oder R₆ und R₇ zusammen mit dem Stickstoff, an das sie gebunden sind, ein optional substituiertes Heterocyclyl oder optional substituiertes Heteroaryl sind;
R₈, bei jedem Auftreten, unabhängig -H, ein Halogen, ein Alkyl, -OR₅, -NR₆R₇, -C(O)R₅, -C(O)OR₅ oder -C(O)NR₆R₇ ist;
R₁₁ H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl, ein optional substituiertes Heteraralkyl, -OR₅, -SR₅, -NR₆R₇, -C(O)NR₆R₇, - C(O)R₅, -C(O)OR₅, -C(O)SR₅, -C(S)NR₆R₇, -C(S)R₅, -C(S)OR₅, -C(S)SR₅, -C(NR₈)NR₆R₇, - C(NR₅)R₅, -C(NR₈)OR₅ oder -C(NR₈)SR₅ ist;
R₁₃, bei jedem Auftreten, unabhängig -H, ein optional substituiertes Alkyl, ein optional substituiertes Alkenyl, ein optional substituiertes Alkynyl, ein optional substituiertes Cycloalkyl, ein optional substituiertes Cycloalkenyl, ein optional substituiertes Heterocyclyl, ein optional substituiertes Aryl, ein optional substituiertes Heteroaryl, ein optional substituiertes Aralkyl oder ein optional substituiertes Heteraralkyl ist;
n 1 ist;
p 1 oder 2 ist;
wobei der Begriff "optional substituiert" sich auf eine optionale Substitution mit einem oder mehreren Substituenten bezieht, die aus der Gruppe, die aus Alkyl, Alkoxy, Alkylsulfanyl, Alkylamino, Dialkylamino, Alkenyl, Alkynyl, Cycloalkyl, Cycloalkenyl, Heterocyclyl, Aryl, Heteroaryl, Aralkyl, Heteraralkyl, Haloalkyl, -C(O)NR₁₃R₁₄, - NR₁₅C(O)R₁₆, Halogen, -OR₁₅, Cyano, Nitro, Haloalkoxy, -C(O)R₁₅, -NR₁₃R₁₄, -SR₁₅, - C(O)OR₁₅, -OC(O)R₁₅, -NR₁₅C(O)NR₁₃R₁₄, -OC(O)NR₁₃R₁₄, -NR₁₅C(O)OR₁₆, -S(O)ₚR₁₅ oder -S(O)ₚNR₁₃R₁₄ besteht, ausgewählt wird; wobei R₁₃ und R₁₄, bei jedem Auftreten, unabhängig H, Alkyl, Alkenyl, Alkynyl, Cycloalkyl, Cycloalkenyl, Heterocyclyl, Aryl, Heteroaryl, Aralkyl oder Heteraralkyl sind; oder R₁₃ und R₁₄ zusammen mit dem Stickstoff, an das sie gebunden sind, Heterocyclyl oder Heteroaryl sind; und R₁₅ und R₁₆ bei jedem Auftreten, unabhängig, H, Alkyl, Alkenyl, Alkynyl, Cycloalkyl, Cycloalkenyl, Heterocyclyl, Aryl, Heteroaryl, Aralkyl oder Heteraralkyl sind; und
mit der Maßgabe, dass die Verbindung nicht ist:
N-{4-[1-(2-Methyl-2H-tetrazol-5-yl)-4-methyl-1,2,5,6-tetrahydro-pyridin-3-yl]-phenyl}-3-fluoro-isonicotinamid oder
2,6-Difluoro-N-{4-[1-(2-methyl-2H-tetrazol-5-yl)-4-methyl-1,2,5,6-tetrahydro-pyridin-3-yl]-phenyl}-benzamid.

19. Eine pharmazeutische Zusammensetzung, die einen pharmazeutisch geeigneten Träger und eine Verbindung nach einem der Ansprüche 1 bis 18 umfasst.

20. Die pharmazeutische Zusammensetzung nach Anspruch 19, die weiter ein oder mehrere therapeutische Mittel umfasst.

21. Die pharmazeutische Zusammensetzung nach Anspruch 20, wobei das zusätzliche therapeutische Mittel aus der Gruppe, die aus Immunosuppressiva, entzündungshemmenden Mitteln und geeigneten Mischungen davon besteht, ausgewählt wird; oder wobei das zusätzliche therapeutische Mittel aus der Gruppe, die aus Steroiden, nicht-steroide entzündungshemmende Mitteln, Antihistaminika, Analgetika, und geeigneten Mischungen davon besteht, ausgewählt wird.

22. Verwendung einer Verbindung nach einem der Ansprüche 1 bis18 für die Herstellung eines Medikaments zur Behandlung oder Prävention einer Immunerkrankung oder zur Behandlung oder Prävention eines Entzündungszustands oder zur Unterdrückung des Immunsystems oder zur Behandlung oder Prävention einer allergischen Erkrankung bei einem Subjekt mit Bedarf.

23. Die Verwendung nach Anspruch 22, wobei die Erkrankung aus der Gruppe, die aus die aus Multipler Sklerose, Myasthenia gravis, Guillain-Barré, Autoimmun-Uveitis, Autoimmun-Hämolytische Anämie, Perniziöse Anämie, Autoimmun-Thrombozytopenie, Arteriitis temporalis, Anti-Phospholipid Syndrom, Vaskulitis wie zum Beispiel Wegener-Granulomatose, Morbus Behçet, Psoriasis, Dermatitis herpetiformis, Pemphigus vulgaris, Vitiligo, Crohn-Krankheit, Colitis ulcerosa, primär biliäre Zirrhose, Autoimmun-Hepatitis, Typ 1 oder immunvermittelte Diabetes mellitus, Morbus Basedow, Hashimoto-Thyroiditis, Autoimmune Oophoritis und Orchitis, Autoimmunerkrankung der Nebenniere, rheumatoide Arthritis, systemischer Lupus erythematosis, Sklerodermie, Polymyositis, Dermatomyositis, Spondylitis ankylosans, Sjögren Syndrom, Transplantatabstoßung, Hautransplantatabstoßung, Arthritis, rheumatoide Arthritis, Osteoarthritis und Knochenerkrankungen die mit erhöhter Knochenresorption, chronisch entzündliche Darmerkrankung, Ileitis, Colitis ulcerosa, Barrett-Syndrom, Crohn-Krankheit, Asthma, Atemnot-Syndrom des Erwachsenen, chronisch obstruktive Lungenerkrankung, korneale Dystrophie, Trachom, Onchozerkose, Uveitis, sympathetische Ophthalmie, Endophthalmitie, Gingivitis, Periodontitis; Tuberkulose; Lepra, urämische Komplikationen, Glomerulonephritis, Nephrose, Sklerodermatitis, Ekzem, chronische demyelinisierende Erkrankungen des Nervensystems, Multiple Sklerose, AIDS-bezogene Neurodegeneration, Alzheimer-Erkrankung, infektiöse Meningitis, Enzephalomyelitis, Parkinson-Erkrankung, Huntington-Erkrankung, virale amyotrophe Lateralsklerose oder Autoimmun-Enzephalitis, Autoimmunerkrankungen, Immun-Komplex Vaskulitis, systemischer Lupus und erythematodes, systemischer Lupus erythematosus (SLE), Kardiomyopathie, ischämische Herzerkrankung Hypercholesterinämie, Atherosklerose, Präeklampsie, chronisches Nierenversagen, Hirn- und Rückenmarksverletzung, Krebs, allergische Rhinitis, Sinusitis, Rhinosinusitis, chronische akute Mittelohrentzündung, rezidive Mittelohrentzündung, Arzneimittelschäden, Insektenstichreaktion, Latexreaktionen, Konjunktivitis, Nesselsucht, anaphylaktische Reaktionen, anaphylaktische Reaktionen, atopische Dermatitis, Asthma und Nahrungsmittelallergien besteht, ausgewählt wird.

## Revendications

1. Un composé représenté par la formule (I): 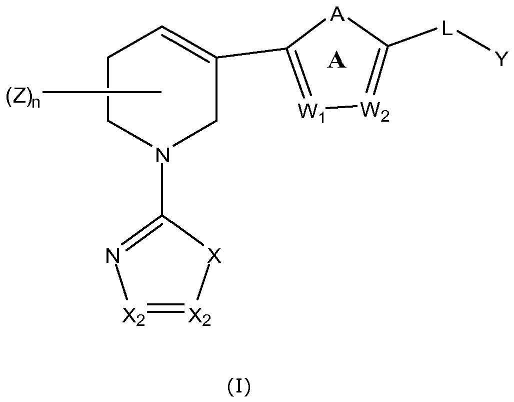 ou d'un sel pharmaceutiquement acceptable, un solvate, ou un clathrate de celui-ci, dans lequel:
A est -CR₁ = CR₂-,-N = CR₁-, ou -CR₁ = N- ;
W1 et W2 sont chacun, indépendamment, CR₁ ou N;
X est-S-,-O-, ou-NR_{c}-;
chacun des X₂ sont choisis indépendamment parmi-N-ou-CR_{d}-;
Y est un aryle facultativement substitué, un hétéroaryle facultativement substitué, un groupe alkyle facultativement substitué, un cycloalkyle facultativement substitué, un alcényle facultativement substitué, un cycloalcényle facultativement substitué;
L est -NRCH₂-, -CH₂NR-, -NR-C(O)-, ou -C(O)-NR-;
Z est un groupe alkyle, un halogène, ou -C(O)R₅;
R, à chaque occurrence, est indépendamment -H, un groupe alkyle, -C(O)-R13, or -C(O)OR13;
R_{c} est -H ou un groupe alkyle C₁-C₄;
R_{d} à chaque occurrence est indépendamment -H ou un groupe alkyle C₁-C₄;
R₁ et R₂, à chaque occurrence, sont indépendamment, H, un alkyle facultativement substitué, un alcényle facultativement substitué, un alcynyle facultativement substitué, un cycloalkyle facultativement substitué, un facultativement cycloalcényle substitué, un hétérocyclyle facultativement substitué, un aryle facultativement substitué, un hétéroaryle facultativement substitué, un aralkyle facultativement substitué, un hétéroaralkyle facultativement substitué, un groupe cyano, un groupe nitro, un halogène, -OR₅, -SR₅, -NR₆R₇, -C(O)NR₆R₇, -NR₅C(O)R₅, - C(O)R₅, -C(O)OR₅, -OC(O)R₅, -C(O)SR₅, -SC(O)R₅, -C(S)NR₆R₇, -NR₅C(S)R₅, -C(S)R₅, - C(S)OR₅, -OC(S)R₅, -C(S)SR₅, -SC(S)R₅, -C(NR₈)NR₆R₇, -NR₅C(NR₈)R₅, -C(NR₈)R₅, - C(NR₈)OR₅, -OC(NR₈)R₅, -C(NR₈)SR₅, -SC(NR₈)R₅, -OC(O)OR₅, -OC(O)NR₆R₇, - NR₅C(O)OR₅, -NR₅C(O)NR₆R₇, -SC(O)OR₅, -SC(O)NR₆R₇, -SC(O)SR₅, -NR₅C(O)SR₅, - OC(O)SR₅, -OC(S)OR₅, -OC(S)NR₆R₇, -NR₅C(S)OR₅, -NR₅C(S)NR₆R₇, -SC(S)OR₅, - SC(S)NR₆R₇, -SC(S)SR₅, -NR₅C(S)SR₅, -OC(S)SR₅, -OC(NR₈)OR₅, -OC(NR₈)NR₆R₇, - NR₅C(NR₈)OR₅, -NR₅C(NR₈)NR₆R₇, -SC(NR₈)OR₅, -SC(NR₈)NR₆R₇, -SC(NR₈)SR₅, - NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, - S(O)ₚOR₅, -OS(O)ₚR₅, ou -OS(O)OR₅;
R₅, à chaque occurrence, est indépendamment, H, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un hétéroaryle facultativement substitué, un aralkyle facultativement substitué, ou un hétéroaralkyle facultativement substitué
R₆ et R₇, à chaque occurrence, sont indépendamment, H, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un facultativement hétéroaryle substitué, un groupe aralkyle facultativement substitué, ou un hétéroaralkyle facultativement substitué, ou R₆ et R₇ pris ensemble avec l'atome d'azote auquel ils sont attachés sont un hétérocyclyle facultativement substitué ou un hétéroaryle facultativement substitué;
R₈, à chaque occurrence, est indépendamment, -H, un halogène, un groupe alkyle, -OR₅, -NR₆R₇, -C(O)R₅, -C(O)OR₅, ou -C(O)NR₆R₇;
R₁₁ est un atome d'halogène, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un hétéroaryle facultativement substitué, un aralkyle facultativement substitué, un hétéroaralkyle facultativement substitué, -OR₅, -SR₅, -NR₆R₇, -C(O)NR₆R₇, -C(O)R₅, -C(O)OR₅, - C(O)SR₅, -C(S)NR₆R₇, -C(S)R₅, -C(S)OR₅, -C(S)SR₅, -C(NR₈)NR₆R₇, -C(NR₈)R₅, - C(NR₈)OR₅, ou -C(NR₈)SR₅;
R₁₃, à chaque occurrence, est indépendamment, -H, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un hétéroaryle facultativement substitué, un groupe aralkyle facultativement substitué, ou un hétéroaralkyle facultativement substitué;
n est 1;
p est 1 ou 2;
**caractérisé en ce que** l'expression "facultativement substitué" se réfère à la substitution facultative avec un ou plusieurs substituants choisis parmi le groupe comprenant alkyle, alkoxy, alkylsulfanyl, alkylamino, dialkylamino, alcényle, alcynyles, cycloalkyle, cycloalkényles, hétérocyclyle, aryle, hétéroaryle, aralkyle, hétéroaralkyl, haloalkyle, -C(O)NR₁₃R₁₄, -NR₁₅C(O)R₁₆, halo, -OR₁₅, cyano, nitro, haloalkoxy, -C(O)R₁₅, -NR₁₃R₁₄, -SR_{15,} -C(O)OR₁₅, -OCCO)R₁₅, -NR₁₅C(O)NR₁₃R₁₄, -OC(O)NR₁₃R₁₄, - NR₁₅C(O)OR₁₆, -S(O)ₚR₁₅, ou -S(O)ₚNR₁₃R₁₄; dans lequel R₁₃ et R₁₄, à chaque occurrence sont, indépendamment, H, alkyle, alcényle, alcynyles, cycloalkyle, cycloalkényles, hétérocyclyle, aryle, hétéroaryle, aralkyle ou hétéroaralkyl; ou R₁₃ et R₁₄, pris ensemble avec l'atome d'azote auquel ils sont attachés sont un hétérocyclyle ou un hétéroaryle; et R₁₅ et R₁₆ à chaque occurrence sont, indépendamment, H, alkyle, alcényle, alcynyles, cycloalkyle, cycloalkényles, hétérocyclyle, aryle, hétéroaryle, aralkyle ou hétéroaralkyl; et
avec la condition que le composé ne soit pas:
2,6-Difluoro-N-[4-(1-thiazole-2-yl-4-méthyl-1,2,5,6-tétrahydro-pyridine-3-yl)-phényl]-benzamide;
N-[4-(1-thiazole-2-yl-4-méthyl-1,2,5,6-tétrahydro-pyridine-3-yl)-phényl]-3-fluoro-isonicotinamide;
N-[4-(1-thiazole-2-yl-4-méthyl-1,2,5,6-tétrahydro-pyridine-3-yl)-phényl]-3-méthyl-isonicotinamide;
N-[4-(1-thiazole-2-yl-4-méthyl-1,2,5,6-tétrahydro-pyridine-3-yl)-phényl]-3-fluoro-isonicotinamide;
2,6-Difluoro-N-[4-(1-oxazole-2-yl-4-méthyl-1,2,5,6-tétrahydro-pyridine-3-yl)-phényl]-benzamide;
N-[4-(1-oxazole-2-yl-4-méthyl-1,2,5,6-tétrahydro-pyridine-3-yl)-phényl]-3-fluoro-isonicotinamide;
2,6-Difluoro-N-[5-(1-thiazole-2-yl-4-méthyl-1,2,5,6-tétrahydro-pyridine-3-yl)-pyridine-2-yl]-benzamide, chlorhydrate;
N-[4-(1-thiazole-2-yl-4-méthyl-1,2,5,6-tétrahydro-pyridine-3-yl)-phényl]-3-fluoro-isonicotinamide, dichlorhydrate;
2,6-Difluoro-N-[4-(1-thiazole-2-yl-4-méthyl-1,2,5,6-tétrahydro-pyridine-3-yl)-phényl]-benzamide, chlorhydrate;
2,6-Difluoro-N-[4-(1-thiazole-2-yl-4-méthyl-1,2,5,6-tétrahydro-pyridine-3-yl)-phényl]-benzamide;
5-Méthyl-pyrimidine-4-acide carboxylique [4-(1-thiazole-2-yl-4-méthyl-1,2,5,6-tétrahydro-pyridine-3-yl)-phényl]-amide;
2,6-Difluoro-N-[4-(1-thiazole-2-yl-4-formyl-1,2,5,6-tétrahydro-pyridine-3-yl)-phényl]-benzamide;
2,6-Difluoro-N-[4-(1-oxazole-2-yl-4-méthyl-1,2,5,6-tétrahydro-pyridine-3-yl)-phényl]-benzamide, chlorhydrate;
2,6-Difluoro-N-{4-[1-(4-méthyl-oxazole-2-yl)-4-méthyl-1,2,5,6-tétrahydro-pyridine-3-yl]-phényl}-benzamide;
5-Méthyl-pyrimidine-4-acide carboxylique {4-[1-(4,5-diméthyl-oxazole-2-yl)-4-méthyl-1,2,5,6-tétrahydro-pyridine-3-yl]-phényl}-amide;
2,6-Difluoro-N-{4-[1-(4,5-diméthyl-oxazole-2-yl)-4-méthyl-1,2,5,6-tétrahydro-pyridine-3-yl]-phényl}-benzamide;
N-{4-[1-(1H-tétrazol-5-yl)-4-méthyl-1,2,5,6-tétrahydro-pyridine-3-yl]-phényl}-3-fluoro-isonicotinamide;
N-{4-[1-(1-méthyl-1H-tétrazol-5-yl)-4-méthyl-1,2,5,6-tétrahydro-pyridine-3-yl]-phényl}-3-fluoro-isonicotinamide;
N-{4-[1-(2-méthyl-2H-tétrazol-5-yl)-4-méthyl-1,2,5,6-tétrahydro-pyridine-3-yl]-phényl}-3-fluoro-isonicotinamide;
2,6-Difluoro-N-{4-[1-(2-méthyl-2H-tétrazol-5-yl)-4-méthyl-1,2,5,6-tétrahydro-pyridine-3-yl]-phényl}-benzamide;
2,6-Difluoro-N-{4-[1-(1H-tétrazol-5-yl)-4-méthyl-1,2,5,6-tétrahydro-pyridine-3-yl]-phényl}-benzamide;
2,6-Difluoro-N-{4-[1-(1-méthyl-1H-tétrazol-5-yl)-4-méthyl-1,2,5,6-tétrahydro-pyridine-3-yl]-phényl}-benzamide;
2,6-Difluoro-N-[5-(1-thiazole-2-yl-4-méthyl-1,2,5,6-tétrahydro-pyridine-3-yl)-pyrimidin-2-yl]-benzamide; or
2,6-Difluoro-N-[5-(1-thiazole-2-yl-4-méthyl-1,2,5,6-tétrahydro-pyridine-3-yl)-pyrimidin-2-yl]-benzamide, chlorhydrate.

2. Composé selon la revendication 1, dans lequel L est -NR-C(O)- ou -C(O)-NR-.

3. Composé selon la revendication 2, dans lequel L est -NH-C(O)- ou -C(O)-NH-.

4. Composé selon la revendication 1, dans lequel Y est un groupe phényle facultativement substitué, un oxazolyle facultativement substitué, un groupe furanyle facultativement substitué, un pyrazolyle facultativement substitué, pyrimidinyle facultativement substitué, un groupe pyridinyle facultativement substitué, un groupe pyridazinyle facultativement substitué, un thiadiazolyle, facultativement substitué, ou un thiophényle facultativement substitué.

5. Composé selon la revendication 4, dans lequel Y est un groupe phényle facultativement substitué, un groupe pyrimidinyle facultativement substitué, ou un groupe pyridinyle facultativement substitué.

6. Composé selon la revendication 5, dans lequel Y est substitué par un à deux substituants.

7. Composé selon la revendication 6, dans lequel les un à deux substituants sont chacun indépendamment un groupe alkyle C₁-C₄ ou un halogène.

8. Composé selon la revendication 7, dans lequel Y est un difluorophényl.

9. Composé selon la revendication 1, dans lequel Z est méthyl.

10. Composé selon la revendication 1, dans lequel le composé est représenté par la formule (II): 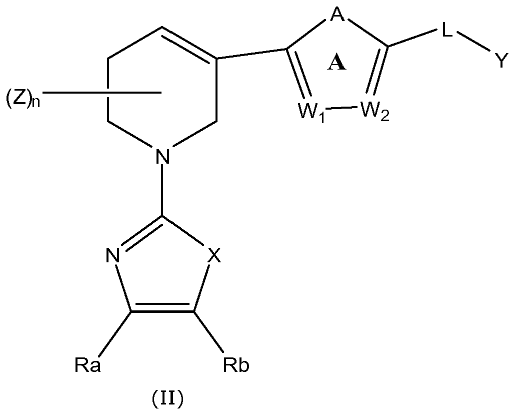 dans lequel Rₐ et R_{b} sont chacun, indépendamment, -H ou un groupe alkyle C₁-C₄.

11. Composé selon la revendication 10, dans lequel le composé est représenté par la formule (III):

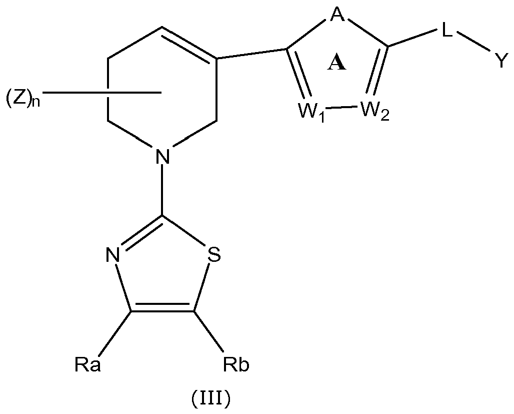

12. Composé selon l'une des revendications 10 ou 11, dans lequel:
L est -NHCH₂-, -CH₂NH-, -NH-C(O)-, ou -C(O)-NH-; et
Y est un phényl facultativement substitué, un pyrimidinyle facultativement substitué, ou un pyridinyle facultativement substitué.

13. Composé selon la revendication 11, dans lequel le composé est représenté par la formule (IV): 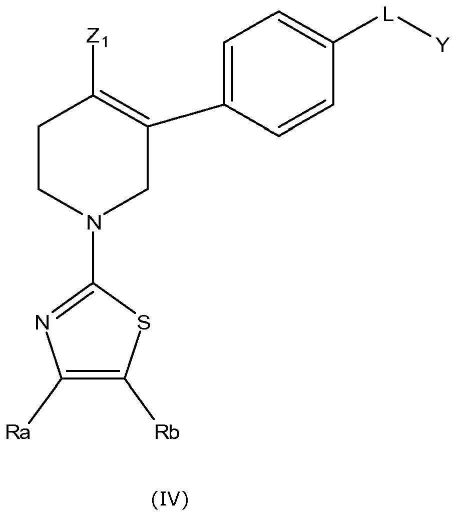 Dans lequel Z₁ est alkyle, un halogène, ou -C(O)R₅.

14. Composé selon la revendication 13, dans lequel:
L est-NR-C(O)- ou -C(O)-NR.

15. Composé selon la revendication 14, dans lequel le composé est représenté par la formule (V): 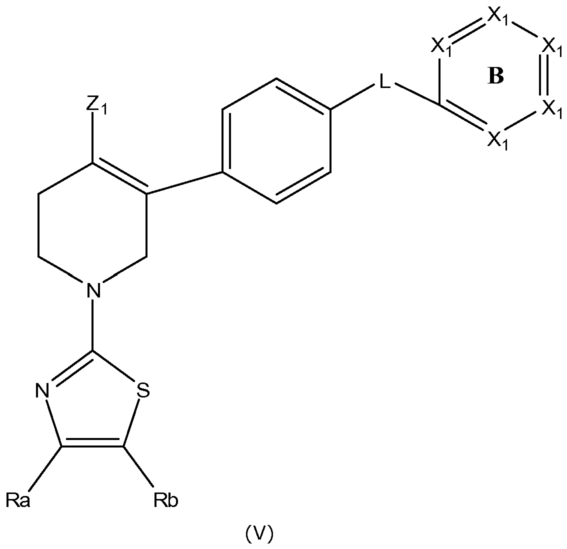 dans lequel chaque X₁ représentent indépendamment N ou CR₁et au moins deux X₁ sont des groupes CR₁.

16. Composé selon la revendication 15, dans lequel:
L est-NR-C(O)- ou -C(O)-NR-.

17. Composé selon la revendication 1, dans lequel le composé est choisi parmi

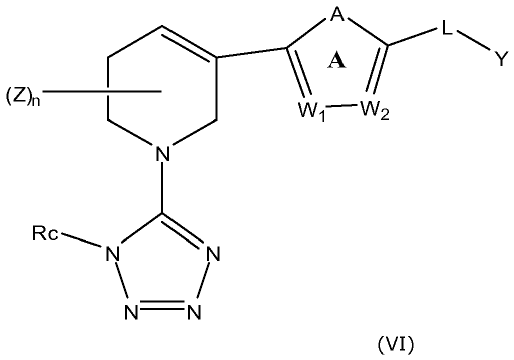

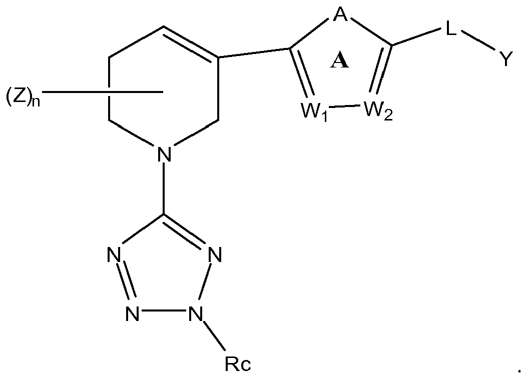

18. Un composé de formule (VII): 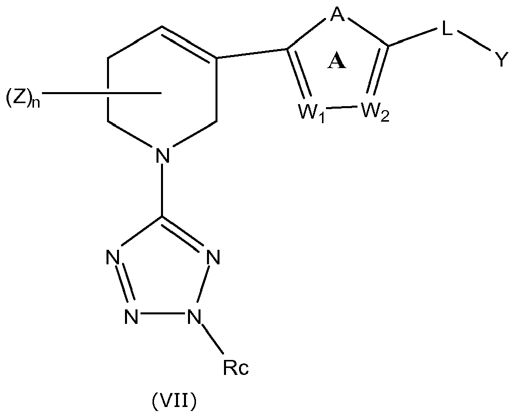 ou d'un sel pharmaceutiquement acceptable, un solvate, ou un clathrate de celui-ci, dans lequel:
A est -CR₁ = CR₂-,-N = CR₁-, ou -CR₁ = N- ;
W1 et W2 sont chacun, indépendamment, CR₁ ou N;
Y est un aryle facultativement substitué, un hétéroaryle facultativement substitué, un groupe alkyle facultativement substitué, un cycloalkyle facultativement substitué, un alcényle facultativement substitué;
L est -NRCH₂-, -CH₂NR-, -NR-C(O)-, ou -C(O)-NR-;
Z est un groupe alkyle, un halogène, ou -C(O)R₅;
R, à chaque occurrence, est indépendamment -H, un groupe alkyle, -C(O)-R13, or -C(O)OR13;
R_{c} est -H ou un groupe alkyle C₁-C₄;
R₁ et R₂, à chaque occurrence, sont indépendamment, H, un alkyle facultativement substitué, un alcényle facultativement substitué, un alcynyle facultativement substitué, un cycloalkyle facultativement substitué, un facultativement cycloalcényle substitué, un hétérocyclyle facultativement substitué, un aryle facultativement substitué, un hétéroaryle facultativement substitué, un aralkyle facultativement substitué, un hétéroaralkyle facultativement substitué, un groupe cyano, un groupe nitro, un halogène, -OR₅, -SR₅, -NR₆R₇, -C(O)NR₆R₇, -NR₅C(O)R₅, - C(O)R₅, -C(O)OR₅, -OC(O)R₅, -C(O)SR₅, -SC(O)R₅, -C(S)NR₆R₇, -NR₅C(S)R₅, -C(S)R₅, - C(S)OR₅, -OC(S)R₅, -C(S)SR₅, -SC(S)R₅, -C(NR₈)NR₆R₇, -NR₅C(NR₈)R₅, -C(NR₈)R₅, - C(NR₈)OR₅, -OC(NR₈)R₅, -C(NR₈)SR₅, -SC(NR₈)R₅, -OC(O)OR₅, -OC(O)NR₆R₇, - NR₅C(O)OR₅, -NR₅C(O)NR₆R₇, -SC(O)OR₅, -SC(O)NR₆R₇, -SC(O)SR₅, -NR₅C(O)SR₅, - OC(O)SR₅, -OC(S)OR₅, -OC(S)NR₆R₇, -NR₅C(S)OR₅, -NR₅C(S)NR₆R₇, -SC(S)OR₅, - SC(S)NR₆R₇, -SC(S)SR₅, -NR₅C(S)SR₅, -OC(S)SR₅, -OC(NR₈)OR₅, -OC(NR₈)NR₆R₇, - NR₅C(NR₈)OR₅, -NR₅C(NR₈)NR₆R₇, -SC(NR₈)OR₅, -SC(NR₈)NR₆R₇, -SC(NR₈)SR₅, - NR₅C(NR₈)SR₅, -OC(NR₈)SR₅, -S(O)ₚR₅, -S(O)ₚNR₆R₇, -NR₅S(O)ₚR₅, -NR₅S(O)NR₆R₇, - S(O)ₚOR₅, -OS(O)ₚR₅, or -OS(O)OR₅;
R₅, à chaque occurrence, est indépendamment, H, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un hétéroaryle facultativement substitué, un aralkyle facultativement substitué, ou un hétéroaralkyle facultativement substitué
R₆ et R₇, à chaque occurrence, sont indépendamment, H, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un facultativement hétéroaryle substitué, un groupe aralkyle facultativement substitué, ou un hétéroaralkyle facultativement substitué, ou R₆ et R₇ pris ensemble avec l'atome d'azote auquel ils sont attachés sont un hétérocyclyle facultativement substitué ou un hétéroaryle facultativement substitué;
R₈, à chaque occurrence, est indépendamment, -H, un halogène, un groupe alkyle, -OR₅, -NR₆R₇, -C(O)R₅, -C(O)OR₅, ou -C(O)NR₆R₇;
R₁₁ est un atome d'halogène, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un hétéroaryle facultativement substitué, un aralkyle facultativement substitué, un hétéroaralkyle facultativement substitué, -OR₅, -SR₅, -NR₆R₇, -C(O)NR₆R₇, -C(O)R₅, -C(O)OR₅, - C(O)SR₅, -C(S)NR₆R₇, -C(S)R₅, -C(S)OR₅, -C(S)SR₅, -C(NR₈)NR₆R₇, -C(NR₈)R₅, - C(NR₈)OR₅, ou -C(NR₈)SR₅;
R₁₃, à chaque occurrence, est indépendamment, -H, un alkyle facultativement substitué, un alcényle facultativement substitué, un alcynyle facultativement substitué, un cycloalkyle facultativement substitué, un cycloalcényle facultativement substitué, un hétérocyclyle facultativement substitué, un aryle facultativement substitué, un hétéroaryle facultativement substitué, un groupe aralkyle facultativement substitué, ou un hétéroaralkyle facultativement substitué;
n est 1;
p est 1 ou 2;
**caractérisé en ce que** l'expression "facultativement substitué" se réfère à la substitution facultative avec un ou plusieurs substituants choisis parmi le groupe comprenant alkyle, alkoxy, alkylsulfanyl, alkylamino, dialkylamino, alcényle, alcynyles, cycloalkyle, cycloalkényles, hétérocyclyle, aryle, hétéroaryle, aralkyle, hétéroaralkyl, haloalkyle, -C(O)NR₁₃R₁₄, -NR₁₅C(O)R₁₆, halo, -OR₁₅, cyano, nitro, haloalkoxy, -C(O)R₁₅, -NR₁₃R₁₄, -SR₅₁, -C(O)OR₁₅, -OC(O)R₁₅, -NR₁₅C(O)NR₁₃R₁₄, -OC(O)NR₁₃R₁₄, - NR₁₅C(O)OR₁₆, -S(O)ₚR₁₅, ou -S(O)ₚNR₁₃R₁₄; dans lequel R₁₃ et R₁₄, à chaque occurrence sont, indépendamment, H, alkyle, alcényle, alcynyles, cycloalkyle, cycloalkényles, hétérocyclyle, aryle, hétéroaryle, aralkyle ou hétéroaralkyl; ou R₁₃ et R₁₄, pris ensemble avec l'atome d'azote auquel ils sont attachés sont un hétérocyclyle ou un hétéroaryle; et R₁₅ et R₁₆ à chaque occurrence sont, indépendamment, H, alkyle, alcényle, alcynyles, cycloalkyle, cycloalkényles, hétérocyclyle, aryle, hétéroaryle, aralkyle ou hétéroaralkyl; et
avec la condition que le composé ne soit pas:
N-{4-[1-(2-méthyl-2H-tétrazol-5-yl)-4-méthyl-1,2,5,6-tétrahydro-pyridine-3-yl]-phényl}-3-fluoro-isonicotinamide; ou
2,6-Difluoro-N-{4-[1-(2-méthyl-2H-tétrazol-5-yl)-4-méthyl-1,2,5,6-tétrahydro-pyridine-3-yl]-phényl}-benzamide.

19. Une composition pharmaceutique, comprenant un excipient pharmaceutiquement acceptable et un composé selon l'une quelconque des revendications 1 à 18.

20. Composition pharmaceutique selon la revendication 19, comprenant en outre un ou plusieurs agents thérapeutiques supplémentaires.

21. La composition pharmaceutique selon la revendication 20, dans laquelle l'agent thérapeutique supplémentaire est choisi parmi le groupe constitué par des agents immunosuppresseurs, des agents anti-inflammatoires et des mélanges appropriés de ceux-ci ou l'agent thérapeutique supplémentaire est choisi dans le groupe constitué par des stéroïdes, des agents anti-inflammatoires non stéroïdiens, des antihistaminiques, des analgésiques, et des mélanges appropriés de ceux-ci.

22. Usage d'un composé selon l'une quelconque des revendications 1 à 18 pour la préparation d'un médicament pour le traitement ou la prévention d'un trouble immunitaire, ou pour le traitement ou la prévention d'une condition inflammatoire, ou pour inhiber le système immunitaire, ou pour le traitement ou la prévention d'un trouble allergique chez un sujet en ayant besoin.

23. Usage selon la revendication 22, dans lequel le trouble est choisi parmi le groupe constitué en la sclérose en plaques, la myasthénie grave, syndrome de Guillain-Barr [epsilon], l'uvéite auto-immune, anémie hémolytique auto-immune, l'anémie pernicieuse, la thrombocytopénie auto-immune, artérite temporale, syndrome anti-phospholipide, vascularites comme la granulomatose de Wegener, la maladie de Behcet, le psoriasis, la dermatite herpétiforme, pemphigus vulgaire, vitiligo, maladie de Crohn, la colite ulcéreuse, la cirrhose biliaire primitive, hépatite auto-immune, diabète de type 1 ou diabète sucré auto-immunes, la maladie de Grave, la thyroïdite de Hashimoto, ovarite et orchite auto-immune, maladie auto-immune de la glande surrénale, la polyarthrite rhumatoïde, le lupus érythémateux disséminé, la sclérodermie, la polymyosite, la dermatomyosite, la spondylarthrite ankylosante, le syndrome de Sjögren, le rejet de greffe, le rejet de greffe de peau, l'arthrite, l'arthrite rhumatoïde, l'ostéoarthrite et les maladies osseuses liées à une augmentation de la résorption osseuse, la maladie intestinale inflammatoire, l'iléite, la colite ulcéreuse, le syndrome de Barrett, la maladie de Crohn; l'asthme, le syndrome respiratoire de l'adulte de détresse, la maladie respiratoire obstructive chronique, dystrophie de la cornée, le trachome, l'onchocercose, l'uvéite, ophtalmie sympathique, endophtalmie, la gingivite, la parodontite, la tuberculose, la lèpre, les complications urémiques, la glomérulonéphrite, néphropathie; sclerodermatitis, le psoriasis, l'eczéma, les maladies démyélinisantes chroniques du système nerveux, la sclérose en plaques, la neurodégénérescence liée au SIDA, la maladie d'Alzheimer, la méningite infectieuse, l'encéphalomyélite, la maladie de Parkinson, la maladie de Huntington, la sclérose encéphalites virales ou auto-immunes latérale amyotrophique, les troubles auto-immuns, la vasculite complexe immun, le lupus systémique et érythémateux; lupus systémique érythémateux disséminé (SLE); cardiomyopathie, ischémique hypercholestérolémie de maladie cardiaque, l'athérosclérose, la pré-éclampsie, une insuffisance hépatique chronique, les traumatismes de la moelle épinière et du cerveau, le cancer, la rhinite allergique, la sinusite, rhino-sinusite, otite moyenne chronique, l'otite moyenne récurrente, des réactions médicamenteuses, les réactions piqûre d'insecte, les réactions au latex, conjunctivite, urticaire, réactions d'anaphylaxie des réactions anaphylactoïdes, la dermatite atopique, d'asthme, et des allergies alimentaires.
